# EUROPEAN PATENT APPLICATION

(11) **EP 4 834 941 A1**
(43) Date of publication of application: **04.11.2026**
(21) Application number: 24911570.0
(22) Date of filing: 27.12.2024
(51) Int. Cl.: C07K 16/28, C07K 16/30, C12N 15/63, A61K 39/395, A61P 35/00

(54) **ANTI-CDH17 ANTIBODY OR ANTIGEN-BINDING FRAGMENT THEREOF AND USE THEREOF**

(30) Priority: 29.12.2023 CN 202311871606
(71) Applicant: Guangdong Fapon Biopharma Inc., Dongguan, Guangdong 523808 (CN)
(72) Inventor: ZHANG, Yibo, Dongguan, Guangdong 523808 (CN); DING, Jiaojiao, Dongguan, Guangdong 523808 (CN); XIAO, Haipeng, Dongguan, Guangdong 523808 (CN); WU, Xiaodong, Dongguan, Guangdong 523808 (CN); XIAO, Liang, Dongguan, Guangdong 523808 (CN); REN, Baiguang, Dongguan, Guangdong 523808 (CN); LU, Di, Dongguan, Guangdong 523808 (CN); HUO, Yongting, Dongguan, Guangdong 523808 (CN)
(74) Representative: Berggren Oy
(86) International application number: PCT/CN2024/143461
(87) International publication number: WO 2025/140654

(57) **Abstract**

An anti-CDH17 antibody or an antigen-binding fragment thereof, and an application thereof. The antibody can specifically bind to one or more Extra Cellular Domains (ECDs) among CDH17 ECDs 1-7, has ADCC activity, and can mediate cell endocytosis, thereby providing a new possibility for the treatment and/or prevention of cancers.

## Description

### Cross-Reference to Related Application

This application claims priority to Chinese Patent Application No. 202311871606.8 filed on November 29, 2023 and entitled "Anti-CDH17 Antibody or Antigen-Binding Fragment Thereof, and Application Thereof", the disclosure of which is hereby incorporated by reference in its entirety.

### Technical Field

The present disclosure relates to the technical field of biology, and in particular, to an anti-CDH17 antibody or an antigen-binding fragment thereof, and an application thereof.

### Background

The occurrence and progression of tumors involve a multifactorial, multistep interactive process, in which the adhesion between cells and the dysregulation of athletic ability are one of the key mechanisms for the occurrence and progression of tumors. Cadherin-17 is a new member of the cadherin family discovered in recent years, is expressed in mouse liver and small intestine, and plays a crucial role in cell adhesion, cell recognition, the development and shape maintenance of tissues and organs, etc. Research indicates that its expression is closely associated with the occurrence and progression of epithelial tumors of the digestive system, making it a promising candidate for a new tumor target and biomarker.

Cadherin-17 (CDH17) is a new member of the cadherin family, and is first obtained from a mouse liver cDNA library. Due to its exclusive expression in mouse liver and small intestine, it is also designated as Liver-Intestine cadherin (LI-cadherin). The CDH17 is commonly expressed in embryonic and adult intestinal epithelial cells, as well as in some pancreatic duct epithelial cells. However, it shows almost no significant expression in hepatocytes, esophageal epithelial cells, or gastric mucosa in healthy individuals.

The cadherin achieves a biological function by mediating the connection between calcium-dependent cells. A classic ligand for the cadherin is calcium catenin, including an α-chain protein, a β-chain protein, a γ-chain protein, a P120 protein, etc. The cadherin interacts with an intracellular skeleton via the calcium catenin, so as to regulate the adhesion between the cells. The CDH17 functions as a Ca²⁺-dependent cell adhesion molecule. However, the CDH17 does not achieve the connection effect by means of intracellular calcium catenin and actin of the cytoskeleton, but instead achieves cell adhesion by directly binding to the cytoskeleton. During tumor progression, by binding an Extra Cellular Domain (ECD) EC6 including an RGD domain (green) to an integrin protein α2β1 on adjacent tumor cells, integrin activation, adhesion, proliferation, invasion, tumorigenesis, and metastasis are improved. Moreover, the study also finds that the expression of the CDH17 is an independent prognostic factor for predicting a survival period of a patient. The expression of the CDH17 affects the prognosis of a patient with lymph node-negative gastric cancer, potentially reflecting the role of the protein in maintaining polarity and adhesion between normal cells. Therefore, the development and optimization of an anti-CDH17 antibody have significant importance for the prevention or treatment of tumor-related diseases.

In view of this, the present disclosure is especially proposed.

### Summary

One of the objectives of the present disclosure is to provide an antibody or an antigen-binding fragment that may specifically bind to CDH17, and an application.

In order to solve the above technical problem, the present disclosure uses the following technical solutions.

A first aspect provides an anti-CDH17 antibody or an antigen-binding fragment thereof. The anti-CDH17 antibody or the antigen-binding fragment thereof binds to one or more of a CDH17 ECD 1 (D1), a CDH17 ECD 2 (D2), a CDH17 ECD 3 (D3), a CDH17 ECD 4 (D4), a CDH17 ECD 5 (D5), a CDH17 ECD 6 (D6), and a CDH17 ECD 7 (D7).

In a second aspect, the present disclosure provides an anti-CDH17 antibody or an antigen-binding fragment thereof, which competitively binds to CDH17 with the anti-CDH17 antibody of the first or second aspect, or an epitope thereof binding to a CDH17 antigen is identical to an epitope of the anti-CDH17 antibody of the first or second aspect binding to the CDH17 antigen.

In a third aspect, the present disclosure provides a biomaterial, including any one of the following (i) to (vi):
(i) a polyspecific antibody, including the anti-CDH17 antibody of the first or second aspect;
(ii) a Chimeric Antigen Receptor (CAR), including an ECD, where the ECD includes an antigen-binding domain, and the antigen-binding domain includes the anti-CDH17 antibody or the antigen-binding fragment thereof of the first or second aspect;
(iii) a nucleic acid molecule, encoding the anti-CDH17 antibody or the antigen-binding fragment thereof of the first or second aspect, or the polyspecific antibody in (i), or the CAR in (ii);
(iv) a vector, having the nucleic acid molecule in (iii);
(v) a recombinant cell, expressing the anti-CDH17 antibody or the antigen-binding fragment thereof of the first or second aspect, or expressing the polyspecific antibody in (i), or expressing the CAR in (ii), or including the nucleic acid molecule in (iii), or including the vector in (iv); and
(vi) an engineered immune cell, expressing the CAR in (ii) or including the nucleic acid molecule encoding the CAR in (ii).

In a fourth aspect, the present disclosure provides a pharmaceutical composition, including the anti-CDH17 antibody or the antigen-binding fragment thereof of the first or second aspect, or the biomaterial of the third aspect.

In a fifth aspect, the present disclosure provides an application of the anti-CDH17 antibody or the antigen-binding fragment thereof of the first or second aspect, or the biomaterial of the third aspect, or the pharmaceutical composition of the fourth aspect. The application includes any one of the following.
(I) CDH17 or cells expressing the CDH17 are detected.
(I) A product for detecting the CDH17 or cells expressing the CDH17 is prepared.
(III) The anti-CDH17 antibody or the antigen-binding fragment thereof, or the biomaterial, or the pharmaceutical composition is used for treating, preventing, or alleviating diseases, symptoms, or conditions associated with the CDH17.
(IV) A pharmaceutical composition for treating, preventing, or alleviating diseases, symptoms, or conditions associated with the CDH17 is prepared.

In a sixth aspect, the present disclosure provides a method for preparing the foregoing anti-CDH17 antibody or the antigen-binding fragment thereof. The method includes the following steps.
(a) The foregoing recombinant cell is cultured under a condition expressing the anti-CDH17 antibody or the antigen-binding fragment thereof.
(b) The anti-CDH17 antibody or the antigen-binding fragment thereof obtained in step (a) is isolated and purified.

In a seventh aspect, the present disclosure provides a kit for detecting CDH17. The kit includes the foregoing anti-CDH17 antibody or the antigen-binding fragment thereof.

In an eighth aspect, the present disclosure provides a method for treating diseases. The method includes: an effective dosage of the anti-CDH17 antibody or the antigen-binding fragment thereof of the first or second aspect, the biomaterial of the third aspect, or the pharmaceutical composition of the fourth aspect is applied to a subject in need. Optionally, the diseases are diseases associated with high expression of human CDH17.

Optionally, the diseases are tumors.

Optionally, the tumors are selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, and hematological system cancer.

### Brief Description of the Drawings

In order to more clearly illustrate the specific implementations of the present disclosure or the technical solutions in the related art, the drawings used in the description of the specific implementations or the related art will be briefly described below. It is apparent that the drawings in the following descriptions are some implementations of the present disclosure. Other drawings can be obtained from those skilled in the art according to these drawings without any creative work.
Fig. 1 is a schematic diagram of a human CDH17 ECD.
Fig. 2 is a schematic diagram of a structure relationship between human CDH17 and a human CDH17 ECD fragment according to Embodiment 1 of the present disclosure.
Fig. 3 shows a flow cytometry analysis method for detecting binding activity of a monoclonal antibody (F1-87.15, F1-99.29, F1-76.12, or F1-86.9) and a CHO-hCDH17 cell according to Embodiment 2.
Fig. 4 shows a flow cytometry analysis method for detecting binding activity of a monoclonal antibody (F2-89.7, F2-155.7, F2-238.14, or F2-324.7) and a CHO-hCDH17 cell according to Embodiment 2.
Fig. 5 shows a flow cytometry analysis method for detecting binding activity of a monoclonal antibody (F1-124.38, F2-55.10, F2-291.2, F3-1.5, or F3-6.18) and a CHO-hCDH17 cell according to Embodiment 2.
Fig. 6 shows a flow cytometry analysis method for detecting binding activity of a monoclonal antibody (F1-87.15, F1-41.16, F2-238.14, F1-111.5, or F2-55.10) and an AsPC-1 cell according to Embodiment 2.
Fig. 7 shows a flow cytometry analysis method for detecting binding activity of a monoclonal antibody (F1-86.23, F1-99.29, F1-139.6, F1-76.12, F1-124.2, F1-122.19, F1-86.9, F1-143.7, F1-124.38, or F1-143.38) and an AsPC-1 cell according to Embodiment 2.
Fig. 8 shows a flow cytometry analysis method for detecting binding activity of a monoclonal antibody (F2-71.18, F2-291.2, F2-324.17, F2-89.7, F2-155.7, F2-324.7, F2-186.1, F3-1.5, F3-6.18, or F3-36.14) and an AsPC-1 cell according to Embodiment 2.
Fig. 9 shows an ELISA for detecting binding activity of a monoclonal antibody (F1-87.15, F1-99.29, F1-127.8, F1-76.12, F1-111.5, F1-86.9, F1-143.7, F1-41.16, F1-124.38, or F2-71.18) and monkey CDH17 according to Embodiment 3.
Fig. 10 shows an ELISA for detecting binding activity of a monoclonal antibody (F2-89.7, F2-155.7, F2-186.1, F2-238.14, F2-324.7, F2-55.10, F2-291.2, F3-1.5, F3-6.18, or F3-36.14) and monkey CDH17 according to Embodiment 3.
Fig. 11 shows an ELISA for detecting binding activity of a monoclonal antibody (F1-87.15, F1-99.29, F1-127.8, F1-76.12, F1-111.5, F1-86.9, F1-143.7, F1-41.16, F1-124.38, or F2-71.18) and mouse CDH17 according to Embodiment 4.
Fig. 12 shows an ELISA for detecting binding activity of a monoclonal antibody (F2-89.7, F2-155.7, F2-186.1, F2-238.14, F2-324.7, F2-55.10, F2-291.2, F3-1.5, F3-6.18, or F3-36.14) and mouse CDH17 according to Embodiment 4.
Fig. 13 shows an ELISA for detecting binding activity of a monoclonal antibody (F1-87.15, F1-41.16, F2-238.14, F1-111.5, F2-55.10, F1-86.23, F1-99.29, F1-139.6, F1-76.12, F1-124.2, F1-122.19, or F1-86.9) and human CDH17 according to Embodiment 5.
Fig. 14 shows an ELISA for detecting binding activity of a monoclonal antibody (F1-143.7, F1-124.38, F1-143.38, F2-71.18, F2-291.2, F2-324.17, F2-89.7, F2-155.7, F2-324.7, F2-186.1, F3-1.5, F3-6.18, or F3-36.14) and human CDH17 according to Embodiment 5.
Fig. 15 shows an ELISA for detecting binding activity of a monoclonal antibody (F1-87.15, F1-41.16, F2-238.14, F1-111.5, F2-55.10, F1-86.23, F1-99.29, F1-139.6, F1-76.12, F1-124.2, F1-122.19, or F1-86.9) and mouse CDH17 (D1-D4) according to Embodiment 6.
Fig. 16 shows an ELISA for detecting binding activity of a monoclonal antibody (F1-143.7, F1-124.38, F1-143.38, F2-71.18, F2-291.2, F2-324.17, F2-89.7, F2-155.7, F2-324.7, F2-186.1, F3-1.5, F3-6.18, or F3-36.14) and mouse CDH17 (D1-D4) according to Embodiment 6.
Fig. 17 shows an ELISA for detecting binding activity of a monoclonal antibody (F1-87.15, F1-41.16, F2-238.14, F1-111.5, F2-55.10, F1-86.23, F1-99.29, F1-139.6, F1-76.12, F1-124.2, F1-122.19, or F1-86.9) and mouse CDH17 (D5-D7) according to Embodiment 6.
Fig. 18 shows an ELISA for detecting binding activity of a monoclonal antibody (F1-143.7, F1-124.38, F1-143.38, F2-71.18, F2-291.2, F2-324.17, F2-89.7, F2-155.7, F2-324.7, F2-186.1, F3-1.5, F3-6.18, or F3-36.14) and mouse CDH17 (D5-D7) according to Embodiment 6.
Fig. 19 shows a flow cytometry for detecting killing activity of an overexpressed CHO-hCDH17 cell mediated by a monoclonal antibody according to Embodiment 7.
Fig. 20 shows an LDH method for detecting killing activity of an AsPC-1 cell endogenously expressing human CDH17 and mediated by a monoclonal antibody according to Embodiment 7.
Fig. 21 shows endocytic activity of an AsPC-1 cell to monoclonal antibody F1-87.15, F1-99.29, F1-76.12, F1-86.9, F1-143.7, F2-238.14, F2-55.10, F3-1.5, F3-6.18, and F3-36.14 according to Embodiment 8.
Fig. 22 shows a flow cytometry for detecting binding activity of a monoclonal antibody (R4723, R4709, R4710, R4711, R4712, R4713, or R4714) and a CHO-CDH17 cell according to Embodiment 10.
Fig. 23 shows a flow cytometry for detecting binding activity of a monoclonal antibody (R4723, R4715, R4716, R4717, or R4718) and a CHO-CDH17 cell according to Embodiment 10.
Fig. 24 shows a flow cytometry for detecting binding activity of a monoclonal antibody (R4850, R4832, R4833, R4834, R4835, R4836, or R4837) and a CHO-CDH17 cell according to Embodiment 10.
Fig. 25 shows a flow cytometry for detecting binding activity of a monoclonal antibody (R4850, R4838, R4839, R4840, R4841, R4842, or R4843) and a CHO-CDH17 cell according to Embodiment 10.
Fig. 26 shows a flow cytometry for detecting binding activity of a monoclonal antibody (R4723, R4709, R4710, R4711, R4712, R4713, or R4714) and an AsPC-1 cell according to Embodiment 10.
Fig. 27 shows a flow cytometry for detecting binding activity of a monoclonal antibody (R4723, R4715, R4716, R4717, or R4718) and an AsPC-1 cell according to Embodiment 10.
Fig. 28 shows a flow cytometry for detecting binding activity of a monoclonal antibody (R4850, R4832, R4833, R4834, R4835, R4836, or R4837) and an AsPC-1 cell according to Embodiment 10.
Fig. 29 shows a flow cytometry for detecting binding activity of a monoclonal antibody (R4850, R4838, R4839, R4840, R4841, R4842, or R4843) and an AsPC-1 cell according to Embodiment 10.
Fig. 30 shows a flow cytometry for detecting binding activity of a monoclonal antibody (R4723, R4709, R4710, R4711, R4712, R4713, or R4714) and an OCUM-1 cell according to Embodiment 10.
Fig. 31 shows a flow cytometry for detecting binding activity of a monoclonal antibody (R4723, R4715, R4716, R4717, or R4718) and an OCUM-1 cell according to Embodiment 10.
Fig. 32 shows a flow cytometry for detecting binding activity of a monoclonal antibody (R4850, R4832, R4833, R4834, R4835, R4836, or R4837) and an OCUM-1 cell according to Embodiment 10.
Fig. 33 shows a flow cytometry for detecting binding activity of a monoclonal antibody (R4850, R4838, R4839, R4840, R4841, R4842, or R4843) and an OCUM-1 cell according to Embodiment 10.
Fig. 34 shows an ELISA for detecting binding activity of a monoclonal antibody (R4723, R4709, R4710, R4711, R4712, R4713, or R4714) and monkey CDH17 according to Embodiment 11.
Fig. 35 shows an ELISA for detecting binding activity of a monoclonal antibody (R4723, R4715, R4716, R4717, or R4718) and monkey CDH17 according to Embodiment 11.
Fig. 36 shows an ELISA for detecting binding activity of a monoclonal antibody (R4850, R4832, R4833, R4834, R4835, R4836, or R4837) and monkey CDH17 according to Embodiment 11.
Fig. 37 shows an ELISA for detecting binding activity of a monoclonal antibody (R4850, R4838, R4839, R4840, R4841, R4842, or R4843) and monkey CDH17 according to Embodiment 11.

### Detailed Description of the Embodiments

The technical solutions of the present disclosure will be clearly and completely described below with reference to the embodiments. It is apparent that the described embodiments are merely part of the embodiments of the present disclosure, not all the embodiments. Based on the embodiments in the present disclosure, all other embodiments obtained by those of ordinary skill in the art without creative work shall fall within the protection scope of the present disclosure.

In the present disclosure, "a/an" and "one" include plural references unless the context clearly indicates otherwise. For example, "an antibody" refers to one antibody or more than one antibody.

In the present disclosure, the terms "first" and "second" are used for descriptive purposes only and are not to be construed as indicating or implying relative importance or implicitly specifying the number of technical features indicated. Therefore, features delimited with "first" or "second" may expressly or implicitly include at least one of that feature. In the description of the present disclosure, "a plurality of" means at least two, for example, two, three, or the like, unless otherwise explicitly specified.

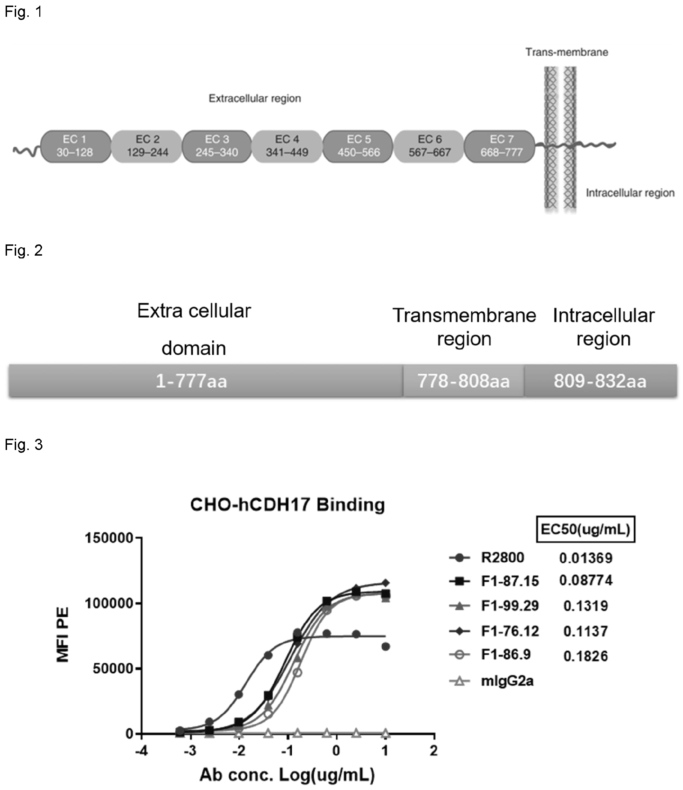

In the present disclosure, the terms "CDH17" and "CDH-17" are used interchangeably to refer to Cadherin-17, which is also referred to as Liver-Intestine cadherin (LI-cadherin). It belongs to a member of a non-classical cadherin superfamily and shares structural homology with classical cadherin. However, the CDH17 also has a unique structure: 1) an ECD of the CDH17 consists of 7 repetitive sequences (Fig. 1), differing from 5 repetitive sequences of the non-classical cadherin; 2) in an extracellular D1 cell adhesion recognition domain, the CDH17 includes an AAL sequence that replaces an HAV sequence corresponding to the classical cadherin; 3) a cytoplasmic tail region of the CDH17 includes only 20 amino acid residues, while the cytoplasmic tail region of the classical cadherin includes 150-160 amino acid residues. Research indicates that structural abnormalities and functional disorders of the cadherin are closely associated with tumor invasion and metastasis. Since the CDH17 has a short cytoplasmic tail domain, the CDH17 cannot interact with a catenin network or an actin cytoskeleton. Therefore, the CDH17 is classified as a variant of the classical cadherin. An N terminal of the CDH17 is located extracellularly, and a C terminal is located intracellularly. The CDH17 includes 7 ECDs (including CDH17 D1-7, also referred to as EC1-EC7), a transmembrane region, and a cytoplasmic tail region, and its adhesion properties are primarily determined by the ECDs. A schematic structural diagram of human CDH17 is shown in Fig. 2. Unless otherwise specified, the term "CDH17" includes human CDH17, subtypes thereof, and homologous species expressed by cells that naturally express the CDH17 or by cells transfected with a CDH17 gene. The term further includes artificially-modified CDH17, where artificial modification includes but is not limited to polypeptides or proteins resulting from mutation, truncation, or fusion with other domains, provided they retain antigenic epitopes necessary for antibody binding. The anti-CDH17 antibody or the antigen-binding fragment thereof provided in the present disclosure can specifically bind to the CDH17.

In the present disclosure, the term "antibody" includes any immunoglobulin capable of binding to a specific antigen. The term "antibody" is used in a broadest manner and encompasses various antibody structures, including but not limited to: a monoclonal antibody/polyclonal antibody, a monospecific antibody/polyspecific antibody, a full-length antibody/antigen-binding fragment, as long as they show the desired antigen-binding activity. Typically, a natural intact antibody includes two heavy (H) chains and two light (L) chains. Based on whether α, δ, ε, γ, and µ heavy chains are included, the antibodies may be classified into five major categories or isotypes: IgA, IgD, IgE, IgG, and IgM. A plurality of major antibody categories may also be divided into subclasses, such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain), etc. Each heavy chain consists of a variable region (VH) and first, second, third, and fourth (optional) constant regions (CH1, CH2, CH3, and CH4, respectively). Light chains of mammals may be classified as either λ or κ, and each light chain consists of a variable region (VL) and a constant region (CL). The variable regions of light chain and heavy chain determine the binding of an antigen. The variable region of each chain typically includes three hypervariable regions, which are also referred to as "CDRs". The light chain CDR includes LCDR1, LCDR2, and LCDR3, and the heavy chain CDR includes HCDR1, HCDR2, and HCDR3. The variable regions (VH and VL) each consist of 3 CDRs connected by 4 Framework Regions (FRs). Generally, the variable regions VL/VH of the heavy chain and the light chain may be obtained by connecting the following numbered CDRs and FRs in the following combination arrangement: FR1-CDR1-FR2-CDR2-FR3-CDR3-FR4.

In the present disclosure, the term "antigen-binding fragment" is a substance containing an antibody CDR, and the fragment lacks some of amino acids present in a full-length chain but can still be capable of specifically binding to an antigen. Such fragments are biologically-active because the fragments bind to target antigens and may competitively bind to given epitope with other antigen-binding molecules (including a complete antibody). Examples of the antigen-binding fragment include, but are not limited to, Fab, Fab', F(ab')₂, a Fv fragment, a disulfide-stabilized Fv fragment (dsFv), (dsFv)₂, bispecific dsFv (dsFv-dsFv'), a disulfide-stabilized bifunctional antibody (ds diabody), a single-chain antibody molecule (scFv), a scFv dimer (bivalent bifunctional antibody), and a minimum recognition unit of the antibody. The above antigen-binding fragment can bind to the same antigen as a parent antibody.

In the present disclosure, the term "Fab" of the antibody refers to a portion of the antibody that is formed by binding the variable regions of the single light chain (including the variable region and the constant region) and single heavy chain to a first constant region via a disulfide bond. The "Fab' fragment" refers to a Fab fragment that includes partial hinge region. The "F(ab')₂" refers to a dimer of Fab'. The "Fv fragment" is formed by binding the variable region of the single light chain and/or the variable region of the single heavy chain. The "single-chain Fv antibody" or "scFv" refers to an antibody fragment formed by directly connecting the variable region of the light chain and the variable region of the heavy chain or via a peptide adapter sequence. The "minimum recognition unit of the antibody" refers to a structure containing only a single CDR in the variable region. Although the minimum recognition unit has low molecular weight and affinity, it retains the ability to bind to the antigen.

A CDR boundary of the antibody or the antigen-binding fragment thereof in the present disclosure may be defined or identified according to IMGT, Kabat, Chothia, AbM, or Contact definition manners. The CDRs defined by other manners acceptable in the art are also within the scope of protection of the present disclosure (Kaas, Q et. al. IMGT unique numbering for immunoglobulin and T cell receptor constant domains and Ig super family C-like domains.Dev.Comp.Immunol.29,185-203,(2005); R.M. MacCallum et. al. Antibody-antigen interactions: contact analysis and binding site topography J.Mol.Biol. (1996); Martin, A.C.R. Protein sequence and structure analysis of antibody variable domains (Book chapter). In Antibody engineering lab manual Eds. Duebel, S. and Kontermann, R.(2001); Marie-Paule Lefranc et. al. IMGT unique numbering for immunoglobulin and T cell receptor variabledomains and Ig superfamily V-like domains, Developmental and Comparative Immunology 27(2003)55-77).

In the present disclosure, the term "amino acid" refers to a naturally-existing amino acid and a synthetic amino acid, as well as an amino acid analogue and amino acid mimic that function in a manner similar to the naturally-existing amino acid. The naturally-existing amino acid includes an amino acid encoded by a genetic code and an amino acid modified by the same, such as hydroxyproline, γ-carboxyglutamic acid, and O-phosphoserine. Common naturally-existing amino acids include Alanine (Ala; A), Arginine (Arg; R), Asparagine (Asn; N), Aspartic acid (Asp; D), Cysteine (Cys; C); Glutamic acid (Glu; E), Glutamine (Gln; Q), Glycine (Gly; G); Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V). The amino acid analogue refers to a compound having the same basic chemical structure as the naturally-existing amino acid (i.e., α-carbon bonded to hydrogen, carboxyl, amino, and an R group), such as homoserine, L-leucine, methionine sulfoxide, and methionine methylsulfonium. The amino acid analogue typically has a modified R-group (e.g., norleucine) or a modified peptide backbone, but retains the same basic chemical structure as the naturally-existing amino acid. The amino acid mimic refers to a chemical compound that has a structure differing from a general chemical structure of amino acids, but functions in a manner similar to the naturally-existing amino acid. In the present disclosure, the term "identity" percentage refers to the degree to which amino acids at equivalent positions in two polypeptides are identical when the two sequences are optimally aligned. The alignment of the identity percentages of the amino acid sequences may be determined using various methods known in the art, such as software commonly used in the art including BLAST, BLAST-2, ALIGN, MEGALIGN (DNASTAR), CLUSTALW, or CLUSTAL OMEGA, etc. Those skilled in the art may determine appropriate parameters for an aligned sequence, including any algorithms required to achieve maximum alignment of the full length of a comparison sequence.

In the present disclosure, the term "specific binding" or "specifically bind" refers to a non-random binding reaction between two molecules, for example, a reaction between an antibody and an antigen. In some specific implementations, for example, determination is performed according to a flow cytometry fluorescence sorting technology.

In the present disclosure, the term "anti-CDH17 antibody" refers to an antibody capable of specifically binding to CDH17. In some specific implementations, the "anti-CDH17 antibody" specifically binds to human and/or monkey CDH17.

In the present disclosure, the term "affinity" refers to the strength of a non-covalent interaction between an immunoglobulin molecule (i.e., an antibody) or a fragment thereof and an antigen. The strength or affinity of an immune binding interaction may be expressed by an equilibrium dissociation constant (KD) of the interaction, where if a KD value is smaller, it indicates the affinity is higher. The KD may be determined using any conventional method known in the art, including but not limited to a Biacore determination method, an Octet method, microcalorimetry, an HPLC-MS method, and the flow cytometry fluorescence sorting technology.

The binding of the antibody or the antigen-binding fragment thereof provided in the present disclosure to the CDH17 may also be measured using a "half-maximal effective concentration (EC50)," which indicates a concentration of a drug or antibody required to achieve 50% of a maximum biological effect after a specific exposure time. Generally, if the EC50 is smaller, it indicates that the affinity is better, indicating that an antibody may bind to a target protein at a lower concentration. The EC50 value may be determined using binding assays known in the art, such as direct or indirect binding assays (e.g., an enzyme-linked immunosorbent assay (ELISA), a flow cytometry fluorescence sorting technology, and other binding assays).

In the present disclosure, the term "epitope" refers to any antigen determinant on an antigen that is bound by a complementary site of an antibody. The antigen determinant is typically a specific chemical group with certain compositions and structures. The epitope may be linear (i.e., continuous) or conformational (i.e., including spaced-apart amino acid residues, non-continuous). The epitope defines a minimum binding site for the antibody, making it a specific target for the antibody or the antigen-binding fragment thereof. The epitope may be determined by any method known in the art, such as a conventional immunoassay, an antibody competitive binding assay, or X-ray crystallography or related structural determination methods (e.g., nuclear magnetic resonance spectroscopy).

In the present disclosure, the term "polyspecific antibody" refers to an antibody molecule capable of binding to a plurality of (two or more) different antigenic epitopes of the same antigen or a plurality of (two or more) different antigens.

The "Chimeric Antigen Receptor (CAR)" in the present disclosure is an artificial receptor that mimics a TCR function. It consists of an extracellular domain, a transmembrane domain, and an intracellular signaling domain connected in sequence. When the antigen (receptors) on a tumor cell surface binds to the antibody (ligand) portion of the CAR, a signal may be transmitted intracellularly via a hinge region and a transmembrane region. The intracellular signaling domain then converts the signal into an activation signal to activate an effector cell. The effector cell kills the tumor cell by secreting perforin or producing cytokines, while simultaneously undergoing proliferation themselves, thereby expanding an immune killing effect. The ECD is typically composed of a single-chain variable fragment (scFv) of a monoclonal antibody responsible for recognizing and binding the antigen, along with a hinge region that serves as a connecting segment. The single-chain variable region is the antigen-binding domain of CAR, and the domain determines the specificity and function of CAR-immune cells. The hinge region is the CAR ECD that connects the single-chain variable fragment to the transmembrane domain. The hinge region typically maintains the stability required for robust CAR expression and activity in the effector cells. The hinge region of most CARs is derived from a hinge of IgG or an ECD of CD8α/CD28. The type and length of the hinge region significantly affect the functional activity of the CAR. The transmembrane domain connects the ECD of the CAR to the intracellular signal transduction domain. Commonly-used transmembrane domains are from CD4, CD8, CD28, and CD3ζ, or derivatives thereof. The selection of the transmembrane domains affects an activation degree of a CAR structure in a cell function. The intracellular domain is composed of a co-stimulatory domain and a signal transduction domain. The co-stimulatory domain is typically from a CD28 receptor family (CD28, ICOS) or a tumor necrosis factor receptor family (4-1BB, OX40, CD27). The signal transduction domain is typically a T-cell receptor TCR/CD3ζ chain or an immunoglobulin Fc receptor Fcε RIγ chain.

The CAR of the present disclosure (including its functional portions and functional variants) may be obtained by methods known in the art, such as by any suitable method for preparing polypeptides or proteins.

In the present disclosure, the term "engineered immune cell" refers to an immune cell expressing the CAR or an immune cell modified by the CAR. The immune cell includes, but is not limited to, a T cell (e.g., a T cell of α/β and a T cell of γ/δ), a natural killer cell (NK cell), a monocyte, a macrophage, a Natural killer T (NKT) cell, a dendritic cell, a granulocyte, a B cell, a lymphocyte, a leukocyte, and/or a peripheral blood mononuclear cell.

In the present disclosure, the term "nucleic acid molecule" refers to a polymeric form of nucleotides of any length, including ribonucleotides and/or deoxyribonucleotides. Examples of the nucleic acid molecule include, but are not limited to, single-stranded, double-stranded, or multi-stranded DNA or RNA; genomic DNA; cDNA; DNA-RNA hybrids; or polymers containing purine and pyrimidine bases or other naturally-existing, chemically or biochemically modified, non-natural, or derived nucleotide bases. When the nucleic acid molecule encodes a protein or polypeptide, the encoding may optionally be for a sense strand or an antisense strand. The nucleic acid molecule may be naturally-existing, synthetic, recombinant, or any combination thereof. The "nucleic acid molecule," "nucleic acid," and "polynucleotide" are interchangeable.

In an optional implementation, the nucleic acid molecule is RNA or DNA, and the nucleic acid molecule may be single-stranded or double-stranded DNA, preferably the double-stranded DNA. The nucleic acid molecule is "effectively connected" when being placed in a functional relationship with another nucleic acid sequence. For example, if a promoter or an enhancer affects the transcription of an encoding sequence, the promoter or the enhancer is effectively linked to the encoding sequence. The nucleic acid preferably uses DNA when being linked into a vector.

In the present disclosure, the term "vector" refers to a carrier capable of operatively inserting a genetic element (e.g., the foregoing nucleic acid molecule) and causing the expression of the genetic element, such as producing the protein, RNA, or DNA encoded by the genetic element, or replicating the genetic element. The vector may be used to transform, transduce, or transfect host cells, causing the genetic elements carried by the vector to be expressed in the host cells. For example, the vector includes: a plasmid; a phagemid; a cosmid; an artificial chromosome, such as a Yeast Artificial Chromosome (YAC), a Bacteriophage Artificial Chromosome (BAC) or a P1-derived Artificial chromosome (PAC); a phage, such as a λ phage or an M13 phage; and animal viruses, etc. The vector may include a plurality of elements for controlling expression, including a promoter sequence, a transcription initiation sequence, an enhancer sequence, a selection element, and a reporter gene. Furthermore, the vector may also include a replication origin site. The vector may also include components that facilitate its entry into cells, including but not limited to viral particles, liposomes, or protein coats. The vector may be an expression vector or a cloning vector. In some implementation solutions, the vector (e.g., expression vector) provided in the present disclosure includes the nucleic acid sequence encoding the antibody or the antigen-binding fragment thereof of the present disclosure, at least one promoter (e.g., SV40, CMV, or EF-1α) capable of being operatively connected to the nucleic acid sequence, and at least one selective marker.

In the present disclosure, the term "purified" or "isolated" associated with polypeptides or nucleic acids refers to the fact that the polypeptides or nucleic acids are not in their natural medium or natural form. Therefore, the term "isolated" includes polypeptides or nucleic acids removed from their original environment, such as if they are naturally-existing, from their natural environment. In association with the nucleic acids, the term "isolated or purified" indicates that the nucleic acid is not in its natural genomic context (e.g., in a vector, as an expression cassette, linked to a promoter, or artificially-introduced into a heterologous host cell).

In the present disclosure, the term "recombinant cell" refers to a cell that may be or has been introduced with exogenous polynucleotides and/or a vector. The exogenous polynucleotides may or may not be integrated into the genome of the "recombinant cell." When the recombinant cell includes a vector, the vector may be introduced into a mammalian cell to construct and obtain the recombinant cell. These recombinant cells are then used to express the antibody or the antigen-binding fragment provided in the present disclosure. The recombinant cells are cultured, such that the corresponding antibodies may be obtained. The available mammalian cells include CHO cells, and the like.

In the present disclosure, the term "pharmaceutical composition" exists in a form that allows the biological activity of active ingredients to be effective, and does not include additional ingredients that are unacceptably toxic to a subject which is about to apply the composition. In some specific implementations, the antibody contained in or expressed by the above pharmaceutical composition can specifically target and bind to the CDH17.

In the present disclosure, the term "binding CDH17" includes binding to isolated CDH17 molecules and/or cells expressing the CDH17.

In the present disclosure, the term "pharmaceutically acceptable carrier" may include any physiologically compatible solvents, dispersion media, coatings, antibacterial agents and antifungal agents, isotonic agents, delayed absorption agents, etc.

In the present disclosure, the term "subject" or "patient" refers to a mammalian subject or patient. Exemplary subjects include, but are not limited to, humans, monkeys, dogs, cats, mice, rats, cattle, horses, camels, poultry, goats, and sheep. In some implementation solutions, the subject is a human. In some implementation solutions, the subject is an individual suspected of having cancer, autoimmune diseases or conditions, and/or infections.

In the present disclosure, the term "diagnosis" refers to the identification of a pathological state, disease, or condition, such as the identification of CDH17-related diseases, or the identification of subjects with CDH17-related diseases who may benefit from specific treatment regimens. In some implementations, the diagnosis includes identifying abnormal levels or activity of the CDH17. In some implementations, diagnosis refers to identifying cancer or autoimmune diseases in the subjects.

In the present disclosure, the "CDH17-related" diseases, symptoms, or conditions refer to any disease or condition caused by, exacerbated by, or associated with increased or decreased expression or activity of the CDH17. In some implementations, the CDH17-related diseases, symptoms, or conditions are symptoms associated with excessive cell proliferation, such as cancer. In some implementations, the CDH17-related diseases or conditions are characterized by the expression or overexpression of CDH17 and/or CDH17-related genes.

In the present disclosure, the term "CDH17-expressing" cancer refers to a cancer in which the CDH17 is expressed in cancer cells or tumor-infiltrating immune cells or immunosuppressive cells, and the level of CDH17 expression in the cancer cells or tumor-infiltrating immune cells or immunosuppressive cells is significantly higher than that expected in normal cells.

In the present disclosure, the term "effective amount" refers to a therapeutic amount sufficient to reduce or improve the severity and/or duration of a disease or one or more of symptoms thereof; prevent disease progression; induce disease remission; prevent the recurrence, development, or progression of one or more disease-related symptoms; detect diseases; or enhance or improve the preventive or treatment effects of another therapy (e.g., a preventive agent or a therapeutic agent). The therapeutically effective dosage of the antibody or the antigen-binding fragment thereof of the present disclosure depends on various factors known in the art, such as body weight, age, past medical history, current treatments, the health status of the subject and potential for cross-infection, allergies, hypersensitivity, and side effects, as well as administration routes and the extent of tumor progression. Those skilled in the art (e.g., a physician or veterinarian) may proportionally reduce or increase the dosage based on these or other conditions or requirements.

A first aspect provides an anti-CDH17 antibody or an antigen-binding fragment thereof. The anti-CDH17 antibody or the antigen-binding fragment thereof binds to one or more of a CDH17 ECD 1 (D1), a CDH17 ECD 2 (D2), a CDH17 ECD 3 (D3), a CDH17 ECD 4 (D4), a CDH17 ECD 5 (D5), a CDH17 ECD 6 (D6), and a CDH17 ECD 7 (D7).

In an optional implementation, an extracellular region amino acid sequence of CDH17 is shown in SEQ ID NO: 597.

MILQAHLHSLCLLMLYLATGYGQEGKFSGPLKPMTFSIYEGQEPSQIIFQFKANPPAVTFELTG
ETDNIFVIEREGLLYYNRALDRETRSTHNLQVAALDANGIIVEGPVPITIKVKDINDNRPTFLQSKYEG
SVRQNSRPGKPFLYVNATDLDDPATPNGQLYYQIVIQLPMINNVMYFQINNKTGAISLTREGSQELN
PAKNPSYNLVISVKDMGGQSENSFSDTTSVDIIVTENIWKAPKPVEMVENSTDPHPIKITQVRWNDP
GAQYSLVDKEKLPRFPFSIDQEGDIYVTQPLDREEKDAYVFYAVAKDEYGKPLSYPLEIHVKVKDIN
DNPPTCPSPVTVFEVQENERLGNSIGTLTAHDRDEENTANSFLNYRIVEQTPKLPMDGLFLIQTYAG
MLQLAKQSLKKQDTPQYNLTIEVSDKDFKTLCFVQINVIDINDQIPIFEKSDYGNLTLAEDTNIGSTILT
IQATDADEPFTGSSKILYHIIKGDSEGRLGVDTDPHTNTGYVIIKKPLDFETAAVSNIVFKAENPEPLV
FGVKYNASSFAKFTLIVTDVNEAPQFSQHVFQAKVSEDVAIGTKVGNVTAKDPEGLDISYSLRGDT
RGWLKIDHVTGEIFSVAPLDREAGSPYRVQVVATEVGGSSLSSVSEFHLILMDVNDNPPRLAKDYT
GLFFCHPLSAPGSLIFEATDDDQHLFRGPHFTFSLGSGSLQNDWEVSKINGTHARLSTRHTEFEER
EYVVLIRINDGGRPPLEGIVSLPVTFCSCVEGSCFRPAGHQTGIPTVGM(SEQ ID NO.597).

In an optional implementation, an amino acid sequence of the ECD 1 (D1) is shown as amino acids 30-128 from an N terminal to a C terminal of the CDH17.

In an optional implementation, an amino acid sequence of the ECD 2 (D2) is shown as amino acids 129-244 from an N terminal to a C terminal of the CDH17.

In an optional implementation, an amino acid sequence of the ECD 3 (D3) is shown as amino acids 245-340 from an N terminal to a C terminal of the CDH17.

In an optional implementation, an amino acid sequence of the ECD 4 (D4) is shown as amino acids 341-449 from an N terminal to a C terminal of the CDH17.

In an optional implementation, an amino acid sequence of the ECD 5 (D5) is shown as amino acids 450-566 from an N terminal to a C terminal of the CDH17.

In an optional implementation, an amino acid sequence of the ECD 6 (D6) is shown as amino acids 567-667 from an N terminal to a C terminal of the CDH17.

In an optional implementation, an amino acid sequence of the ECD 7 (D7) is shown as amino acids 668-777 from an N terminal to a C terminal of the CDH17.

In an optional implementation, the anti-CDH17 antibody or the antigen-binding fragment thereof binds to one or more of a CDH17 ECD 1 (D1), a CDH17 ECD 2 (D2), a CDH17 ECD 3 (D3), a CDH17 ECD 4 (D4), a CDH17 ECD 5 (D5), a CDH17 ECD 6 (D6), and a CDH17 ECD 7 (D7).

In an optional implementation, the anti-CDH17 antibody or the antigen-binding fragment thereof binds to one or more of the CDH17 ECD 1 (D1), the CDH17 ECD 2 (D2), the CDH17 ECD 3 (D3), and the CDH17 ECD 4 (D4).

In an optional implementation, the anti-CDH17 antibody or the antigen-binding fragment thereof binds to one or more of the CDH17 ECD 5 (D5), the CDH17 ECD 6 (D6), and the CDH17 ECD 7 (D7).

In an optional implementation, the antibody or the antigen-binding fragment thereof includes a CDR of the heavy chain variable region and/or a CDR of the light chain variable region.

The CDR of the heavy chain variable region includes HCDR1, HCDR2, and HCDR3, and the CDR of the light chain variable region includes LCDR1, LCDR2, and LCDR3.

The HCDR1 includes an amino acid sequence of DY, an amino acid sequence of EY, an amino acid sequence of GY, an amino acid sequence of NY, an amino acid sequence of SG, an amino acid sequence of SH, an amino acid sequence of SY, an amino acid sequence of TS, or an amino acid sequence of TY.

In an optional implementation, according to an IMGT definition manner, the HCDR1 includes an amino acid sequence shown in any one of SEQ ID NOs: 45, 50, 54, 58, 63, 67, 72, 77, 81, 85, 90, 95, 100, 104, 107, and 113.

The HCDR2 includes an amino acid sequence shown in any one of SEQ ID NOs: 184-186, 188, 191-199, 202-205, and 208.

In an optional implementation, according to the IMGT definition manner, the HCDR2 includes an amino acid sequence shown in any one of SEQ ID NO: 121, 125, 129, 133, 137, 141, 145, 149, 153, 157, 162, 167, 171, 175, 179, 183, 189, and 190.

The HCDR3 includes an amino acid sequence shown in any one of SEQ ID NO: 209, 212, 215, 218, 221, 224, 227, 230, 233, 236, 239, 242, 245, 248, 251, 254, 257, 260, and 278.

In an optional implementation, according to the IMGT definition manner, the HCDR3 includes an amino acid sequence shown in any one of SEQ ID NO: 211, 214, 217, 220, 223, 226, 229, 232, 235, 238, 241, 244, 247, 250, 253, 256, 259, 262, and 266.

The LCDR1 includes an amino acid sequence of NID, an amino acid sequence of NNF, an amino acid sequence of NNW, an amino acid sequence of NY, an amino acid sequence of SDF, an amino acid sequence of SGY, an amino acid sequence of SND, an amino acid sequence of SSA, or an amino acid sequence of SY, or an amino acid sequence shown in any one of SEQ ID NOs: 290, 294, 299, 308, 319, and 332.

In an optional implementation, according to the IMGT definition manner, the LCDR1 includes an amino acid sequence shown in any one of SEQ ID NOs: 286, 288, 292, 293, 296, 298, 301, 303, 305, 307, 310, 312, 321, 322, and 333.

The LCDR2 includes an amino acid sequence of AA, an amino acid sequence of DT, an amino acid sequence of FA, an amino acid sequence of GA, an amino acid sequence of KV, an amino acid sequence of RA, an amino acid sequence of RT, an amino acid sequence of SA, an amino acid sequence of ST, an amino acid sequence of WA, an amino acid sequence of YA, or an amino acid sequence of YT.

In an optional implementation, according to the IMGT definition manner, the LCDR2 includes an amino acid sequence of AA, an amino acid sequence of DT, an amino acid sequence of FA, an amino acid sequence of GA, an amino acid sequence of KV, an amino acid sequence of RA, an amino acid sequence of RT, an amino acid sequence of SA, an amino acid sequence of ST, an amino acid sequence of WA, an amino acid sequence of YA, or an amino acid sequence of YT.

The LCDR3 comprises an amino acid sequence shown in any one of SEQ ID NOs: 368, 370, 372, 374, 376, 378, 380, 382, 384, 386, 388, 390, 392, 394, and 396.

In an optional implementation, according to the IMGT definition manner, the LCDR3 includes an amino acid sequence shown in any one of SEQ ID NOs: 369, 371, 373, 375, 377, 379, 381, 383, 385, 387, 389, 391, 393, 395, and 397.

In an optional implementation, the CDR of the anti-CDH17 antibody or an antigen-binding fragment includes any one of the following (a)-(t).
(a) The HCDR1 includes the amino acid sequence of SY, the HCDR2 includes an amino acid sequence shown in SEQ ID NO: 185, the HCDR3 includes an amino acid sequence shown in SEQ ID NO: 209, the LCDR1 includes the amino acid sequence of SND, the LCDR2 includes the amino acid sequence of YA, and the LCDR3 includes an amino acid sequence shown in SEQ ID NO: 386; or
(b) The HCDR1 includes the amino acid sequence of EY, the HCDR2 includes an amino acid sequence shown in SEQ ID NO: 192, the HCDR3 includes an amino acid sequence shown in SEQ ID NO: 278, the LCDR1 includes an amino acid sequence shown in SEQ ID NO: 319, the LCDR2 includes the amino acid sequence of WA, and the LCDR3 includes an amino acid sequence shown in SEQ ID NO: 376; or
(c) The HCDR1 includes the amino acid sequence of SY, the HCDR2 includes an amino acid sequence shown in SEQ ID NO: 203, the HCDR3 includes an amino acid sequence shown in SEQ ID NO: 212, the LCDR1 includes the amino acid sequence of SND, the LCDR2 includes the amino acid sequence of YA, and the LCDR3 includes an amino acid sequence shown in SEQ ID NO: 378; or
(d) The HCDR1 includes the amino acid sequence of SY, the HCDR2 includes an amino acid sequence shown in SEQ ID NO: 196, the HCDR3 includes an amino acid sequence shown in SEQ ID NO: 215, the LCDR1 includes the amino acid sequence of SY, the LCDR2 includes the amino acid sequence of DT, and the LCDR3 includes an amino acid sequence shown in SEQ ID NO: 372; or
(e) The HCDR1 includes the amino acid sequence of GY, the HCDR2 includes an amino acid sequence shown in SEQ ID NO: 197, the HCDR3 includes an amino acid sequence shown in SEQ ID NO: 218, the LCDR1 includes an amino acid sequence shown in SEQ ID NO: 332, the LCDR2 includes the amino acid sequence of ST, and the LCDR3 includes an amino acid sequence shown in SEQ ID NO: 374; or
(f) The HCDR1 includes the amino acid sequence of SH, the HCDR2 includes an amino acid sequence shown in SEQ ID NO: 208, the HCDR3 includes an amino acid sequence shown in SEQ ID NO: 221, the LCDR1 includes an amino acid sequence shown in SEQ ID NO: 290, the LCDR2 includes the amino acid sequence of RA, and the LCDR3 includes an amino acid sequence shown in SEQ ID NO: 394; or
(g) The HCDR1 includes the amino acid sequence of TY, the HCDR2 includes an amino acid sequence shown in SEQ ID NO: 191, the HCDR3 includes an amino acid sequence shown in SEQ ID NO: 224, the LCDR1 includes an amino acid sequence shown in SEQ ID NO: 332, the LCDR2 includes the amino acid sequence of ST, and the LCDR3 includes an amino acid sequence shown in SEQ ID NO: 374; or
(h) The HCDR1 includes the amino acid sequence of GY, the HCDR2 includes an amino acid sequence shown in SEQ ID NO: 195, the HCDR3 includes an amino acid sequence shown in SEQ ID NO: 227, the LCDR1 includes an amino acid sequence shown in SEQ ID NO: 294, the LCDR2 includes the amino acid sequence of YA, and the LCDR3 includes an amino acid sequence shown in SEQ ID NO: 382; or
(i) The HCDR1 includes the amino acid sequence of SG, the HCDR2 includes an amino acid sequence shown in SEQ ID NO: 205, the HCDR3 includes an amino acid sequence shown in SEQ ID NO: 230, the LCDR1 includes the amino acid sequence of SSA, the LCDR2 includes the amino acid sequence of SA, and the LCDR3 includes an amino acid sequence shown in SEQ ID NO: 392; or
(j) The HCDR1 includes the amino acid sequence of TS, the HCDR2 includes an amino acid sequence shown in SEQ ID NO: 186, the HCDR3 includes an amino acid sequence shown in SEQ ID NO: 233, the LCDR1 includes an amino acid sequence shown in SEQ ID NO: 299, the LCDR2 includes the amino acid sequence of KV, and the LCDR3 includes an amino acid sequence shown in SEQ ID NO: 370; or
(k) The HCDR1 includes the amino acid sequence of EY, the HCDR2 includes an amino acid sequence shown in SEQ ID NO: 194, the HCDR3 includes an amino acid sequence shown in SEQ ID NO: 278, the LCDR1 includes an amino acid sequence shown in SEQ ID NO: 319, the LCDR2 includes the amino acid sequence of WA, and the LCDR3 includes an amino acid sequence shown in SEQ ID NO: 376; or
(l) The HCDR1 includes the amino acid sequence of SY, the HCDR2 includes an amino acid sequence shown in SEQ ID NO: 199, the HCDR3 includes an amino acid sequence shown in SEQ ID NO: 236, the LCDR1 includes the amino acid sequence of SY, the LCDR2 includes the amino acid sequence of DT, and the LCDR3 includes an amino acid sequence shown in SEQ ID NO: 368; or
(m) The HCDR1 includes the amino acid sequence of EY, the HCDR2 includes an amino acid sequence shown in SEQ ID NO: 194, the HCDR3 includes an amino acid sequence shown in SEQ ID NO: 239, the LCDR1 includes an amino acid sequence shown in SEQ ID NO: 319, the LCDR2 includes the amino acid sequence of WA, and the LCDR3 includes an amino acid sequence shown in SEQ ID NO: 376; or
(n) The HCDR1 includes the amino acid sequence of DY, the HCDR2 includes an amino acid sequence shown in SEQ ID NO: 193, the HCDR3 includes an amino acid sequence shown in SEQ ID NO: 242, the LCDR1 includes the amino acid sequence of NNW, the LCDR2 includes the amino acid sequence of GA, and the LCDR3 includes an amino acid sequence shown in SEQ ID NO: 396; or
(o) The HCDR1 includes the amino acid sequence of TY, the HCDR2 includes an amino acid sequence shown in SEQ ID NO: 202, the HCDR3 includes an amino acid sequence shown in SEQ ID NO: 245, the LCDR1 includes the amino acid sequence of NY, the LCDR2 includes the amino acid sequence of YT, and the LCDR3 includes an amino acid sequence shown in SEQ ID NO: 388; or
(p) The HCDR1 includes the amino acid sequence of NY, the HCDR2 includes an amino acid sequence shown in SEQ ID NO: 198, the HCDR3 includes an amino acid sequence shown in SEQ ID NO: 248, the LCDR1 includes the amino acid sequence of SDF, the LCDR2 includes the amino acid sequence of YA, and the LCDR3 includes an amino acid sequence shown in SEQ ID NO: 384; or
(q) The HCDR1 includes the amino acid sequence of TY, the HCDR2 includes an amino acid sequence shown in SEQ ID NO: 204, the HCDR3 includes an amino acid sequence shown in SEQ ID NO: 251, the LCDR1 includes the amino acid sequence of SGY, the LCDR2 includes the amino acid sequence of AA, and the LCDR3 includes an amino acid sequence shown in SEQ ID NO: 380; or
(r) The HCDR1 includes the amino acid sequence of TY, the HCDR2 includes an amino acid sequence shown in SEQ ID NO: 204, the HCDR3 includes an amino acid sequence shown in SEQ ID NO: 254, the LCDR1 includes the amino acid sequence of SGY, the LCDR2 includes the amino acid sequence of AA, and the LCDR3 includes an amino acid sequence shown in SEQ ID NO: 380; or
(s) The HCDR1 includes the amino acid sequence of NY, the HCDR2 includes an amino acid sequence shown in SEQ ID NO: 184, the HCDR3 includes an amino acid sequence shown in SEQ ID NO: 257, the LCDR1 includes an amino acid sequence shown in SEQ ID NO: 308, the LCDR2 includes the amino acid sequence of FA, and the LCDR3 includes an amino acid sequence shown in SEQ ID NO: 382; or
(t) The HCDR1 includes the amino acid sequence of DY, the HCDR2 includes an amino acid sequence shown in SEQ ID NO: 188, the HCDR3 includes an amino acid sequence shown in SEQ ID NO: 260, the LCDR1 includes the amino acid sequence of NID, the LCDR2 includes the amino acid sequence of SA, and the LCDR3 includes an amino acid sequence shown in SEQ ID NO: 390.

In an optional implementation, according to the IMGT definition manner, the CDR of the anti-CDH17 antibody or an antigen-binding fragment includes any one of the following (a')-(t').
(a') The HCDR1 includes an amino acid sequence shown in SEQ ID NO: 45, the HCDR2 includes an amino acid sequence shown in SEQ ID NO: 121, the HCDR3 includes an amino acid sequence shown in SEQ ID NO: 211, the LCDR1 includes an amino acid sequence shown in SEQ ID NO: 286, the LCDR2 includes the amino acid sequence of YA, and the LCDR3 includes an amino acid sequence shown in SEQ ID NO: 387; or
(b') The HCDR1 includes an amino acid sequence shown in SEQ ID NO: 113, the HCDR2 includes an amino acid sequence shown in SEQ ID NO: 125, the HCDR3 includes an amino acid sequence shown in SEQ ID NO: 266, the LCDR1 includes an amino acid sequence shown in SEQ ID NO: 322, the LCDR2 includes the amino acid sequence of WA, and the LCDR3 includes an amino acid sequence shown in SEQ ID NO: 377; or
(c') The HCDR1 includes an amino acid sequence shown in SEQ ID NO: 50, the HCDR2 includes an amino acid sequence shown in SEQ ID NO: 129, the HCDR3 includes an amino acid sequence shown in SEQ ID NO: 214, the LCDR1 includes an amino acid sequence shown in SEQ ID NO: 288, the LCDR2 includes the amino acid sequence of RT, and the LCDR3 includes an amino acid sequence shown in SEQ ID NO: 379; or
(d') The HCDR1 includes an amino acid sequence shown in SEQ ID NO: 54, the HCDR2 includes an amino acid sequence shown in SEQ ID NO: 133, the HCDR3 includes an amino acid sequence shown in SEQ ID NO: 217, the LCDR1 includes an amino acid sequence shown in SEQ ID NO: 333, the LCDR2 includes the amino acid sequence of DT, and the LCDR3 includes an amino acid sequence shown in SEQ ID NO: 373; or
(e') The HCDR1 includes an amino acid sequence shown in SEQ ID NO: 58, the HCDR2 includes an amino acid sequence shown in SEQ ID NO: 137, the HCDR3 includes an amino acid sequence shown in SEQ ID NO: 220, the LCDR1 includes an amino acid sequence shown in SEQ ID NO: 293, the LCDR2 includes the amino acid sequence of ST, and the LCDR3 includes an amino acid sequence shown in SEQ ID NO: 375; or
(f') The HCDR1 includes an amino acid sequence shown in SEQ ID NO: 63, the HCDR2 includes an amino acid sequence shown in SEQ ID NO: 141, the HCDR3 includes an amino acid sequence shown in SEQ ID NO: 223, the LCDR1 includes an amino acid sequence shown in SEQ ID NO: 292, the LCDR2 includes the amino acid sequence of RA, and the LCDR3 includes an amino acid sequence shown in SEQ ID NO: 395; or
(g') The HCDR1 includes an amino acid sequence shown in SEQ ID NO: 107, the HCDR2 includes an amino acid sequence shown in SEQ ID NO: 145, the HCDR3 includes an amino acid sequence shown in SEQ ID NO: 226, the LCDR1 includes an amino acid sequence shown in SEQ ID NO: 293, the LCDR2 includes the amino acid sequence of ST, and the LCDR3 includes an amino acid sequence shown in SEQ ID NO: 375; or
(h') The HCDR1 includes an amino acid sequence shown in SEQ ID NO: 67, the HCDR2 includes an amino acid sequence shown in SEQ ID NO: 149, the HCDR3 includes an amino acid sequence shown in SEQ ID NO: 229, the LCDR1 includes an amino acid sequence shown in SEQ ID NO: 296, the LCDR2 includes the amino acid sequence of YA, and the LCDR3 includes an amino acid sequence shown in SEQ ID NO: 383; or
(i') The HCDR1 includes an amino acid sequence shown in SEQ ID NO: 72, the HCDR2 includes an amino acid sequence shown in SEQ ID NO: 153, the HCDR3 includes an amino acid sequence shown in SEQ ID NO: 232, the LCDR1 includes an amino acid sequence shown in SEQ ID NO: 298, the LCDR2 includes the amino acid sequence of SA, and the LCDR3 includes an amino acid sequence shown in SEQ ID NO: 393; or
(j') The HCDR1 includes an amino acid sequence shown in SEQ ID NO: 77, the HCDR2 includes an amino acid sequence shown in SEQ ID NO: 157, the HCDR3 includes an amino acid sequence shown in SEQ ID NO: 235, the LCDR1 includes an amino acid sequence shown in SEQ ID NO: 301, the LCDR2 includes the amino acid sequence of KV, and the LCDR3 includes an amino acid sequence shown in SEQ ID NO: 371; or
(k') The HCDR1 includes an amino acid sequence shown in SEQ ID NO: 113, the HCDR2 includes an amino acid sequence shown in SEQ ID NO: 189, the HCDR3 includes an amino acid sequence shown in SEQ ID NO: 266, the LCDR1 includes an amino acid sequence shown in SEQ ID NO: 322, the LCDR2 includes the amino acid sequence of WA, and the LCDR3 includes an amino acid sequence shown in SEQ ID NO: 377; or
(l') The HCDR1 includes an amino acid sequence shown in SEQ ID NO: 81, the HCDR2 includes an amino acid sequence shown in SEQ ID NO: 162, the HCDR3 includes an amino acid sequence shown in SEQ ID NO: 238, the LCDR1 includes an amino acid sequence shown in SEQ ID NO: 333, the LCDR2 includes the amino acid sequence of DT, and the LCDR3 includes an amino acid sequence shown in SEQ ID NO: 369; or
(m') The HCDR1 includes an amino acid sequence shown in SEQ ID NO: 113, the HCDR2 includes an amino acid sequence shown in SEQ ID NO: 189, the HCDR3 includes an amino acid sequence shown in SEQ ID NO: 241, the LCDR1 includes an amino acid sequence shown in SEQ ID NO: 322, the LCDR2 includes the amino acid sequence of WA, and the LCDR3 includes an amino acid sequence shown in SEQ ID NO: 377; or
(n') The HCDR1 includes an amino acid sequence shown in SEQ ID NO: 85, the HCDR2 includes an amino acid sequence shown in SEQ ID NO: 167, the HCDR3 includes an amino acid sequence shown in SEQ ID NO: 244, the LCDR1 includes an amino acid sequence shown in SEQ ID NO: 303, the LCDR2 includes the amino acid sequence of GA, and the LCDR3 includes an amino acid sequence shown in SEQ ID NO: 397; or
(o') The HCDR1 includes an amino acid sequence shown in SEQ ID NO: 90, the HCDR2 includes an amino acid sequence shown in SEQ ID NO: 171, the HCDR3 includes an amino acid sequence shown in SEQ ID NO: 247, the LCDR1 includes an amino acid sequence shown in SEQ ID NO: 305, the LCDR2 includes the amino acid sequence of YT, and the LCDR3 includes an amino acid sequence shown in SEQ ID NO: 389; or
(p') The HCDR1 includes an amino acid sequence shown in SEQ ID NO: 95, the HCDR2 includes an amino acid sequence shown in SEQ ID NO: 175, the HCDR3 includes an amino acid sequence shown in SEQ ID NO: 250, the LCDR1 includes an amino acid sequence shown in SEQ ID NO: 307, the LCDR2 includes the amino acid sequence of YA, and the LCDR3 includes an amino acid sequence shown in SEQ ID NO: 385; or
(q') The HCDR1 includes an amino acid sequence shown in SEQ ID NO: 107, the HCDR2 includes an amino acid sequence shown in SEQ ID NO: 190, the HCDR3 includes an amino acid sequence shown in SEQ ID NO: 253, the LCDR1 includes an amino acid sequence shown in SEQ ID NO: 321, the LCDR2 includes the amino acid sequence of AA, and the LCDR3 includes an amino acid sequence shown in SEQ ID NO: 381; or
(r') The HCDR1 includes an amino acid sequence shown in SEQ ID NO: 107, the HCDR2 includes an amino acid sequence shown in SEQ ID NO: 190, the HCDR3 includes an amino acid sequence shown in SEQ ID NO: 256, the LCDR1 includes an amino acid sequence shown in SEQ ID NO: 321, the LCDR2 includes the amino acid sequence of AA, and the LCDR3 includes an amino acid sequence shown in SEQ ID NO: 381; or
(s') The HCDR1 includes an amino acid sequence shown in SEQ ID NO: 100, the HCDR2 includes an amino acid sequence shown in SEQ ID NO: 179, the HCDR3 includes an amino acid sequence shown in SEQ ID NO: 259, the LCDR1 includes an amino acid sequence shown in SEQ ID NO: 310, the LCDR2 includes the amino acid sequence of FA, and the LCDR3 includes an amino acid sequence shown in SEQ ID NO: 383; or
(t') The HCDR1 includes an amino acid sequence shown in SEQ ID NO: 104, the HCDR2 includes an amino acid sequence shown in SEQ ID NO: 183, the HCDR3 includes an amino acid sequence shown in SEQ ID NO: 262, the LCDR1 includes an amino acid sequence shown in SEQ ID NO: 312, the LCDR2 includes the amino acid sequence of SA, and the LCDR3 includes an amino acid sequence shown in SEQ ID NO: 391.

In an optional implementation, the anti-CDH17 antibody or the antigen-binding fragment thereof includes CDRs HCDR1, HCDR2, and HCDR3 of the heavy chain variable region and/or CDRs LCDR1, LCDR2, and LCDR3 of the light chain variable region.

The HCDR1, HCDR2, and HCDR3 include amino acid sequences consistent with those of HCDR1, HCDR2, and HCDR3 of a heavy chain variable region shown in any one of SEQ ID NOs: 1, 3, 5, 7, 9, 11, 13, 15, 17, 19, 21, 23, 25, 27, 29, 31, 33, 35, 37, and 39.

The LCDR1, LCDR2, and LCDR3 include amino acid sequences consistent with those of LCDR1, LCDR2, and LCDR3 of a light chain variable region shown in any one of SEQ ID NOs: 2, 4, 6, 8, 10, 12, 14, 16, 18, 20, 22, 24, 26, 28, 30, 32, 34, 36, 38, and 40.

The above HCDR1, HCDR2, and HCDR3, as well as the LCDR1, LCDR2, and LCDR3, are determined according to a Kabat definition manner, a Chothia definition manner, an AbM definition manner, a Contact definition manner, or an IMGT definition manner.

In an optional implementation, the HCDR1, HCDR2, HCDR3, LCDR1, LCDR2, and LCDR3 of the anti-CDH17 antibody or the antigen-binding fragment thereof may be independently selected from Tables 1-20, respectively.

Table 1 shows an exemplary antibody F1-41.16 (the amino acid sequence of the heavy chain variable region is shown in SEQ ID NO: 1, and the amino acid sequence of the light chain variable region is shown in SEQ ID NO: 2).

**Table 1**

| Definition manner | HCDR1 | HCDR2 | HCDR3 |
|---|---|---|---|
| Chothia | GYTFTSY (SEQ ID NO:41) | FPGDGS (SEQ ID NO:185) | AGGYNVFTY (SEQ ID NO:263) |
| AbM | GYTFTSYHIN (SEQ ID NO:42) | WIFPGDGSTE (SEQ ID NO:118) | AGGYNVFTY (SEQ ID NO:263) |
| Kabat | SYHIN (SEQ ID NO:43) | WIFPGDGSTEYNEKFKD (SEQ ID NO:119) | AGGYNVFTY (SEQ ID NO:263) |
| Contact | TSYHIN (SEQ ID NO:44) | WIGWIFPGDGSTE (SEQ ID NO:120) | TRAGGYNVFT (SEQ ID NO:210) |
| IMGT | GYTFTSYH (SEQ ID NO:45) | IFPGDGST (SEQ ID NO:121) | TRAGGYNVFTY (SEQ ID NO:211) |

| | LCDR1 | LCDR2 | LCDR3 |
|---|---|---|---|
| Chothia | KASQTVSNDVA (SEQ ID NO:317) | YASNRYT (SEQ ID NO:364) | QQDYSSPYT (SEQ ID NO:387) |
| AbM | KASQTVSNDVA (SEQ ID NO:317) | YASNRYT (SEQ ID NO:364) | QQDYSSPYT (SEQ ID NO:387) |
| Kabat | KASQTVSNDVA (SEQ ID NO:317) | YASNRYT (SEQ ID NO:364) | QQDYSSPYT (SEQ ID NO:387) |
| Contact | SNDVAWY (SEQ ID NO:285) | LLIYYASNRY (SEQ ID NO:336) | QQDYSSPY (SEQ ID NO:386) |
| IMGT | QTVSND (SEQ ID NO:286) | YA | QQDYSSPYT (SEQ ID NO:387) |

Table 2 shows an exemplary antibody F1-87.15 (the amino acid sequence of the heavy chain variable region is shown in SEQ ID NO: 3, and the amino acid sequence of the light chain variable region is shown in SEQ ID NO: 4).

**Table 2**

| | HCDR1 | HCDR2 | HCDR3 |
|---|---|---|---|
| Chothia | GYTFTEY (SEQ ID NO:112) | NPKNGD (SEQ ID NO:192) | SHLSYGGKTWFAY (SEQ ID NO:279) |
| AbM | GYTFTEYTMH (SEQ ID NO:114) | GINPKNGDST (SEQ ID NO:122) | SHLSYGGKTWFAY (SEQ ID NO:279) |
| Kabat | EYTMH (SEQ ID NO:105) | GINPKNGDSTYNQKFK G (SEQ ID NO:123) | SHLSYGGKTWFAY (SEQ ID NO:279) |
| Contact | TEYTMH (SEQ ID NO:116) | WIGGINPKNGDST (SEQ ID NO:124) | ARSHLSYGGKTWFA (SEQ ID NO:265) |
| IMGT | GYTFTEYT (SEQ ID NO:113) | INPKNGDS (SEQ ID NO:125) | ARSHLSYGGKTWFAY (SEQ ID NO:266) |

| | LCDR1 | LCDR2 | LCDR3 |
|---|---|---|---|
| Chothia | KSSQSLLYSSNQKN YLA (SEQ ID NO:318) | WASTRES (SEQ ID NO:363) | HQYYSYPPWT (SEQ ID NO:377) |
| AbM | KSSQSLLYSSNQKN YLA (SEQ ID NO:318) | WASTRES (SEQ ID NO:363) | HQYYSYPPWT (SEQ ID NO:377) |
| Kabat | KSSQSLLYSSNQKN YLA (SEQ ID NO:318) | WASTRES (SEQ ID NO:363) | HQYYSYPPWT (SEQ ID NO:377) |
| Contact | LYSSNQKNYLAWY (SEQ ID NO:320) | LLIYWASTRE (SEQ ID NO:355) | HQYYSYPPW (SEQ ID NO:376) |
| IMGT | QSLLYSSNQKNY (SEQ ID NO:322) | WA | HQYYSYPPWT (SEQ ID NO:377) |

Table 3 shows an exemplary antibody F1-111.5 (the amino acid sequence of the heavy chain variable region is shown in SEQ ID NO: 5, and the amino acid sequence of the light chain variable region is shown in SEQ ID NO: 6).

**Table 3**

| | HCDR1 | HCDR2 | HCDR3 |
|---|---|---|---|
| Chothia | GFTFSSY (SEQ ID NO:46) | SSGDGY (SEQ ID NO:203) | AGNFAY (SEQ ID NO:264) |
| AbM | GFTFSSYAMS (SEQ ID NO:47) | SISSGDGYTY (SEQ ID NO:126) | AGNFAY (SEQ ID NO:264) |
| Kabat | SYAMS (SEQ ID NO:48) | SISSGDGYTYYPDSVKG (SEQ ID NO:127) | AGNFAY (SEQ ID NO:264) |
| Contact | SSYAMS (SEQ ID NO:49) | WVASISSGDGYTY (SEQ ID NO:128) | ARAGNFA (SEQ ID NO:213) |
| IMGT | GFTFSSYA (SEQ ID NO:50) | ISSGDGYT (SEQ ID NO:129) | ARAGNFAY (SEQ ID NO:214) |

| | LCDR1 | LCDR2 | LCDR3 |
|---|---|---|---|
| Chothia | KASQDINNFLS (SEQ ID NO:314) | RTNRLVD (SEQ ID NO:359) | LKYDEFPLT (SEQ ID NO:379) |
| AbM | KASQDINNFLS (SEQ ID NO:314) | RTNRLVD (SEQ ID NO:359) | LKYDEFPLT (SEQ ID NO:379) |
| Kabat | KASQDINNFLS (SEQ ID NO:314) | RTNRLVD (SEQ ID NO:359) | LKYDEFPLT (SEQ ID NO:379) |
| Contact | NNFLSWF (SEQ ID NO:287) | TLIYRTNRLV (SEQ ID NO:337) | LKYDEFPL (SEQ ID NO:378) |
| IMGT | QDINNF (SEQ ID NO:288) | RT | LKYDEFPLT (SEQ ID NO:379) |

Table 4 shows an exemplary antibody F1-143.7 (the amino acid sequence of the heavy chain variable region is shown in SEQ ID NO: 7, and the amino acid sequence of the light chain variable region is shown in SEQ ID NO: 8).

**Table 4**

| | HCDR1 | HCDR2 | HCDR3 |
|---|---|---|---|
| Chothia | GYSFTSY (SEQ ID NO:111) | NPSHGG (SEQ ID NO:196) | TLRLRPPDY (SEQ ID NO:283) |
| AbM | GYSFTSYYMS (SEQ ID NO:51) | EINPSHGGTI (SEQ ID NO:130) | TLRLRPPDY (SEQ ID NO:283) |
| Kabat | SYYMS (SEQ ID NO:52) | EINPSHGGTIFNENFKN (SEQ ID NO:131) | TLRLRPPDY (SEQ ID NO:283) |
| Contact | TSYYMS (SEQ ID NO:53) | WIGEINPSHGGTI (SEQ ID NO:132) | IRTLRLRPPD (SEQ ID NO:216) |
| IMGT | GYSFTSYY (SEQ ID NO:54) | INPSHGGT (SEQ ID NO:133) | IRTLRLRPPDY (SEQ ID NO:217) |

| | LCDR1 | LCDR2 | LCDR3 |
|---|---|---|---|
| Chothia | SASSSVSYMH (SEQ ID NO:330) | DTSNLAS (SEQ ID NO:351) | HQRSSYPWT (SEQ ID NO:373) |
| AbM | SASSSVSYMH (SEQ ID NO:330) | DTSNLAS (SEQ ID NO:351) | HQRSSYPWT (SEQ ID NO:373) |
| Kabat | SASSSVSYMH (SEQ ID NO:330) | DTSNLAS (SEQ ID NO:351) | HQRSSYPWT (SEQ ID NO:373) |
| Contact | SYMHWY (SEQ ID NO:334) | PLIYDTSNLA (SEQ ID NO:338) | HQRSSYPW (SEQ ID NO:372) |
| IMGT | SSVSY (SEQ ID NO:333) | DT | HQRSSYPWT (SEQ ID NO:373) |

Table 5 shows an exemplary antibody F2-55.10 (the amino acid sequence of the heavy chain variable region is shown in SEQ ID NO: 9, and the amino acid sequence of the light chain variable region is shown in SEQ ID NO: 10).

**Table 5**

| | HCDR1 | HCDR2 | HCDR3 |
|---|---|---|---|
| Chothia | GYSFTGY (SEQ ID NO:110) | NPYNGA (SEQ ID NO:197) | WGNYPGYVMDY (SEQ ID NO:284) |
| AbM | GYSFTGYAMN (SEQ ID NO:55) | LINPYNGATT (SEQ ID NO:134) | WGNYPGYVMDY (SEQ ID NO:284) |
| Kabat | GYAMN | LINPYNGATTYNQKFRG | WGNYPGYVMDY |
| | (SEQ ID NO:56) | (SEQ ID NO:135) | (SEQ ID NO:284) |
| Contact | TGYAMN (SEQ ID NO:57) | WIGLINPYNGATT (SEQ ID NO:136) | ARWGNYPGYVMD (SEQ ID NO:219) |
| IMGT | GYSFTGYA (SEQ ID NO:58) | INPYNGAT (SEQ ID NO:137) | ARWGNYPGYVMDY (SEQ ID NO:220) |

| | LCDR1 | LCDR2 | LCDR3 |
|---|---|---|---|
| Chothia | TASSSVSSNYLH (SEQ ID NO:335) | STSNLAS (SEQ ID NO:362) | HQYHRSPYT (SEQ ID NO:375) |
| AbM | TASSSVSSNYLH (SEQ ID NO:335) | STSNLAS (SEQ ID NO:362) | HQYHRSPYT (SEQ ID NO:375) |
| Kabat | TASSSVSSNYLH (SEQ ID NO:335) | STSNLAS (SEQ ID NO:362) | HQYHRSPYT (SEQ ID NO:375) |
| Contact | SSNYLHWF (SEQ ID NO:289) | LWIYSTSNLA (SEQ ID NO:356) | HQYHRSPY (SEQ ID NO:374) |
| IMGT | SSVSSNY (SEQ ID NO:293) | ST | HQYHRSPYT (SEQ ID NO:375) |

Table 6 shows an exemplary antibody F2-155.7 (the amino acid sequence of the heavy chain variable region is shown in SEQ ID NO: 11, and the amino acid sequence of the light chain variable region is shown in SEQ ID NO: 12).

**Table 6**

| | HCDR1 | HCDR2 | HCDR3 |
|---|---|---|---|
| Chothia | GYTLTSH (SEQ ID NO:59) | YPGTGY (SEQ ID NO:208) | SYDAYFVGYFDY (SEQ ID NO:282) |
| AbM | GYTLTSHWIH (SEQ ID NO:60) | RIYPGTGYTY (SEQ ID NO:138) | SYDAYFVGYFDY (SEQ ID NO:282) |
| Kabat | SHWIH (SEQ ID NO:61) | RIYPGTGYTYYNENFRG (SEQ ID NO:139) | SYDAYFVGYFDY (SEQ ID NO:282) |
| Contact | TSHWIH (SEQ ID NO:62) | WIARIYPGTGYTY (SEQ ID NO:140) | ARSYDAYFVGYFD (SEQ ID NO:222) |
| IMGT | GYTLTSHW (SEQ ID NO:63) | IYPGTGYT (SEQ ID NO:141) | ARSYDAYFVGYFDY (SEQ ID NO:223) |

| | LCDR1 | LCDR2 | LCDR3 |
|---|---|---|---|
| Chothia | RASESVDSYGNSIMH (SEQ ID NO:323) | RASNLES (SEQ ID NO:357) | QQSNEDPYT (SEQ ID NO:395) |
| AbM | RASESVDSYGNSIMH (SEQ ID NO:323) | RASNLES (SEQ ID NO:357) | QQSNEDPYT (SEQ ID NO:395) |
| Kabat | RASESVDSYGNSIMH (SEQ ID NO:323) | RASNLES (SEQ ID NO:357) | QQSNEDPYT (SEQ ID NO:395) |
| Contact | DSYGNSIMHWY (SEQ ID NO:291) | LLIYRASNLE (SEQ ID NO:339) | QQSNEDPY (SEQ ID NO:394) |
| IMGT | ESVDSYGNSI (SEQ ID NO:292) | RA | QQSNEDPYT (SEQ ID NO:395) |

Table 7 shows an exemplary antibody F2-186.1 (the amino acid sequence of the heavy chain variable region is shown in SEQ ID NO: 13, and the amino acid sequence of the light chain variable region is shown in SEQ ID NO: 14).

**Table 7**

| | HCDR1 | HCDR2 | HCDR3 |
|---|---|---|---|
| Chothia | GFTFSTY (SEQ ID NO:106) | NNGVSY (SEQ ID NO:191) | QGYDYAYFDH (SEQ ID NO:272) |
| AbM | GFTFSTYAMS (SEQ ID NO:108) | TINNGVSYTY (SEQ ID NO:142) | QGYDYAYFDH (SEQ ID NO:272) |
| Kabat | TYAMS (SEQ ID NO:117) | TINNGVSYTYYPDSVKG (SEQ ID NO:143) | QGYDYAYFDH (SEQ ID NO:272) |
| Contact | STYAMS (SEQ ID NO:115) | WVATINNGVSYTY (SEQ ID NO:144) | ARQGYDYAYFD (SEQ ID NO:225) |
| IMGT | GFTFSTYA (SEQ ID NO:107) | INNGVSYT (SEQ ID NO:145) | ARQGYDYAYFDH (SEQ ID NO:226) |

| | LCDR1 | LCDR2 | LCDR3 |
|---|---|---|---|
| Chothia | TASSSVSSNYLH (SEQ ID NO:335) | STSNLAS (SEQ ID NO:362) | HQYHRSPYT (SEQ ID NO:375) |
| AbM | TASSSVSSNYLH (SEQ ID NO:335) | STSNLAS (SEQ ID NO:362) | HQYHRSPYT (SEQ ID NO:375) |
| Kabat | TASSSVSSNYLH (SEQ ID NO:335) | STSNLAS (SEQ ID NO:362) | HQYHRSPYT (SEQ ID NO:375) |
| Contact | SSNYLHWF (SEQ ID NO:289) | LWIYSTSNLA (SEQ ID NO:356) | HQYHRSPY (SEQ ID NO:374) |
| IMGT | SSVSSNY (SEQ ID NO:293) | ST | HQYHRSPYT (SEQ ID NO:375) |

Table 8 shows an exemplary antibody F2-238.14 (the amino acid sequence of the heavy chain variable region is shown in SEQ ID NO: 15, and the amino acid sequence of the light chain variable region is shown in SEQ ID NO: 16).

**Table 8**

| | HCDR1 | HCDR2 | HCDR3 |
|---|---|---|---|
| Chothia | GYSFTGY (SEQ ID NO:110) | NPNNGG (SEQ ID NO:195) | SLITTLVVHNYWYFDV (SEQ ID NO:281) |
| AbM | GYSFTGYTVN (SEQ ID NO:64) | LINPNNGGVN (SEQ ID NO:146) | SLITTLVVHNYWYFDV (SEQ ID NO:281) |
| Kabat | GYTVN (SEQ ID NO:65) | LINPNNGGVNYNQKFKD (SEQ ID NO:147) | SLITTLVVHNYWYFDV (SEQ ID NO:281) |
| Contact | TGYTVN (SEQ ID NO:66) | WIGLINPNNGGVN (SEQ ID NO:148) | ARSLITTLVVHNYWYFD (SEQ ID NO:228) |
| IMGT | GYSFTGYT (SEQ ID NO:67) | INPNNGGV (SEQ ID NO:149) | ARSLITTLVVHNYWYFDV (SEQ ID NO:229) |

| | LCDR1 | LCDR2 | LCDR3 |
|---|---|---|---|
| Chothia | RASQSVSSSTYGY VH (SEQ ID NO:326) | YASTLES (SEQ ID NO:366) | QHSWEIPYT (SEQ ID NO:383) |
| AbM | RASQSVSSSTYGY VH (SEQ ID NO:326) | YASTLES (SEQ ID NO:366) | QHSWEIPYT (SEQ ID NO:383) |
| Kabat | RASQSVSSSTYGY VH (SEQ ID NO:326) | YASTLES (SEQ ID NO:366) | QHSWEIPYT (SEQ ID NO:383) |
| Contact | SSSTYGYVHWY (SEQ ID NO:295) | LLIKYASTLE (SEQ ID NO:340) | QHSWEIPY (SEQ ID NO:382) |
| IMGT | QSVSSSTYGY (SEQ ID NO:296) | YA | QHSWEIPYT (SEQ ID NO:383) |

Table 9 shows an exemplary antibody F2-324.7 (the amino acid sequence of the heavy chain variable region is shown in SEQ ID NO: 17, and the amino acid sequence of the light chain variable region is shown in SEQ ID NO: 18).

**Table 9**

| | HCDR1 | HCDR2 | HCDR3 |
|---|---|---|---|
| Chothia | GDAITSG (SEQ ID NO:68) | TFSGD (SEQ ID NO:205) | RDYGNYWFAY (SEQ ID NO:273) |
| AbM | GDAITSGYWN (SEQ ID NO:69) | YITFSGDTY (SEQ ID NO:150) | RDYGNYWFAY (SEQ ID NO:273) |
| Kabat | SGYWN (SEQ ID NO:70) | YITFSGDTYYSPSLKS (SEQ ID NO:151) | RDYGNYWFAY (SEQ ID NO:273) |
| Contact | TSGYWN (SEQ ID NO:71) | YMGYITFSGDTY (SEQ ID NO:152) | ARRDYGNYWFA (SEQ ID NO:231) |
| IMGT | GDAITSGY (SEQ ID NO:72) | ITFSGDT (SEQ ID NO:153) | ARRDYGNYWFAY (SEQ ID NO:232) |

| | LCDR1 | LCDR2 | LCDR3 |
|---|---|---|---|
| Chothia | KASQDVSSAVA (SEQ ID NO:316) | SASFRYT (SEQ ID NO:360) | QQHYSGPFT (SEQ ID NO:393) |
| AbM | KASQDVSSAVA (SEQ ID NO:316) | SASFRYT (SEQ ID NO:360) | QQHYSGPFT (SEQ ID NO:393) |
| Kabat | KASQDVSSAVA (SEQ ID NO:316) | SASFRYT (SEQ ID NO:360) | QQHYSGPFT (SEQ ID NO:393) |
| Contact | SSAVAWY (SEQ ID NO:297) | LLIYSASFRY (SEQ ID NO:341) | QQHYSGPF (SEQ ID NO:392) |
| IMGT | QDVSSA (SEQ ID NO:298) | SA | QQHYSGPFT (SEQ ID NO:393) |

Table 10 shows an exemplary antibody F3-1.5 (the amino acid sequence of the heavy chain variable region is shown in SEQ ID NO: 19, and the amino acid sequence of the light chain variable region is shown in SEQ ID NO: 20).

**Table 10**

| | HCDR1 | HCDR2 | HCDR3 |
|---|---|---|---|
| Chothia | GFSLSTSGM (SEQ ID NO:73) | FWDDD (SEQ ID NO:186) | RQWAYQFDS (SEQ ID NO:275) |
| AbM | GFSLSTSGMGVS (SEQ ID NO:74) | HIFWDDDKR (SEQ ID NO:154) | RQWAYQFDS (SEQ ID NO:275) |
| Kabat | TSGMGVS (SEQ ID NO:75) | HIFWDDDKRYNPSLKS (SEQ ID NO:155) | RQWAYQFDS (SEQ ID NO:275) |
| Contact | STSGMGVS (SEQ ID NO:76) | WLAHIFWDDDKR (SEQ ID NO:156) | ARRQWAYQFD (SEQ ID NO:234) |
| IMGT | GFSLSTSGMG (SEQ ID NO:77) | IFWDDDK (SEQ ID NO:157) | ARRQWAYQFDS (SEQ ID NO:235) |

| | LCDR1 | LCDR2 | LCDR3 |
|---|---|---|---|
| Chothia | RSSQSILHSNGNTYLE (SEQ ID NO:329) | KVSNRFS (SEQ ID NO:354) | FQGSHVPPA (SEQ ID NO:371) |
| AbM | RSSQSILHSNGNTYLE (SEQ ID NO:329) | KVSNRFS (SEQ ID NO:354) | FQGSHVPPA (SEQ ID NO:371) |
| Kabat | RSSQSILHSNGNTYLE (SEQ ID NO:329) | KVSNRFS (SEQ ID NO:354) | FQGSHVPPA (SEQ ID NO:371) |
| Contact | LHSNGNTYLEWY (SEQ ID NO:300) | LLIYKVSNRF (SEQ ID NO:342) | FQGSHVPP (SEQ ID NO:370) |
| IMGT | QSILHSNGNTY (SEQ ID NO:301) | KV | FQGSHVPPA (SEQ ID NO:371) |

Table 11 shows an exemplary antibody F1-76.12 (the amino acid sequence of the heavy chain variable region is shown in SEQ ID NO: 21, and the amino acid sequence of the light chain variable region is shown in SEQ ID NO: 22).

**Table 11**

| | HCDR1 | HCDR2 | HCDR3 |
|---|---|---|---|
| Chothia | GYTFTEY (SEQ ID NO:112) | NPNNGD (SEQ ID NO:194) | SHLSYGGKTWFAY (SEQ ID NO:279) |
| AbM | GYTFTEYTMH (SEQ ID NO:114) | GINPNNGDTT (SEQ ID NO:187) | SHLSYGGKTWFAY (SEQ ID NO:279) |
| Kabat | EYTMH (SEQ ID NO:105) | GINPNNGDTTYNQKFKG (SEQ ID NO:158) | SHLSYGGKTWFAY (SEQ ID NO:279) |
| Contact | TEYTMH (SEQ ID NO:116) | WIGGINPNNGDTT (SEQ ID NO:206) | ARSHLSYGGKTWFA (SEQ ID NO:265) |
| IMGT | GYTFTEYT (SEQ ID NO:113) | INPNNGDT (SEQ ID NO:189) | ARSHLSYGGKTWFAY (SEQ ID NO:266) |

| | LCDR1 | LCDR2 | LCDR3 |
|---|---|---|---|
| Chothia | KSSQSLLYSSNQKNY LA (SEQ ID NO:318) | WASTRES (SEQ ID NO:363) | HQYYSYPPWT (SEQ ID NO:377) |
| AbM | KSSQSLLYSSNQKNY LA (SEQ ID NO:318) | WASTRES (SEQ ID NO:363) | HQYYSYPPWT (SEQ ID NO:377) |
| Kabat | KSSQSLLYSSNQKNY LA (SEQ ID NO:318) | WASTRES (SEQ ID NO:363) | HQYYSYPPWT (SEQ ID NO:377) |
| Contact | LYSSNQKNYLAWY (SEQ ID NO:320) | LLIYWASTRE (SEQ ID NO:355) | HQYYSYPPW (SEQ ID NO:376) |
| IMGT | QSLLYSSNQKNY (SEQ ID NO:322) | WA | HQYYSYPPWT (SEQ ID NO:377) |

Table 12 shows an exemplary antibody F1-86.9 (the amino acid sequence of the heavy chain variable region is shown in SEQ ID NO: 23, and the amino acid sequence of the light chain variable region is shown in SEQ ID NO: 24).

**Table 12**

| | HCDR1 | HCDR2 | HCDR3 |
|---|---|---|---|
| Chothia | GYSFTSY (SEQ ID NO:111) | RPSDME (SEQ ID NO:199) | GTGSWFAY (SEQ ID NO:271) |
| AbM | GYSFTSYWMN (SEQ ID NO:78) | MIRPSDMETR (SEQ ID NO:159) | GTGSWFAY (SEQ ID NO:271) |
| Kabat | SYWMN (SEQ ID NO:79) | MIRPSDMETRLNQKFRD (SEQ ID NO:160) | GTGSWFAY (SEQ ID NO:271) |
| Contact | TSYWMN (SEQ ID NO:80) | WIGMIRPSDMETR (SEQ ID NO:161) | AFGTGSWFA (SEQ ID NO:237) |
| IMGT | GYSFTSYW (SEQ ID NO:81) | IRPSDMET (SEQ ID NO:162) | AFGTGSWFAY (SEQ ID NO:238) |
| | LCDR1 | LCDR2 | LCDR3 |
| Chothia | SASSSVSYMH (SEQ ID NO:330) | DTSKLAS (SEQ ID NO:350) | FQGSGYPLT (SEQ ID NO:369) |
| AbM | SASSSVSYMH (SEQ ID NO:330) | DTSKLAS (SEQ ID NO:350) | FQGSGYPLT (SEQ ID NO:369) |
| Kabat | SASSSVSYMH (SEQ ID NO:330) | DTSKLAS (SEQ ID NO:350) | FQGSGYPLT (SEQ ID NO:369) |
| Contact | SYMHWY (SEQ ID NO:334) | LWIYDTSKLA (SEQ ID NO:343) | FQGSGYPL (SEQ ID NO:368) |
| IMGT | SSVSY (SEQ ID NO:333) | DT | FQGSGYPLT (SEQ ID NO:369) |

Table 13 shows an exemplary antibody F1-99.29 (the amino acid sequence of the heavy chain variable region is shown in SEQ ID NO: 25, and the amino acid sequence of the light chain variable region is shown in SEQ ID NO: 26).

**Table 13**

| | HCDR1 | HCDR2 | HCDR3 |
|---|---|---|---|
| Chothia | GYTFTEY (SEQ ID NO:112) | NPNNGD (SEQ ID NO:194) | SHLSYGSKTWFAY (SEQ ID NO:280) |
| AbM | GYTFTEYTMH (SEQ ID NO:114) | GINPNNGDTT (SEQ ID NO:187) | SHLSYGSKTWFAY (SEQ ID NO:280) |
| Kabat | EYTMH (SEQ ID NO:105) | GINPNNGDTTYNRKFKG (SEQ ID NO:163) | SHLSYGSKTWFAY (SEQ ID NO:280) |
| Contact | TEYTMH (SEQ ID NO:116) | WIGGINPNNGDTT (SEQ ID NO:206) | ARSHLSYGSKTWFA (SEQ ID NO:240) |
| IMGT | GYTFTEYT (SEQ ID NO:113) | INPNNGDT (SEQ ID NO:189) | ARSHLSYGSKTWFAY (SEQ ID NO:241) |

| | LCDR1 | LCDR2 | LCDR3 |
|---|---|---|---|
| Share | LYSSNQKNY (SEQ ID NO:319) | WA | HQYYSYPPW (SEQ ID NO:376) |
| Chothia | KSSQSLLYSSNQKNY LA (SEQ ID NO:318) | WASTRES (SEQ ID NO:363) | HQYYSYPPWT (SEQ ID NO:377) |
| AbM | KSSQSLLYSSNQKNY LA (SEQ ID NO:318) | WASTRES (SEQ ID NO:363) | HQYYSYPPWT (SEQ ID NO:377) |
| Kabat | KSSQSLLYSSNQKNY LA (SEQ ID NO:318) | WASTRES (SEQ ID NO:363) | HQYYSYPPWT (SEQ ID NO:377) |
| Contact | LYSSNQKNYLAWY (SEQ ID NO:320) | LLIYWASTRE (SEQ ID NO:355) | HQYYSYPPW (SEQ ID NO:376) |
| IMGT | QSLLYSSNQKNY (SEQ ID NO:322) | WA | HQYYSYPPWT (SEQ ID NO:377) |

Table 14 shows an exemplary antibody F1-124.38 (the amino acid sequence of the heavy chain variable region is shown in SEQ ID NO: 27, and the amino acid sequence of the light chain variable region is shown in SEQ ID NO: 28).

**Table 14**

| | HCDR1 | HCDR2 | HCDR3 |
|---|---|---|---|
| Chothia | GYSFTDY (SEQ ID NO:109) | NPKNGG (SEQ ID NO:193) | EYDNYDAMDY (SEQ ID NO:268) |
| AbM | GYSFTDYYLH (SEQ ID NO:82) | RINPKNGGTT (SEQ ID NO:164) | EYDNYDAMDY (SEQ ID NO:268) |
| Kabat | DYYLH (SEQ ID NO:83) | RINPKNGGTTYNQKFKG (SEQ ID NO:165) | EYDNYDAMDY (SEQ ID NO:268) |
| Contact | TDYYLH (SEQ ID NO:84) | WIGRINPKNGGTT (SEQ ID NO:166) | AKEYDNYDAMD (SEQ ID NO:243) |
| IMGT | GYSFTDYY (SEQ ID NO:85) | INPKNGGT (SEQ ID NO:167) | AKEYDNYDAMDY (SEQ ID NO:244) |

| | LCDR1 | LCDR2 | LCDR3 |
|---|---|---|---|
| Chothia | KASDHINNWLA (SEQ ID NO:313) | GATSLET (SEQ ID NO:353) | QWYWGNPP (SEQ ID NO:397) |
| AbM | KASDHINNWLA (SEQ ID NO:313) | GATSLET (SEQ ID NO:353) | QWYWGNPP (SEQ ID NO:397) |
| Kabat | KASDHINNWLA (SEQ ID NO:313) | GATSLET (SEQ ID NO:353) | QWYWGNPP (SEQ ID NO:397) |
| Contact | NNWLAWY (SEQ ID NO:302) | LLISGATSLE (SEQ ID NO:344) | QWYWGNP (SEQ ID NO:396) |
| IMGT | DHINNW (SEQ ID NO:303) | GA | QWYWGNPP (SEQ ID NO:397) |

Table 15 shows an exemplary antibody F1-127.8 (the amino acid sequence of the heavy chain variable region is shown in SEQ ID NO: 29, and the amino acid sequence of the light chain variable region is shown in SEQ ID NO: 30).

**Table 15**

| | HCDR1 | HCDR2 | HCDR3 |
|---|---|---|---|
| Chothia | GYTFTTY (SEQ ID NO:86) | SPSGIF (SEQ ID NO:202) | SGGNYAMDY (SEQ ID NO:277) |
| AbM | GYTFTTYWIN (SEQ ID NO:87) | NISPSGIFIN (SEQ ID NO:168) | SGGNYAMDY (SEQ ID NO:277) |
| Kabat | TYWIN (SEQ ID NO:88) | NISPSGIFINYNQKFKD (SEQ ID NO:169) | SGGNYAMDY (SEQ ID NO:277) |
| Contact | TTYWIN (SEQ ID NO:89) | WIGNISPSGIFIN (SEQ ID NO:170) | TRSGGNYAMD (SEQ ID NO:246) |
| IMGT | GYTFTTYW (SEQ ID NO:90) | ISPSGIFI (SEQ ID NO:171) | TRSGGNYAMDY (SEQ ID NO:247) |

| | LCDR1 | LCDR2 | LCDR3 |
|---|---|---|---|
| Chothia | RASSSVNYMY (SEQ ID NO:328) | YTSNLAP (SEQ ID NO:367) | QQFTSSPFT (SEQ ID NO:389) |
| AbM | RASSSVNYMY (SEQ ID NO:328) | YTSNLAP (SEQ ID NO:367) | QQFTSSPFT (SEQ ID NO:389) |
| Kabat | RASSSVNYMY (SEQ ID NO:328) | YTSNLAP (SEQ ID NO:367) | QQFTSSPFT (SEQ ID NO:389) |
| Contact | NYMYWY (SEQ ID NO:304) | IWIYYTSNLA (SEQ ID NO:345) | QQFTSSPF (SEQ ID NO:388) |
| IMGT | SSVNY (SEQ ID NO:305) | YT | QQFTSSPFT (SEQ ID NO:389) |

Table 16 shows an exemplary antibody F2-71.18 (the amino acid sequence of the heavy chain variable region is shown in SEQ ID NO: 31, and the amino acid sequence of the light chain variable region is shown in SEQ ID NO: 32).

**Table 16**

| | HCDR1 | HCDR2 | HCDR3 |
|---|---|---|---|
| Chothia | GYTFTNY (SEQ ID NO:91) | NTYTGA (SEQ ID NO:198) | DHDGGFVY (SEQ ID NO:267) |
| AbM | GYTFTNYGMN (SEQ ID NO:92) | WINTYTGAPT (SEQ ID NO:172) | DHDGGFVY (SEQ ID NO:267) |
| Kabat | NYGMN (SEQ ID NO:93) | WINTYTGAPTYADDFKG (SEQ ID NO:173) | DHDGGFVY (SEQ ID NO:267) |
| Contact | TNYGMN (SEQ ID NO:94) | WLGWINTYTGAPT (SEQ ID NO:174) | ARDHDGGFV (SEQ ID NO:249) |
| IMGT | GYTFTNYG (SEQ ID NO:95) | INTYTGAP (SEQ ID NO:175) | ARDHDGGFVY (SEQ ID NO:250) |

| | LCDR1 | LCDR2 | LCDR3 |
|---|---|---|---|
| Chothia | RASQSISDFLH (SEQ ID NO:325) | YASQSIS (SEQ ID NO:365) | QNGHSFPRT (SEQ ID NO:385) |
| AbM | RASQSISDFLH (SEQ ID NO:325) | YASQSIS (SEQ ID NO:365) | QNGHSFPRT (SEQ ID NO:385) |
| Kabat | RASQSISDFLH (SEQ ID NO:325) | YASQSIS (SEQ ID NO:365) | QNGHSFPRT (SEQ ID NO:385) |
| Contact | SDFLHWY (SEQ ID NO:306) | LLIKYASQSI (SEQ ID NO:346) | QNGHSFPR (SEQ ID NO:384) |
| IMGT | QSISDF (SEQ ID NO:307) | YA | QNGHSFPRT (SEQ ID NO:385) |

Table 17 shows an exemplary antibody F2-89.7 (the amino acid sequence of the heavy chain variable region is shown in SEQ ID NO: 33, and the amino acid sequence of the light chain variable region is shown in SEQ ID NO: 34).

**Table 17**

| | HCDR1 | HCDR2 | HCDR3 |
|---|---|---|---|
| Chothia | GFTFSTY (SEQ ID NO:106) | SSGGS (SEQ ID NO:204) | GSFFTTVITDFDY (SEQ ID NO:269) |
| AbM | GFTFSTYAMS (SEQ ID NO:108) | SISSGGSTY (SEQ ID NO:200) | GSFFTTVITDFDY (SEQ ID NO:269) |
| Kabat | TYAMS (SEQ ID NO:117) | SISSGGSTYYPDSVRG (SEQ ID NO:201) | GSFFTTVITDFDY (SEQ ID NO:269) |
| Contact | STYAMS (SEQ ID NO:115) | WVASISSGGSTY (SEQ ID NO:207) | TRGSFFTTVITDFD (SEQ ID NO:252) |
| IMGT | GFTFSTYA (SEQ ID NO:107) | ISSGGST (SEQ ID NO:190) | TRGSFFTTVITDFDY (SEQ ID NO:253) |

| | LCDR1 | LCDR2 | LCDR3 |
|---|---|---|---|
| Chothia | RASQEISGYLS (SEQ ID NO:324) | AASTLDS (SEQ ID NO:349) | LQYAIYPWT (SEQ ID NO:381) |
| AbM | RASQEISGYLS (SEQ ID NO:324) | AASTLDS (SEQ ID NO:349) | LQYAIYPWT (SEQ ID NO:381) |
| Kabat | RASQEISGYLS (SEQ ID NO:324) | AASTLDS (SEQ ID NO:349) | LQYAIYPWT (SEQ ID NO:381) |
| Contact | SGYLSWL (SEQ ID NO:331) | RLIYAASTLD (SEQ ID NO:358) | LQYAIYPW (SEQ ID NO:380) |
| IMGT | QEISGY (SEQ ID NO:321) | AA | LQYAIYPWT (SEQ ID NO:381) |

Table 18 shows an exemplary antibody F2-291.2 (the amino acid sequence of the heavy chain variable region is shown in SEQ ID NO: 35, and the amino acid sequence of the light chain variable region is shown in SEQ ID NO: 36).

**Table 18**

| | HCDR1 | HCDR2 | HCDR3 |
|---|---|---|---|
| Chothia | GFTFSTY (SEQ ID NO:106) | SSGGS (SEQ ID NO:204) | GSFFTTVVTDFDY (SEQ ID NO:270) |
| AbM | GFTFSTYAMS (SEQ ID NO:108) | SISSGGSTY (SEQ ID NO:200) | GSFFTTVVTDFDY (SEQ ID NO:270) |
| Kabat | TYAMS (SEQ ID NO:117) | SISSGGSTYYPDSVRG (SEQ ID NO:201) | GSFFTTVVTDFDY (SEQ ID NO:270) |
| Contact | STYAMS (SEQ ID NO:115) | WVASISSGGSTY (SEQ ID NO:207) | TRGSFFTTVVTDFD (SEQ ID NO:255) |
| IMGT | GFTFSTYA (SEQ ID NO:107) | ISSGGST (SEQ ID NO:190) | TRGSFFTTVVTDFDY (SEQ ID NO:256) |

| | LCDR1 | LCDR2 | LCDR3 |
|---|---|---|---|
| Chothia | RASQEISGYLS (SEQ ID NO:324) | AASTLDS (SEQ ID NO:349) | LQYAIYPWT (SEQ ID NO:381) |
| AbM | RASQEISGYLS (SEQ ID NO:324) | AASTLDS (SEQ ID NO:349) | LQYAIYPWT (SEQ ID NO:381) |
| Kabat | RASQEISGYLS (SEQ ID NO:324) | AASTLDS (SEQ ID NO:349) | LQYAIYPWT (SEQ ID NO:381) |
| Contact | SGYLSWL (SEQ ID NO:331) | RLIYAASTLD (SEQ ID NO:358) | LQYAIYPW (SEQ ID NO:380) |
| IMGT | QEISGY (SEQ ID NO:321) | AA | LQYAIYPWT (SEQ ID NO:381) |

Table 19 shows an exemplary antibody F3-6.18 (the amino acid sequence of the heavy chain variable region is shown in SEQ ID NO: 37, and the amino acid sequence of the light chain variable region is shown in SEQ ID NO: 38).

**Table 19**

| | HCDR1 | HCDR2 | HCDR3 |
|---|---|---|---|
| Chothia | GYTFSNY (SEQ ID NO:96) | DPSDSY (SEQ ID NO:184) | RGDDGVLDY (SEQ ID NO:274) |
| AbM | GYTFSNYWMH (SEQ ID NO:97) | EIDPSDSYTN (SEQ ID NO:176) | RGDDGVLDY (SEQ ID NO:274) |
| Kabat | NYWMH (SEQ ID NO:98) | EIDPSDSYTNYNQNFKG (SEQ ID NO:177) | RGDDGVLDY (SEQ ID NO:274) |
| Contact | SNYWMH (SEQ ID NO:99) | WIGEIDPSDSYTN (SEQ ID NO:178) | ARRGDDGVLD (SEQ ID NO:258) |
| IMGT | GYTFSNYW (SEQ ID NO:100) | IDPSDSYT (SEQ ID NO:179) | ARRGDDGVLDY (SEQ ID NO:259) |

| | LCDR1 | LCDR2 | LCDR3 |
|---|---|---|---|
| Chothia | RASQSVSTSGYSYMH (SEQ ID NO:327) | FASNLES (SEQ ID NO:352) | QHSWEIPYT (SEQ ID NO:383) |
| AbM | RASQSVSTSGYSYMH (SEQ ID NO:327) | FASNLES (SEQ ID NO:352) | QHSWEIPYT (SEQ ID NO:383) |
| Kabat | RASQSVSTSGYSYMH (SEQ ID NO:327) | FASNLES (SEQ ID NO:352) | QHSWEIPYT (SEQ ID NO:383) |
| Contact | STSGYSYMHWY (SEQ ID NO:309) | LLIKFASNLE (SEQ ID NO:347) | QHSWEIPY (SEQ ID NO:382) |
| IMGT | QSVSTSGYSY (SEQ ID NO:310) | FA | QHSWEIPYT (SEQ ID NO:383) |

Table 20 shows an exemplary antibody F3-36.14 (the amino acid sequence of the heavy chain variable region is shown in SEQ ID NO: 39, and the amino acid sequence of the light chain variable region is shown in SEQ ID NO: 40).

**Table 20**

| | HCDR1 | HCDR2 | HCDR3 |
|---|---|---|---|
| Chothia | GYSFTDY (SEQ ID NO:109) | HPNSGE (SEQ ID NO:188) | RRGYGNYFFDY (SEQ ID NO:276) |
| AbM | GYSFTDYFMN (SEQ ID NO:101) | RIHPNSGETF (SEQ ID NO:180) | RRGYGNYFFDY (SEQ ID NO:276) |
| Kabat | DYFMN (SEQ ID NO:102) | RIHPNSGETFYNRQFKG (SEQ ID NO:181) | RRGYGNYFFDY (SEQ ID NO:276) |
| Contact | TDYFMN (SEQ ID NO:103) | WIGRIHPNSGETF (SEQ ID NO:182) | GRRRGYGNYFFD (SEQ ID NO:261) |
| IMGT | GYSFTDYF (SEQ ID NO:104) | IHPNSGET (SEQ ID NO:183) | GRRRGYGNYFFDY (SEQ ID NO:262) |

| | LCDR1 | LCDR2 | LCDR3 |
|---|---|---|---|
| Chothia | KASQDVNIDVA (SEQ ID NO:315) | SASYRYT (SEQ ID NO:361) | QQHYGTPWT (SEQ ID NO:391) |
| AbM | KASQDVNIDVA (SEQ ID NO:315) | SASYRYT (SEQ ID NO:361) | QQHYGTPWT (SEQ ID NO:391) |
| Kabat | KASQDVNIDVA (SEQ ID NO:316) | SASYRYT (SEQ ID NO:361) | QQHYGTPWT (SEQ ID NO:391) |
| Contact | NIDVAWY (SEQ ID NO:311) | LLIYSASYRY (SEQ ID NO:348) | QQHYGTPW (SEQ ID NO:390) |
| IMGT | QDVNID (SEQ ID NO:312) | SA | QQHYGTPWT (SEQ ID NO:391) |

In an optional implementation, the above anti-CDH17 antibody or the antigen-binding fragment thereof includes a heavy chain framework region (FR) and/or a light chain framework region (FR), and the above heavy chain FR and/or the light chain FR is derived from at least one of a mouse-derived antibody, a human antibody, a primate-derived antibody, or mutants thereof.

In an optional implementation, the three CDRs of the above anti-CDH17 antibody or the antigen-binding fragment thereof are separated by flanking portions, which are referred to as FRs (the light chain FR includes LFR1, LFR2, LFR3, and LFR4, and the heavy chain FR includes HFR1, HFR2, HFR3, and HFR4).

In an optional implementation, the HFR1 includes an amino acid sequence shown in any one of SEQ ID NO: 398-415, or includes a sequence having at least 78% (e.g., 78%, 80%, 82%, 85%, 87%, 89%, 90%, 91%, 92%, 93%, 95%, 97%, 98%, or 99%) sequence identity to the amino acid sequence shown in any one of SEQ ID NO: 398-415.

In an optional implementation, the HFR2 includes an amino acid sequence shown in any one of SEQ ID NO: 416, 418, 422, 426, 428, 431, 433, 436, 439, 442, and 445-448, or includes a sequence having at least 70% (e.g., 70%, 72%, 75%, 78%, 80%, 82%, 85%, 87%, 89%, 90%, 91%, 92%, 93%, 95%, 97%, 98%, or 99%) sequence identity to the amino acid sequence shown in any one of SEQ ID NO: 416, 418, 422, 426, 428, 431, 433, 436, 439, 442, and 445-448.

In an optional implementation, the HFR3 includes an amino acid sequence shown in any one of SEQ ID NO: 449, 451, 453, 455, 457, 459, 461, 463, 465, 467, 469, 472, 474, 477, 479, 481, 483, 485, and 487, or includes a sequence having at least 72% (e.g., 72%, 75%, 78%, 80%, 82%, 85%, 87%, 89%, 90%, 91%, 92%, 93%, 95%, 97%, 98%, or 99%) sequence identity to the amino acid sequence shown in any one of SEQ ID NO: 449, 451, 453, 455, 457, 459, 461, 463, 465, 467, 469, 472, 474, 477, 479, 481, 483, 485, and 487.

In an optional implementation, the HFR4 includes an amino acid sequence shown in any one of SEQ ID NO: 488-493, or includes a sequence having at least 86% (e.g., 86%, 87%, 89%, 90%, 91%, 92%, 93%, 95%, 97%, 98%, or 99%) sequence identity to the amino acid sequence shown in any one of SEQ ID NO: 488-493.

In an optional implementation, the LFR1 includes an amino acid sequence shown in any one of SEQ ID NO: 494, 496, 498, 500, 502, 504, 506, 508, 510, 512, 514, 516, 518, 520, 522, 524, and 526, or includes a sequence having at least 70% (e.g., 70%, 72%, 75%, 78%, 80%, 82%, 85%, 87%, 89%, 90%, 91%, 92%, 93%, 95%, 97%, 98%, or 99%) sequence identity to the amino acid sequence shown in any one of SEQ ID NO: 494, 496, 498, 500, 502, 504, 506, 508, 510, 512, 514, 516, 518, 520, 522, 524, and 526.

In an optional implementation, the LFR2 includes an amino acid sequence shown in any one of SEQ ID NO: 528, 533, 535, 537, 539, 541, 543, and 549-552, or includes a sequence having at least 80% (e.g., 80%, 82%, 85%, 87%, 89%, 90%, 91%, 92%, 93%, 95%, 97%, 98%, or 99%) sequence identity to the amino acid sequence shown in any one of SEQ ID NO: 528, 533, 535, 537, 539, 541, 543, and 549-552.

In an optional implementation, the LFR3 includes an amino acid sequence shown in any one of SEQ ID NO: 554, 556, 558, 560, 562, 564, 566, 568, 570, 572, 574, 576, 578, 580, 581, and 582, or includes a sequence having at least 79% (e.g., 79%, 80%, 82%, 85%, 87%, 89%, 90%, 91%, 92%, 93%, 95%, 97%, 98%, or 99%) sequence identity to the amino acid sequence shown in any one of SEQ ID NO: 554, 556, 558, 560, 562, 564, 566, 568, 570, 572, 574, 576, 578, 580, 581, and 582.

In an optional implementation, the LFR4 includes an amino acid sequence shown in any one of SEQ ID NO: 586-590, or includes a sequence having at least 95% (e.g., 95%, 97%, 98%, or 99%) sequence identity to the amino acid sequence shown in any one of SEQ ID NO: 586-590.

In an optional implementation, the HFR1 includes an HFR1 of the heavy chain variable region shown in any one of SEQ ID NO: 1, 3, 5, 7, 9, 11, 13, 15, 17, 19, 21, 23, 25, 27, 29, 31, 33, 35, 37, and 39.

In an optional implementation, the HFR2 includes an HFR2 of the heavy chain variable region shown in any one of SEQ ID NO: 1, 3, 5, 7, 9, 11, 13, 15, 17, 19, 21, 23, 25, 27, 29, 31, 33, 35, 37, and 39.

In an optional implementation, the HFR3 includes an HFR3 of the heavy chain variable region shown in any one of SEQ ID NO: 1, 3, 5, 7, 9, 11, 13, 15, 17, 19, 21, 23, 25, 27, 29, 31, 33, 35, 37, and 39.

In an optional implementation, the HFR4 includes an HFR4 of the heavy chain variable region shown in any one of SEQ ID NO: 1, 3, 5, 7, 9, 11, 13, 15, 17, 19, 21, 23, 25, 27, 29, 31, 33, 35, 37, and 39.

In an optional implementation, the LFR1 includes an LFR1 of the light chain variable region shown in any one of SEQ ID NO: 2, 4, 6, 8, 10, 12, 14, 16, 18, 20, 22, 24, 26, 28, 30, 32, 34, 36, 38, and 40.

In an optional implementation, the LFR2 includes an LFR2 of the light chain variable region shown in any one of SEQ ID NO: 2, 4, 6, 8, 10, 12, 14, 16, 18, 20, 22, 24, 26, 28, 30, 32, 34, 36, 38, and 40.

In an optional implementation, the LFR3 includes an LFR3 of the light chain variable region shown in any one of SEQ ID NO: 2, 4, 6, 8, 10, 12, 14, 16, 18, 20, 22, 24, 26, 28, 30, 32, 34, 36, 38, and 40.

In an optional implementation, the LFR4 includes an LFR4 of the light chain variable region shown in any one of SEQ ID NO: 2, 4, 6, 8, 10, 12, 14, 16, 18, 20, 22, 24, 26, 28, 30, 32, 34, 36, 38, and 40.

In an optional implementation, the HFR1, HFR2, HFR3, and HFR4 include the HFR1, HFR2, HFR3, and HFR4 of the heavy chain variable region shown in any one of SEQ ID NO: 1, 3, 5, 7, 9, 11, 13, 15, 17, 19, 21, 23, 25, 27, 29, 31, 33, 35, 37, and 39. The CDRs are determined according to the Kabat definition manner, the Chothia definition manner, the AbM definition manner, the Contact definition manner, or the IMGT definition manner in Tables 1-20, and the FRs of the corresponding definition manners according to the structure of the heavy chain variable region.

In an example of the IMGT definition manner, the heavy chain FR of the anti-CDH17 antibody or an antigen-binding fragment includes any one of the following (A)-(T).
(A) The HFR1 includes an amino acid sequence shown in SEQ ID NO: 414; the HFR2 includes an amino acid sequence shown in SEQ ID NO: 417; the HFR3 includes an amino acid sequence shown in SEQ ID NO: 450, and the HFR4 includes an amino acid sequence shown in SEQ ID NO: 490.
(B) The HFR1 includes an amino acid sequence shown in SEQ ID NO: 405; the HFR2 includes an amino acid sequence shown in SEQ ID NO: 444; the HFR3 includes an amino acid sequence shown in SEQ ID NO: 452, and the HFR4 includes an amino acid sequence shown in SEQ ID NO: 491.
(C) The HFR1 includes an amino acid sequence shown in SEQ ID NO: 399; the HFR2 includes an amino acid sequence shown in SEQ ID NO: 419; the HFR3 includes an amino acid sequence shown in SEQ ID NO: 454, and the HFR4 includes an amino acid sequence shown in SEQ ID NO: 491.
(D) The HFR1 includes an amino acid sequence shown in SEQ ID NO: 414; the HFR2 includes an amino acid sequence shown in SEQ ID NO: 420; the HFR3 includes an amino acid sequence shown in SEQ ID NO: 456, and the HFR4 includes an amino acid sequence shown in SEQ ID NO: 493.
(E) The HFR1 includes an amino acid sequence shown in SEQ ID NO: 404; the HFR2 includes an amino acid sequence shown in SEQ ID NO: 421; the HFR3 includes an amino acid sequence shown in SEQ ID NO: 458, and the HFR4 includes an amino acid sequence shown in SEQ ID NO: 492.
(F) The HFR1 includes an amino acid sequence shown in SEQ ID NO: 411; the HFR2 includes an amino acid sequence shown in SEQ ID NO: 423; the HFR3 includes an amino acid sequence shown in SEQ ID NO: 460, and the HFR4 includes an amino acid sequence shown in SEQ ID NO: 493.
(G) The HFR1 includes an amino acid sequence shown in SEQ ID NO: 407; the HFR2 includes an amino acid sequence shown in SEQ ID NO: 424; the HFR3 includes an amino acid sequence shown in SEQ ID NO: 462, and the HFR4 includes an amino acid sequence shown in SEQ ID NO: 493.
(H) The HFR1 includes an amino acid sequence shown in SEQ ID NO: 401; the HFR2 includes an amino acid sequence shown in SEQ ID NO: 425; the HFR3 includes an amino acid sequence shown in SEQ ID NO: 464, and the HFR4 includes an amino acid sequence shown in SEQ ID NO: 488.
(I) The HFR1 includes an amino acid sequence shown in SEQ ID NO: 409; the HFR2 includes an amino acid sequence shown in SEQ ID NO: 427; the HFR3 includes an amino acid sequence shown in SEQ ID NO: 466, and the HFR4 includes an amino acid sequence shown in SEQ ID NO: 491.
(J) The HFR1 includes an amino acid sequence shown in SEQ ID NO: 415; the HFR2 includes an amino acid sequence shown in SEQ ID NO: 429; the HFR3 includes an amino acid sequence shown in SEQ ID NO: 468, and the HFR4 includes an amino acid sequence shown in SEQ ID NO: 493.
(K) The HFR1 includes an amino acid sequence shown in SEQ ID NO: 403; the HFR2 includes an amino acid sequence shown in SEQ ID NO: 444; the HFR3 includes an amino acid sequence shown in SEQ ID NO: 470, and the HFR4 includes an amino acid sequence shown in SEQ ID NO: 491.
(L) The HFR1 includes an amino acid sequence shown in SEQ ID NO: 410; the HFR2 includes an amino acid sequence shown in SEQ ID NO: 430; the HFR3 includes an amino acid sequence shown in SEQ ID NO: 471, and the HFR4 includes an amino acid sequence shown in SEQ ID NO: 491.
(M) The HFR1 includes an amino acid sequence shown in SEQ ID NO: 406; the HFR2 includes an amino acid sequence shown in SEQ ID NO: 432; the HFR3 includes an amino acid sequence shown in SEQ ID NO: 473, and the HFR4 includes an amino acid sequence shown in SEQ ID NO: 491.
(N) The HFR1 includes an amino acid sequence shown in SEQ ID NO: 400; the HFR2 includes an amino acid sequence shown in SEQ ID NO: 434; the HFR3 includes an amino acid sequence shown in SEQ ID NO: 475, and the HFR4 includes an amino acid sequence shown in SEQ ID NO: 492.
(O) The HFR1 includes an amino acid sequence shown in SEQ ID NO: 412; the HFR2 includes an amino acid sequence shown in SEQ ID NO: 435; the HFR3 includes an amino acid sequence shown in SEQ ID NO: 476, and the HFR4 includes an amino acid sequence shown in SEQ ID NO: 492.
(P) The HFR1 includes an amino acid sequence shown in SEQ ID NO: 408; the HFR2 includes an amino acid sequence shown in SEQ ID NO: 437; the HFR3 includes an amino acid sequence shown in SEQ ID NO: 478, and the HFR4 includes an amino acid sequence shown in SEQ ID NO: 491.
(Q) The HFR1 includes an amino acid sequence shown in SEQ ID NO: 398; the HFR2 includes an amino acid sequence shown in SEQ ID NO: 438; the HFR3 includes an amino acid sequence shown in SEQ ID NO: 480, and the HFR4 includes an amino acid sequence shown in SEQ ID NO: 489.
(R) The HFR1 includes an amino acid sequence shown in SEQ ID NO: 398; the HFR2 includes an amino acid sequence shown in SEQ ID NO: 440; the HFR3 includes an amino acid sequence shown in SEQ ID NO: 482, and the HFR4 includes an amino acid sequence shown in SEQ ID NO: 493.
(S) The HFR1 includes an amino acid sequence shown in SEQ ID NO: 413; the HFR2 includes an amino acid sequence shown in SEQ ID NO: 441; the HFR3 includes an amino acid sequence shown in SEQ ID NO: 484, and the HFR4 includes an amino acid sequence shown in SEQ ID NO: 492.
(T) The HFR1 includes an amino acid sequence shown in SEQ ID NO: 402; the HFR2 includes an amino acid sequence shown in SEQ ID NO: 443; the HFR3 includes an amino acid sequence shown in SEQ ID NO: 486, and the HFR4 includes an amino acid sequence shown in SEQ ID NO: 493.

In an optional implementation, the LFR1, LFR2, LFR3, and LFR4 include the LFR1, LFR2, LFR3, and LFR4 of the light chain variable region shown in any one of SEQ ID NO: 2, 4, 6, 8, 10, 12, 14, 16, 18, 20, 22, 24, 26, 28, 30, 32, 34, 36, 38, and 40. The CDRs are determined according to the Kabat definition manner, the Chothia definition manner, the AbM definition manner, the Contact definition manner, or the IMGT definition manner in Tables 1-20, and the FRs of the corresponding definition manners according to the structure of the light chain variable region.

In an example of the IMGT definition manner, the light chain FR of the anti-CDH17 antibody or an antigen-binding fragment includes any one of the following (A')-(T').
(A') The LFR1 includes an amino acid sequence shown in SEQ ID NO: 495; the LFR2 includes an amino acid sequence shown in SEQ ID NO: 553; the LFR3 includes an amino acid sequence shown in SEQ ID NO: 555, and the LFR4 includes an amino acid sequence shown in SEQ ID NO: 587.
(B') The LFR1 includes an amino acid sequence shown in SEQ ID NO: 527; the LFR2 includes an amino acid sequence shown in SEQ ID NO: 546; the LFR3 includes an amino acid sequence shown in SEQ ID NO: 585, and the LFR4 includes an amino acid sequence shown in SEQ ID NO: 587.
(C') The LFR1 includes an amino acid sequence shown in SEQ ID NO: 497; the LFR2 includes an amino acid sequence shown in SEQ ID NO: 529; the LFR3 includes an amino acid sequence shown in SEQ ID NO: 557, and the LFR4 includes an amino acid sequence shown in SEQ ID NO: 586.
(D') The LFR1 includes an amino acid sequence shown in SEQ ID NO: 499; the LFR2 includes an amino acid sequence shown in SEQ ID NO: 530; the LFR3 includes an amino acid sequence shown in SEQ ID NO: 559, and the LFR4 includes an amino acid sequence shown in SEQ ID NO: 587.
(E') The LFR1 includes an amino acid sequence shown in SEQ ID NO: 501; the LFR2 includes an amino acid sequence shown in SEQ ID NO: 547; the LFR3 includes an amino acid sequence shown in SEQ ID NO: 583, and the LFR4 includes an amino acid sequence shown in SEQ ID NO: 587.
(F') The LFR1 includes an amino acid sequence shown in SEQ ID NO: 525; the LFR2 includes an amino acid sequence shown in SEQ ID NO: 531; the LFR3 includes an amino acid sequence shown in SEQ ID NO: 561, and the LFR4 includes an amino acid sequence shown in SEQ ID NO: 588.
(G') The LFR1 includes an amino acid sequence shown in SEQ ID NO: 503; the LFR2 includes an amino acid sequence shown in SEQ ID NO: 547; the LFR3 includes an amino acid sequence shown in SEQ ID NO: 583, and the LFR4 includes an amino acid sequence shown in SEQ ID NO: 587.
(H') The LFR1 includes an amino acid sequence shown in SEQ ID NO: 505; the LFR2 includes an amino acid sequence shown in SEQ ID NO: 532; the LFR3 includes an amino acid sequence shown in SEQ ID NO: 563, and the LFR4 includes an amino acid sequence shown in SEQ ID NO: 587.
(I') The LFR1 includes an amino acid sequence shown in SEQ ID NO: 507; the LFR2 includes an amino acid sequence shown in SEQ ID NO: 534; the LFR3 includes an amino acid sequence shown in SEQ ID NO: 565, and the LFR4 includes an amino acid sequence shown in SEQ ID NO: 589.
(J') The LFR1 includes an amino acid sequence shown in SEQ ID NO: 509; the LFR2 includes an amino acid sequence shown in SEQ ID NO: 536; the LFR3 includes an amino acid sequence shown in SEQ ID NO: 567, and the LFR4 includes an amino acid sequence shown in SEQ ID NO: 587.
(K') The LFR1 includes an amino acid sequence shown in SEQ ID NO: 527; the LFR2 includes an amino acid sequence shown in SEQ ID NO: 546; the LFR3 includes an amino acid sequence shown in SEQ ID NO: 585, and the LFR4 includes an amino acid sequence shown in SEQ ID NO: 587.
(L') The LFR1 includes an amino acid sequence shown in SEQ ID NO: 511; the LFR2 includes an amino acid sequence shown in SEQ ID NO: 538; the LFR3 includes an amino acid sequence shown in SEQ ID NO: 569, and the LFR4 includes an amino acid sequence shown in SEQ ID NO: 589.
(M') The LFR1 includes an amino acid sequence shown in SEQ ID NO: 513; the LFR2 includes an amino acid sequence shown in SEQ ID NO: 546; the LFR3 includes an amino acid sequence shown in SEQ ID NO: 585, and the LFR4 includes an amino acid sequence shown in SEQ ID NO: 587.
(N') The LFR1 includes an amino acid sequence shown in SEQ ID NO: 515; the LFR2 includes an amino acid sequence shown in SEQ ID NO: 540; the LFR3 includes an amino acid sequence shown in SEQ ID NO: 571, and the LFR4 includes an amino acid sequence shown in SEQ ID NO: 590.
(O') The LFR1 includes an amino acid sequence shown in SEQ ID NO: 517; the LFR2 includes an amino acid sequence shown in SEQ ID NO: 542; the LFR3 includes an amino acid sequence shown in SEQ ID NO: 573, and the LFR4 includes an amino acid sequence shown in SEQ ID NO: 589.
(P') The LFR1 includes an amino acid sequence shown in SEQ ID NO: 519; the LFR2 includes an amino acid sequence shown in SEQ ID NO: 544; the LFR3 includes an amino acid sequence shown in SEQ ID NO: 575, and the LFR4 includes an amino acid sequence shown in SEQ ID NO: 587.
(Q') The LFR1 includes an amino acid sequence shown in SEQ ID NO: 523; the LFR2 includes an amino acid sequence shown in SEQ ID NO: 548; the LFR3 includes an amino acid sequence shown in SEQ ID NO: 584, and the LFR4 includes an amino acid sequence shown in SEQ ID NO: 587.
(R') The LFR1 includes an amino acid sequence shown in SEQ ID NO: 523; the LFR2 includes an amino acid sequence shown in SEQ ID NO: 548; the LFR3 includes an amino acid sequence shown in SEQ ID NO: 584, and the LFR4 includes an amino acid sequence shown in SEQ ID NO: 587.
(S') The LFR1 includes an amino acid sequence shown in SEQ ID NO: 525; the LFR2 includes an amino acid sequence shown in SEQ ID NO: 545; the LFR3 includes an amino acid sequence shown in SEQ ID NO: 577, and the LFR4 includes an amino acid sequence shown in SEQ ID NO: 587.
(T') The LFR1 includes an amino acid sequence shown in SEQ ID NO: 521; the LFR2 includes an amino acid sequence shown in SEQ ID NO: 553; the LFR3 includes an amino acid sequence shown in SEQ ID NO: 579, and the LFR4 includes an amino acid sequence shown in SEQ ID NO: 587.

In an optional implementation, the above anti-CDH17 antibody or the antigen-binding fragment thereof includes the heavy chain variable region having the amino acid sequence shown in any one of SEQ ID NO: 1, 3, 5, 7, 9, 11, 13, 15, 17, 19, 21, 23, 25, 27, 29, 31, 33, 35, 37, and 39 or having a sequence having at least 64% (e.g., 64%, 65%, 68%, 70%, 72%, 75%, 77%, 80%, 85%, 90%, 91%, 92%, 93%, 94%, 95%, 96%, 97%, 98%, 99%, or 100%) sequence identity to the amino acid sequence shown in any one of SEQ ID NO: 1, 3, 5, 7, 9, 11, 13, 15, 17, 19, 21, 23, 25, 27, 29, 31, 33, 35, 37, and 39.

In an optional implementation, the above anti-CDH17 antibody or the antigen-binding fragment thereof includes the light chain variable region having the amino acid sequence shown in any one of SEQ ID NO: 2, 4, 6, 8, 10, 12, 14, 16, 18, 20, 22, 24, 26, 28, 30, 32, 34, 36, 38, and 40 or having a sequence having at least 70% (e.g., 70%, 72%, 75%, 77%, 80%, 85%, 90%, 91%, 92%, 93%, 94%, 95%, 96%, 97%, 98%, 99%, or 100%) sequence identity to the amino acid sequence shown in any one of SEQ ID NO: 2, 4, 6, 8, 10, 12, 14, 16, 18, 20, 22, 24, 26, 28, 30, 32, 34, 36, 38, and 40.

In an optional implementation, the anti-CDH17 antibody or the antigen-binding fragment thereof includes any one of the following heavy chain variable regions and light chain variable regions:
(a") the heavy chain variable region having the amino acid sequence shown in SEQ ID NO: 1; and the light chain variable region having the amino acid sequence shown in SEQ ID NO: 2;
(b") the heavy chain variable region having the amino acid sequence shown in SEQ ID NO: 3; and the light chain variable region having the amino acid sequence shown in SEQ ID NO: 4;
(c") the heavy chain variable region having the amino acid sequence shown in SEQ ID NO: 5; and the light chain variable region having the amino acid sequence shown in SEQ ID NO: 6;
(d") the heavy chain variable region having the amino acid sequence shown in SEQ ID NO: 7; and the light chain variable region having the amino acid sequence shown in SEQ ID NO: 8;
(e") the heavy chain variable region having the amino acid sequence shown in SEQ ID NO: 9; and the light chain variable region having the amino acid sequence shown in SEQ ID NO: 10;
(f") the heavy chain variable region having the amino acid sequence shown in SEQ ID NO: 11; and the light chain variable region having the amino acid sequence shown in SEQ ID NO: 12;
(g") the heavy chain variable region having the amino acid sequence shown in SEQ ID NO: 13; and the light chain variable region having the amino acid sequence shown in SEQ ID NO: 14;
(h") the heavy chain variable region having the amino acid sequence shown in SEQ ID NO: 15; and the light chain variable region having the amino acid sequence shown in SEQ ID NO: 16;
(i") the heavy chain variable region having the amino acid sequence shown in SEQ ID NO: 17; and the light chain variable region having the amino acid sequence shown in SEQ ID NO: 18;
(j") the heavy chain variable region having the amino acid sequence shown in SEQ ID NO: 19; and the light chain variable region having the amino acid sequence shown in SEQ ID NO: 20;
(k") the heavy chain variable region having the amino acid sequence shown in SEQ ID NO: 21; and the light chain variable region having the amino acid sequence shown in SEQ ID NO: 22;
(l") the heavy chain variable region having the amino acid sequence shown in SEQ ID NO: 23; and the light chain variable region having the amino acid sequence shown in SEQ ID NO: 24;
(m") the heavy chain variable region having the amino acid sequence shown in SEQ ID NO: 25; and the light chain variable region having the amino acid sequence shown in SEQ ID NO: 26;
(n") the heavy chain variable region having the amino acid sequence shown in SEQ ID NO: 27; and the light chain variable region having the amino acid sequence shown in SEQ ID NO: 28;
(o") the heavy chain variable region having the amino acid sequence shown in SEQ ID NO: 29; and the light chain variable region having the amino acid sequence shown in SEQ ID NO: 30;
(p") the heavy chain variable region having the amino acid sequence shown in SEQ ID NO: 31; and the light chain variable region having the amino acid sequence shown in SEQ ID NO: 32;
(q") the heavy chain variable region having the amino acid sequence shown in SEQ ID NO: 33; and the light chain variable region having the amino acid sequence shown in SEQ ID NO: 34;
(r") the heavy chain variable region having the amino acid sequence shown in SEQ ID NO: 35; and the light chain variable region having the amino acid sequence shown in SEQ ID NO: 36;
(s") the heavy chain variable region having the amino acid sequence shown in SEQ ID NO: 37; and the light chain variable region having the amino acid sequence shown in SEQ ID NO: 38; and
(t") the heavy chain variable region having the amino acid sequence shown in SEQ ID NO: 39; and the light chain variable region having the amino acid sequence shown in SEQ ID NO: 40.

In an optional implementation, the antigen-binding fragment includes one or more of F(ab')2, Fab', Fab, Fv, scFv, dsFv, and a minimum recognition unit of the antibody.

In an optional implementation, the anti-CDH17 antibody or the antigen-binding fragment thereof includes part or all sequences of a constant region, where the constant region is derived from at least one of a mouse-derived antibody, a human antibody, a primate-derived antibody, or mutants thereof.

In an optional implementation, a heavy chain constant region comprises sequences of part or all constant regions of any one of IgG1, IgG2a, IgG2b, IgG3, IgG4, IgA, IgM, IgE, and IgD.

In an optional implementation, the heavy chain constant region includes sequences of part or all constant regions of the IgG1, and the anti-CDH17 antibody or the antigen-binding fragment thereof includes any one of the following heavy chain variable regions and light chain variable regions:
(a") the heavy chain variable region having the amino acid sequence shown in SEQ ID NO: 1; and the light chain variable region having the amino acid sequence shown in SEQ ID NO: 2;
(c") the heavy chain variable region having the amino acid sequence shown in SEQ ID NO: 5; and the light chain variable region having the amino acid sequence shown in SEQ ID NO: 6; and
(t") the heavy chain variable region having the amino acid sequence shown in SEQ ID NO: 39; and the light chain variable region having the amino acid sequence shown in SEQ ID NO: 40.

In an optional implementation, the heavy chain constant region includes sequences of part or all constant regions of the IgG2a, and the anti-CDH17 antibody or the antigen-binding fragment thereof includes any one of the following heavy chain variable regions and light chain variable regions:
(d") the heavy chain variable region having the amino acid sequence shown in SEQ ID NO: 7; and the light chain variable region having the amino acid sequence shown in SEQ ID NO: 8;
(f") the heavy chain variable region having the amino acid sequence shown in SEQ ID NO: 11; and the light chain variable region having the amino acid sequence shown in SEQ ID NO: 12;
(g") the heavy chain variable region having the amino acid sequence shown in SEQ ID NO: 13; and the light chain variable region having the amino acid sequence shown in SEQ ID NO: 14;
(i") the heavy chain variable region having the amino acid sequence shown in SEQ ID NO: 17; and the light chain variable region having the amino acid sequence shown in SEQ ID NO: 18;
(j") the heavy chain variable region having the amino acid sequence shown in SEQ ID NO: 19; and the light chain variable region having the amino acid sequence shown in SEQ ID NO: 20;
(n") the heavy chain variable region having the amino acid sequence shown in SEQ ID NO: 27; and the light chain variable region having the amino acid sequence shown in SEQ ID NO: 28;
(o") the heavy chain variable region having the amino acid sequence shown in SEQ ID NO: 29; and the light chain variable region having the amino acid sequence shown in SEQ ID NO: 30;
(p") the heavy chain variable region having the amino acid sequence shown in SEQ ID NO: 31; and the light chain variable region having the amino acid sequence shown in SEQ ID NO: 32;
(q") the heavy chain variable region having the amino acid sequence shown in SEQ ID NO: 33; and the light chain variable region having the amino acid sequence shown in SEQ ID NO: 34;
(r") the heavy chain variable region having the amino acid sequence shown in SEQ ID NO: 35; and the light chain variable region having the amino acid sequence shown in SEQ ID NO: 36; and
(s") the heavy chain variable region having the amino acid sequence shown in SEQ ID NO: 37; and the light chain variable region having the amino acid sequence shown in SEQ ID NO: 38.

In an optional implementation, the heavy chain constant region includes sequences of part or all constant regions of the IgG2b, and the anti-CDH17 antibody or the antigen-binding fragment thereof includes any one of the following heavy chain variable regions and light chain variable regions:
(b") the heavy chain variable region having the amino acid sequence shown in SEQ ID NO: 3; and the light chain variable region having the amino acid sequence shown in SEQ ID NO: 4;
(e") the heavy chain variable region having the amino acid sequence shown in SEQ ID NO: 9; and the light chain variable region having the amino acid sequence shown in SEQ ID NO: 10;
(h") the heavy chain variable region having the amino acid sequence shown in SEQ ID NO: 15; and the light chain variable region having the amino acid sequence shown in SEQ ID NO: 16;
(k") the heavy chain variable region having the amino acid sequence shown in SEQ ID NO: 21; and the light chain variable region having the amino acid sequence shown in SEQ ID NO: 22;
(l") the heavy chain variable region having the amino acid sequence shown in SEQ ID NO: 23; and the light chain variable region having the amino acid sequence shown in SEQ ID NO: 24; and
(m") the heavy chain variable region having the amino acid sequence shown in SEQ ID NO: 25; and the light chain variable region having the amino acid sequence shown in SEQ ID NO: 26.

In an optional implementation, the light chain constant region is a κ or λ chain.

In an optional implementation, the constant region-source species include one or more combinations of mice, rats, guinea pigs, hamsters, rabbits, ferrets, cats, dogs, goats, sheep, cows, pigs, horses, monkeys, and humans.

In an optional implementation, the anti-CDH17 antibody or the antigen-binding fragment thereof has one or more properties in the following (i) to (iv).
(i) The anti-CDH17 antibody or the antigen-binding fragment thereof binds to human CDH17 with EC50 not exceeding 1 µg/mL (e.g., not exceeding 0.01 µg/mL, 0.02 µg/mL, 0.03 µg/mL, 0.04 µg/mL, 0.05 µg/mL, 0.06 µg/mL, 0.07 µg/mL, 0.08 µg/mL, 0.09 µg/mL, 0.1 µg/mL, 0.2 µg/mL, 0.3 µg/mL, 0.4 µg/mL, 0.5 µg/mL, 0.6 µg/mL, 0.7 µg/mL, 0.8 µg/mL, 0.9 µg/mL, or 1 µg/mL), where the binding is determined through a flow cytometry fluorescence sorting technology; and the determination method may be referenced to Embodiment 2.
(ii) The anti-CDH17 antibody or the antigen-binding fragment thereof binds to monkey CDH17 with EC50 not exceeding 1 µg/mL (e.g., not exceeding 0.01 µg/mL, 0.02 µg/mL, 0.03 µg/mL, 0.04 µg/mL, 0.05 µg/mL, 0.06 µg/mL, 0.07 µg/mL, 0.08 µg/mL, 0.09 µg/mL, 0.1 µg/mL, 0.2 µg/mL, 0.3 µg/mL, 0.4 µg/mL, 0.5 µg/mL, 0.6 µg/mL, 0.7 µg/mL, 0.8 µg/mL, 0.9 µg/mL, or 1 µg/mL), where the binding is determined through an ELISA technology; and the determination method may be referenced to Embodiment 3.
(iii) The anti-CDH17 antibody or the antigen-binding fragment thereof has ADCC activity (antibody-dependent cell-mediated cytotoxicity), where the ADCC activity is detected by the flow cytometry fluorescence sorting technology or a lactate dehydrogenase (LDH) cytotoxicity assayl, and the determination method may be referenced to Embodiment 5.
(iv) The anti-CDH17 antibody or the antigen-binding fragment thereof has mediated cell endocytosis activity, where the mediated cell endocytosis activity is detected through the ELISA technology, and the determination method may be referenced to Embodiment 6.

In an optional implementation, the anti-CDH17 antibody or the antigen-binding fragment thereof binds to the mouse CDH17 or does not bind to the mouse CDH17.

In an optional implementation, the CDH17 in (i) to (iii) above is either an isolated CDH17 molecule or cells expressing the CDH17, where the cells include, but are not limited to, mammalian cells, such as tumor cells.

The anti-CDH17 antibody or the antigen-binding fragment thereof provided in the first aspect can specifically bind to a CDH17 protein. In some implementations, the anti-CDH17 antibody or the antigen-binding fragment thereof can bind to the CDH17 protein expressed in the mammalian cells (e.g., CHO cells) or to the CDH17 endogenously expressed in the tumor cells (e.g., AsPC-1 cells endogenously expressing the CDH17). The anti-CDH17 antibody or the antigen-binding fragment thereof provided in the first aspect has the ADCC activity, and can mediate cell endocytosis, thereby providing new possibilities for cancer treatment and/or prevention.

In a second aspect, the present disclosure provides an anti-CDH17 antibody or an antigen-binding fragment thereof, which competitively binds to CDH17 with the anti-CDH17 antibody of the first or second aspect, or an epitope thereof binding to a CDH17 antigen is identical to an epitope of the anti-CDH17 antibody of the first or second aspect binding to the CDH17 antigen.

In a third aspect, the present disclosure provides a polyspecific antibody, including the anti-CDH17 antibody or the antigen-binding fragment thereof shown in any one of the above.

In an optional implementation, the polyspecific antibody further includes an antibody or an antigen-binding fragment thereof that specifically binds to other target molecules. Exemplarily, other target molecules include, but are not limited to, tumor surface antigens or immune checkpoints.

Exemplarily, the tumor surface antigens include, but are not limited to, MUC16, VEGFR-2 (KDR/FIK-1), K-RAS, CD2, CD3, CD19, CD19, CD20, CD21, CD22, CD30, CD32B, CD33, CD38, CD39, CD40, CD45, CD52, CD70, CD80, CD60, CD62, CD72, CD79a, CD79B, CD123, EGFR, HER2, HER3, HER4, GPC3, CLL1, TGF-β, TGF-beta RII, VEGF, GD3, CCR4, CCR5, BCMA, CTLA4, mesothelin, and OX40.

Exemplarily, the immune checkpoints include, but are not limited to, PD-1, PD-L1, PD-L2, TIGIT, CD47, CD27, CD28, CD40, CD122, CD137, CD94/NKG2A, OX40, GITR, ICOS, A2AR, B7-H3, B7-H4, BTLA, LAG3, TIM-3, VISTA, GARP, PS, CSF1R, TDO, CTLA-4, IDO, KIR, GITR, TNFR, FasR/DcR, and CEACAM1.

In a fourth aspect, the present disclosure provides a CAR, including an ECD. The above ECD includes an antigen-binding domain. Moreover, the above antigen-binding domain includes the anti-CDH17 antibody or the antigen-binding fragment thereof shown in any one of the above.

In an optional implementation, the anti-CDH17 antibody or the antigen-binding fragment thereof in the CAR is a single-chain antibody scFv.

A fifth aspect provides a nucleic acid molecule, which encodes the foregoing anti-CDH17 antibody or the antigen-binding fragment thereof of the first or second aspect, or the polyspecific antibody of the third aspect, or the CAR of the fourth aspect.

In an optional implementation, the nucleic acid molecule is an isolated nucleic acid molecule.

A sixth aspect provides a vector. The vector carries the nucleic acid molecule of the fifth aspect.

In a seventh aspect, the present disclosure provides a recombinant cell, expressing the anti-CDH17 antibody or the antigen-binding fragment thereof of the first or second aspect, or expressing the polyspecific antibody of the third aspect, or expressing the CAR of the fourth aspect, or including the nucleic acid molecule of the fifth aspect, or including the vector of the sixth aspect.

In an eighth aspect, the present disclosure provides an engineered immune cell, expressing the CAR of the fourth aspect or including the nucleic acid molecule encoding the CAR of the fourth aspect.

In a ninth aspect, the present disclosure provides a pharmaceutical composition, including the anti-CDH17 antibody or the antigen-binding fragment thereof of the first or second aspect, or the polyspecific antibody of the third aspect, or the CAR of the fourth aspect, or the nucleic acid molecule of the fifth aspect, or the vector of the sixth aspect, or the recombinant cell of the seventh aspect, or the engineered immune cell of the eighth aspect.

In an optional implementation, the above pharmaceutical composition further includes pharmaceutically acceptable carriers and/or excipients. The acceptable carriers and the pharmaceutically acceptable excipient may be selected from any carrier and/or excipient known in the art and commonly used. Examples of the carrier include, but are not limited to, any physiologically compatible solvent, a dispersing medium, a coating agent, an antibacterial agent, an antifungal agent, an isotonic agent, a delayed absorption agent, etc.; and examples of the excipient include, but are not limited to, a filler, a disintegrant, a preservative, a cosolvent, an emulsifying agent, etc.

In an optional implementation, the pharmaceutical composition further includes one or more drug active ingredients with other therapeutic effects, including but not limited to a combination of one or more of the following: a chemotherapeutic agent, an anticancer drug, a radiotherapy agent, an immunotherapy agent, an anti-angiogenic agent, a targeted therapeutic agent, a cell therapeutic agent, a gene therapeutic agent, a hormone therapeutic agent, an antiviral agent, antibiotics, analgesics, an antioxidant, a metal chelator, and cytokines.

In an optional implementation, the pharmaceutical composition is used as a drug for treating, preventing, or alleviating diseases, symptoms, or conditions associated with the CDH17.

In a tenth aspect, the present disclosure provides any one of the following applications of the anti-CDH17 antibody or the antigen-binding fragment thereof of the first or second aspect, or the polyspecific antibody of the third aspect, or the CAR of the fourth aspect, or the nucleic acid molecule of the fifth aspect, or the vector of the sixth aspect, or the recombinant cell of the seventh aspect, or the engineered immune cell of the eighth aspect, or the pharmaceutical composition of the ninth aspect.
(I) CDH17 or cells expressing the CDH17 are detected.
(II) A product for detecting the CDH17 or cells expressing the CDH17 is prepared.
(III) The anti-CDH17 antibody or the antigen-binding fragment thereof, or the biomaterial, or the pharmaceutical composition is used for treating, preventing, or alleviating diseases, symptoms, or conditions associated with the CDH17.
(IV) A pharmaceutical composition for treating, preventing, or alleviating diseases, symptoms, or conditions associated with the CDH17 is prepared.

In an optional implementation, the above application of aspect (I) may use the anti-CDH17 antibody or the antigen-binding fragment thereof to achieve specific targeting binding to the CDH17, and realize the detection of the CDH17 or the cells expressing the CDH17 via an immunodetection technology. For example, when the antibody or the antigen-binding fragment thereof is an immunoconjugate, such as connected to a fluorescent group, a fluorescence detection apparatus may be used to position or detect the CDH17 in real time. For example, it may be used in techniques such as immunoblotting, immunoprecipitation, or flow cytometry, etc. that use the specific binding properties of the CDH17 antigen and antibody to detect the CDH17 or the cells expressing the CDH17. Accordingly, in the aspect (II) above, those skilled in the art may select the composition of reagents in the kit based on actual detection methods, including but not limited to an antagonist, an anti-CDH17 antibody, or a drug reference material; a protein purification column; an immunoglobulin affinity purification buffer; a cell assay diluent; instructions or literature, etc. The kit may also be used to detect the diseases, symptoms, or conditions associated with the CDH17.

In an optional implementation, the application described in aspect (I) above is for non-diagnostic and non-therapeutic purposes.

In an optional implementation, a method for treating, preventing, or alleviating diseases, symptoms, or conditions associated with CDH17 includes: a therapeutically effective dosage of the foregoing anti-CDH17 antibody or the antigen-binding fragment thereof and/or the foregoing pharmaceutical composition is applied to a subject.

In an eleventh aspect, the present disclosure provides a method for preparing the foregoing anti-CDH17 antibody or the antigen-binding fragment thereof. The method includes the following steps.
(a) The foregoing recombinant cell is cultured under a condition expressing the anti-CDH17 antibody or the antigen-binding fragment thereof.
(b) The anti-CDH17 antibody or the antigen-binding fragment thereof obtained in step (a) is isolated and purified.

In an optional implementation, the diseases, symptoms, or conditions "associated with the CDH17" in any of the above aspects include: solid tumors or hematologic malignancies.

In an optional implementation, the diseases, symptoms, or conditions "associated with the CDH17" are characterized by high expression of the CDH17.

In an optional implementation, the solid tumors associated with the diseases, symptoms, or conditions in any of the above aspects include one or more of the following: gastric cancer, colorectal cancer, pancreatic cancer, liver cancer, breast cancer, lung cancer, colon cancer, bladder cancer, thyroid cancer, skin cancer, esophageal cancer, and cervical cancer.

In an optional implementation, the hematologic malignancies associated with the diseases, symptoms, or conditions in any of the above aspects include one or more of the following groups: Chronic Lymphocytic Leukemia (CLL), Chronic Myeloid Leukemia (CML), Acute Lymphoblastic Leukemia (ALL), Acute Myeloid Leukemia (AML), multiple myeloma, and T- or B-cell lymphoma.

In an optional implementation, the antibody or the antigen-binding fragment or pharmaceutical composition of the present disclosure may be administered at a therapeutically effective dosage ranging from approximately 0.001 mg/kg to approximately 1000 mg/kg. In some implementations, an administration dosage may be adjusted as treatment progresses. For example, in some implementations, an initial administration dosage may be higher than subsequent administration dosages. In some implementations, the administration dosage is adjusted during the course of treatment based on the response of the subject.

In an optional implementation, the antibody or the antigen-binding fragment thereof or pharmaceutical composition of the present disclosure may be administered alone or in combination with a second therapeutic agent at a therapeutically effective dosage. For example, the antibody or the antigen-binding fragment thereof or pharmaceutical composition of the present disclosure may be administered in combination with the second therapeutic agent (e.g., a chemotherapeutic agent, an anticancer drug, a radiotherapy agent, an immunotherapy agent, an anti-angiogenic agent, a targeted therapeutic agent, a cell therapeutic agent, a gene therapeutic agent, a hormone therapeutic agent, an antiviral agent, antibiotics, analgesics, an antioxidant, a metal chelator, or cytokines).

In an optional implementation, the antibody or the antigen-binding fragment thereof or pharmaceutical composition of the present disclosure may be administered concurrently with one or more additional therapeutic agents when used in combination with such agents. In some implementations, the antibody or the antigen-binding fragment thereof and the additional therapeutic agents may be administered simultaneously as part of the same composition. However, the antibody or the antigen-binding fragment thereof or pharmaceutical composition used "in combination with" other therapeutic agents need not be administered simultaneously or in the same composition as the therapeutic agent. The term "in combination" in the present disclosure also includes the antibody or the antigen-binding fragment thereof or pharmaceutical composition administered before or after another therapeutic agent being also considered to be "in combination" with the therapeutic agent, that is, the antibody or the antigen-binding fragment thereof or pharmaceutical composition is administered with a second substance via different administration routes.

In a twelfth aspect, the present disclosure provides a kit for detecting CDH17. The kit includes the foregoing anti-CDH17 antibody or the antigen-binding fragment thereof.

In an optional implementation, the product in any of the above aspects, such as but not limited to a reagent, a kit, a blocker, and the anti-CDH17 antibody or the antigen-binding fragment thereof in the pharmaceutical composition, may be coupled to at least one of a diagnostic agent and/or a therapeutic agent to form the immunoconjugate.

The diagnostic agent includes one or more of the following: a radioactive contrast agent, a paramagnetic ion, a metal, a fluorescent label, a chemiluminescent label, an ultrasound contrast agent, and a photosensitizer.

The therapeutic agent includes one or more of the following: a cytotoxic agent, a drug, a radionuclide, a boron atom, an immunomodulator, an anti-apoptotic agent, a photosensitizing agent, an immunoconjugate, and oligonucleotide.

The radionuclide includes, but is not limited to, one or more of ¹¹⁰In, ¹¹¹In, ¹⁷⁷Lu, ¹⁸F, ⁵²Fe, ⁶²Cu, ⁶⁴Cu, ⁶⁷Cu, ⁶⁷Ga, ⁶⁸Ga, ⁸⁶Y, ⁹⁰Y, ⁸⁹Zr, ⁹⁴mTc, ⁹⁴Tc, ⁹⁹mTc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ⁵²mMn, ⁵⁵Co, ⁷²As, ⁷⁵Br, ⁷⁶Br, ⁸²mRb, and ⁸³Sr

The paramagnetic ion includes, but are not limited to, one or more of chromium(III), manganese(II), iron(III), iron(II), cobalt(II), nickel(II), copper(II), neodymium(III), samarium(III), ytterbium(III), gadolinium(III), vanadium(II), terbium(III), dysprosium(III), holmium(III), and erbium(III).

The fluorescent label includes, but are not limited to, one or more of Alexa 350, Alexa 405, Alexa 430, Alexa 488, Alexa 555, Alexa 647, AMCA, aminoacridine, BODIPY630/650, BODIPY650/665, BODIPY-FL, BODIPY-R6G, BODIPY-TMR, BODIPY-TRX, 5-carboxy-4',5'-dichloro-2',7'-dimethoxyfluorescein, 5-carboxy-2',4',5',7'-tetrachlorofluorescein, 5-carboxyfluorescein, 5-carboxyrhodamine, 6-carboxyrhodamine, 6-carboxytetramethylrhodamine, Cascade Blue, Cy2, Cy3, Cy5, Cy7, 6-FAM, dansyl chloride, fluorescein, HEX, 6-JOE, NBD (7-nitrobenzo-2-oxa-1,3-diazole), Oregon Green488, Oregon Green 500, Oregon Green514, Pacific Blue, phthalic acid, terephthalic acid, isophthalic acid, cresol purple, cresol blue purple, brilliant cresyl blue, 4-aminobenzoic acid, erythrosine, phthalocyanine, azomethine, cyanine, xanthine, succinyl fluorescein, a rare earth metal porous compound, tripyridyl diamine europium, an europium porous compound or chelate, diamine, dicyanin, La Jolla blue dye, allophycocyanin, allococyanin B, phycocyanin C, phycocyanin R, thiamine, phycoerythrin, phycoerythrin R, REG, rhodamine green, rhodamine isothiocyanate, rhodamine red, ROX, TAMRA, TET, TRIT (tetramethylrhodamine isothiocyanate), tetramethylrhodamine, and Texas red.

The oligonucleotide includes, but is not limited to, one or more of shRNA, miRNA, and siRNA.

The drug includes, but is not limited to, methotrexate, fluorouracil, mercaptopurine, hydroxyurea, cytarabine, nitrogen mustard, cyclophosphamide, thiotepa, cisplatin, mitomycin, bleomycin, camptothecin, podophyllotoxin, actinomycin D, doxorubicin, daunorubicin, vincristine, paclitaxel, cephalotaxine, and L-asparaginase.

The immunomodulator includes, but is not limited to, cytokines, chemokines, stem cell growth factors, lymphotoxins, hematopoietic factors, colony-stimulating factors (CSF), interferons, erythropoietin, thrombopoietin, tumor necrosis factor (TNF), interleukins (IL), granulocyte colony-stimulating factor (G-CSF), granulocyte-macrophage colony-stimulating factor (GM-CSF), and stem cell growth factors.

The radionuclide includes, but is not limited to, one or more of ¹¹¹In, ¹¹¹At, ¹⁷⁷Lu, ²¹¹Bi, ²¹²Bi, ²¹³Bi, ²¹¹At, ⁶²Cu, ⁶⁷Cu, ⁹⁰Y, ¹²⁵I, ¹³¹I, ¹³³I, ³²P, ³³P, ⁴⁷Sc, ¹¹¹Ag, ⁶⁷Ga, ¹⁵³Sm, ¹⁶¹Tb, ¹⁵²Dy, ¹⁶⁶Dy, ¹⁶¹Ho, ¹⁶⁶Ho, ¹⁸⁶Re, ¹⁸⁸Re, ¹⁸⁹Re, ²¹¹Pb, ²¹²Pb, ²²³Ra, ²²⁵Ac, ⁷⁷As, ⁸⁹Sr, ⁹⁹Mo, ¹⁰⁵Rh, ¹⁴⁹Pm, ¹⁶⁹Er, ¹⁹⁴Ir, ⁵⁸Co, ⁸⁰mBr, ⁹⁹mTc, ¹⁰³mRh, ¹⁰⁹Pt, ¹¹⁹Sb, ¹⁸⁹mOs, ¹⁹²Ir, ²¹⁹Rn, ²¹⁵Po, ²²¹Fr, ²⁵⁵Fm, ¹¹C, ¹³N, ¹⁵O, ⁷⁵Br, ¹⁹⁸Au, ¹⁹⁹Au, ²²⁴Ac, ⁷⁷Br, ¹¹³mIn, ⁹⁵Ru, ⁹⁷Ru, ¹⁰³Ru, ¹⁰⁵Ru, ¹⁰⁷Hg, ²⁰³Hg, ¹²¹mTe, ¹²²mTe, ¹²⁵mTe, ¹⁶⁵Tm, ¹⁶⁷Tm, ¹⁶⁸Tm, ¹⁹⁷Pt, ¹⁰⁹Pd, ¹⁴²Pr, ¹⁴³Pr, ¹⁶¹Tb, ⁵⁷Co, ⁵⁸Co, ⁵¹Cr, ⁵⁹Fe, ⁷⁵Se, ²⁰¹Tl, ⁷⁶Br, and ¹⁶⁹Yb.

The present disclosure is further described through specific embodiments below. However, it should be understood that these embodiments are merely for the purpose of providing a more detailed description and should not be construed as limiting the scope of the present disclosure in any way.

### Embodiment 1 Production of mouse antibody for CDH17

### 1. Material preparation

In order to produce cells stably expressing human CDH17, CHO cells were transfected with a vector encoding the human CDH17 (SEQ ID NO: 591), then an antibody (Boehringer-Ingelheim) R2800 (a sequence from Patent CN110582513A, a heavy chain variable region amino acid sequence being shown in SEQ ID NO: 592, and a light chain variable region amino acid sequence being shown in SEQ ID NO: 593) targeting a CDH17 protein was used as a positive control, a flow cytometry was used to screen for stable cell lines, with CHO cells serving as a negative control. A eukaryotic expression vector encoding ECD fragments (SEQ ID NO: 594, SEQ ID NO: 595, and SEQ ID NO: 596) of the human CDH17 was constructed, and the vector was used to transiently transfect HEK293 cells for expression and purification of immunogen, as well as for screening of proteins. A schematic diagram of a structure relationship between the human CDH17 and the ECD fragment of the human CDH17 (i.e., human CDH17 ECD) was shown in Fig. 2. The human CDH17 ECD included 7 domains D1-D7 (also referred to as EC1-EC7), with the CDH17 ECD structure shown in Fig. 1.

An amino acid sequence of the human CDH17 was shown in SEQ ID NO: 591 (only the portion marked exclusively by the underline represented an AAL sequence; only the portion marked exclusively in bold represented the D1-D4 domains of the human CDH17; the bold and underlined portion represented the D5-D7 domains of the human CDH17; only the portion marked exclusively in italic represented a transmembrane region of the human CDH17, and italicized and underlined portions represented an intracellular region of the human CDH17).

MILQAHLHSLCLLMLYLATGYGQEGKFSGPLKPMTFSIYEGQEPSQIIFQFKANPPAVTFELTG
ETDNIFVIEREGLLYYNRALDRETRSTHNLQVAALDANGIIVEGPVPITIKVKDINDNRPTFLQSKYE
GSVRQNSRPGKPFLYVNATDLDDPATPNGQLYYQIVIQLPMINNVMYFQINNKTGAISLTREGSQE
LNPAKNPSYNLVISVKDMGGQSENSFSDTTSVDIIVTENIWKAPKPVEMVENSTDPHPIKITQVRW
NDPGAQYSLVDKEKLPRFPFSIDQEGDIYVTQPLDREEKDAYVFYAVAKDEYGKPLSYPLEIHVKV
KDINDNPPTCPSPVTVFEVQENERLGNSIGTLTAHDRDEENTANSFLNYRIVEQTPKLPMDGLFLI
QTYAGMLQLAKQSLKKQDTPQYNLTIEVSDKDFKTLCFVQINVIDINDQIPIFEKSDYGNLTLAEDT
NIGSTILTIQATDADEPFTGSSKILYHIIKGDSEGRLGVDTDPHTNTGYVIIKKPLDFETAAVSNIVFK
AENPEPLVFGVKYNASSFAKFTLIVTDVNEAPQFSQHVFQAKVSEDVAIGTKVGNVTAKDPEGLD
ISYSLRGDTRGWLKIDHVTGEIFSVAPLDREAGSPYRVQVVATEVGGSSLSSVSEFHLILMDVNDN
PPRLAKDYTGLFFCHPLSAPGSLIFEATDDDQHLFRGPHFTFSLGSGSLQNDWEVSKINGTHAR
LSTRHTEFEEREYVVLIRINDGGRPPLEGIVSLPVTFCSCVEGSCFRPAGHQTGIPTVGMAVGILLT
TLLVIGIILAVVFIRIKKDKGKDNVESAQASEVKPLRS(SEQ ID NO:591).

The mFc tag human CDH17 ECD had an amino acid sequence shown in SEQ ID NO: 594 (only the portion marked exclusively by the underline represented the AAL sequence, only the portion marked exclusively in bold represented the human CDH17 ECD, and only the portion marked exclusively by the underline represented the mFc tag).

MILQAHLHSLCLLMLYLATGYGQEGKFSGPLKPMTFSIYEGQEPSQIIFQFKANPPAVTFELTG
ETDNIFVIEREGLLYYNRALDRETRSTHNLQVAALDANGIIVEGPVPITIKVKDINDNRPTFLQSKYE
GSVRQNSRPGKPFLYVNATDLDDPATPNGQLYYQIVIQLPMINNVMYFQINNKTGAISLTREGSQE
LNPAKNPSYNLVISVKDMGGQSENSFSDTTSVDIIVTENIWKAPKPVEMVENSTDPHPIKITQVRW
NDPGAQYSLVDKEKLPRFPFSIDQEGDIYVTQPLDREEKDAYVFYAVAKDEYGKPLSYPLEIHVKV
KDINDNPPTCPSPVTVFEVQENERLGNSIGTLTAHDRDEENTANSFLNYRIVEQTPKLPMDGLFLI
QTYAGMLQLAKQSLKKQDTPQYNLTIEVSDKDFKTLCFVQINVIDINDQIPIFEKSDYGNLTLAEDT
NIGSTILTIQATDADEPFTGSSKILYHIIKGDSEGRLGVDTDPHTNTGYVIIKKPLDFETAAVSNIVFK
AENPEPLVFGVKYNASSFAKFTLIVTDVNEAPQFSQHVFQAKVSEDVAIGTKVGNVTAKDPEGLD
ISYSLRGDTRGWLKIDHVTGEIFSVAPLDREAGSPYRVQVVATEVGGSSLSSVSEFHLILMDVNDN
PPRLAKDYTGLFFCHPLSAPGSLIFEATDDDQHLFRGPHFTFSLGSGSLQNDWEVSKINGTHAR
LSTRHTEFEEREYVVLIRINDGGRPPLEGIVSLPVTFCSCVEGSCFRPAGHQTGIPTVGMVPRDSG
CKPCICTVPEVSSVFIFPPKPKDVLTITLTPKVTCVVVDISKDDPEVQFSWFVDDVEVHTAQTKPRE
EQFNSTFRSVSELPIMHQDWLNGKEFKCRVNSAAFPAPIEKTISKTKGRPKAPQVYTIPPPKEQMA
KDKVSLTCMITNFFPEDITVEWQWNGQPAENYKNTQPIMDTDGSYFVYSKLNVQKSNWEAGNTFT
CSVLHEGLHNHHTEKSLSHSPGK(SEQ ID NO:594).

The His tag human CDH17 ECD had an amino acid sequence shown in SEQ ID NO: 595 (only the portion marked exclusively by the underline represented the AAL sequence, only the portion marked exclusively in bold represented the human CDH17 ECD, and only the portion marked exclusively by the underline represented the His tag).

MILQAHLHSLCLLMLYLATGYGQEGKFSGPLKPMTFSIYEGQEPSQIIFQFKANPPAVTFELTG
ETDNIFVIEREGLLYYNRALDRETRSTHNLQVAALDANGIIVEGPVPITIKVKDINDNRPTFLQSKYE
GSVRQNSRPGKPFLYVNATDLDDPATPNGQLYYQIVIQLPMINNVMYFQINNKTGAISLTREGSQE
LNPAKNPSYNLVISVKDMGGQSENSFSDTTSVDIIVTENIWKAPKPVEMVENSTDPHPIKITQVRW
NDPGAQYSLVDKEKLPRFPFSIDQEGDIYVTQPLDREEKDAYVFYAVAKDEYGKPLSYPLEIHVKV
KDINDNPPTCPSPVTVFEVQENERLGNSIGTLTAHDRDEENTANSFLNYRIVEQTPKLPMDGLFLI
QTYAGMLQLAKQSLKKQDTPQYNLTIEVSDKDFKTLCFVQINVIDINDQIPIFEKSDYGNLTLAEDT
NIGSTILTIQATDADEPFTGSSKILYHIIKGDSEGRLGVDTDPHTNTGYVIIKKPLDFETAAVSNIVFK
AENPEPLVFGVKYNASSFAKFTLIVTDVNEAPQFSQHVFQAKVSEDVAIGTKVGNVTAKDPEGLD
ISYSLRGDTRGWLKIDHVTGEIFSVAPLDREAGSPYRVQVVATEVGGSSLSSVSEFHLILMDVNDN
PPRLAKDYTGLFFCHPLSAPGSLIFEATDDDQHLFRGPHFTFSLGSGSLQNDWEVSKINGTHAR
LSTRHTEFEEREYVVLIRINDGGRPPLEGIVSLPVTFCSCVEGSCFRPAGHQTGIPTVGMHHHHH
H(SEQ ID NO:595).

The His tag human CDH17 (D5-D7) had an amino acid sequence shown in SEQ ID NO: 596 (only the portion marked exclusively in bold represented the human CDH17 D5-D7 domain, and only the portion marked exclusively by the underline represented the His tag).

EKSDYGNLTLAEDTNIGSTILTIQATDADEPFTGSSKILYHIIKGDSEGRLGVDTDPHTNTGYVII
KKPLDFETAAVSNIVFKAENPEPLVFGVKYNASSFAKFTLIVTDVNEAPQFSQHVFQAKVSEDVAI
GTKVGNVTAKDPEGLDISYSLRGDTRGWLKIDHVTGEIFSVAPLDREAGSPYRVQVVATEVGGSS
LSSVSEFHLILMDVNDNPPRLAKDYTGLFFCHPLSAPGSLIFEATDDDQHLFRGPHFTFSLGSGS
LQNDWEVSKINGTHARLSTRHTEFEEREYVVLIRINDGGRPPLEGIVSLPVTFCSCVEGSCFRPA
GHHHHHH(SEQ ID NO:596).

### 2. Immunity

Mice were immunized according to the following immunization strategy (Table 21).

Immunized animals were BALB/c mice, female, 5- to 8-week-old. An immunization frequency was once a week. The initial immunization was administered via subcutaneous injection in the dorsal and ventral regions, with subsequent immunizations delivered via intraperitoneal injection. The immunogen was R2426 (an amino acid sequence shown in SEQ ID NO: 594), and an immunization strategy was shown in Table 21.

**Table 21 Immunization solution for producing monoclonal antibody**

| Strategy | Immunization, dosage, and number of doses | | | Use of adjuvants | | Immune group |
|---|---|---|---|---|---|---|
| | Number of immunogens | Immune preparation | | Adjuvant 1 | Adjuvant 2 | |
| | | Immunogen | Dosage | | | |
| Strategy 1 | Single immunogen | R2426 | 50 µg/mouse/time | Titermax Gold&DNA (first dose only) | Alum&DNA (except for the first dose) | G1 |
| Strategy 2 | Single immunogen | R2426 | 50 µg/mouse/time | Re5959(L6895)&DNA | N/A | G2 |
| Strategy 3 | Single immunogen | R2426 | 50 µg/mouse/time | MPLA&DNA | N/A | G3 |

### 3. Production of hybridoma for producing anti-CDH17 mouse monoclonal antibody

A booster immunization was performed on fusion target mice, and sufficient quantities of sp2/mIL6 myeloma cells were resuscitated and expanded in advance to ensure the number and viability of the cells. 12 mice were euthanized 3 days in advance, and abdominal cavity suspension was taken out and spread on a 96-well plate to serve as a culture fusion plate for later use, with 150 µL per well. Spleen cells from the mice were isolated using a standard protocol and mixed 1:1 with mouse myeloma cells at concentrations of 5×10⁶ cells/plate, 3.1×10⁶ cells/plate, and 4.4×10⁶ cells/plate. Fusion was performed under the action of an electric fusion device, then the mixture was added to the culture fusion plate in a 50µL volume and placed in an incubator (Panasonic MCO-18AIC) under conditions of 37 °C and 5% CO₂. Six days after electrofusion, the old medium was sucked from the fusion plate, and each well was replenished with 200 µL of a fresh medium. On Day 7, the supernatant was collected for an hCDH17-his (SEQ ID NO: 595) ELISA binding assay, and based on the experimental results, positive target wells were determined, sterile fluid exchange was performed, and a liquid-replenishing volume was 200 µL. On Day 8 after electroporation, 100 µL of the culture supernatant was collected for screening via the hCDH17(D5-D7)-his(SEQ ID NO: 596) ELISA binding assay. On Day 9 after electroporation, the culture supernatant was collected for AsPC-1 flow cytometry binding assays. On Day 12 after electroporation, the culture supernatant was collected for CHO-hCDH17 flow cytometry binding screening. Positive pools were selected from the comprehensive screening results for subcloning. Then, at least one clone was selected from the in-vitro cultured hybridoma that retained parent cell reactivity (as determined by FACS), and 9-20 tubes were produced for each clone and stored in liquid nitrogen.

### 4. Selection of monoclonal antibody binding to CDH17

An isotype ELISA test was performed to determine an isotype of an antibody. An ELISA kit for identifying Ig classes/subclasses in mouse monoclonal antibodies (IgG1\IgG2a\IgG2b\IgG3\IgM\IgA) (Biodragon, Cat#BF06001) was used to determine the Ig subclass of an identified CDH17-reactive monoclonal antibody. The antibody isotype results are shown in Table 22, the amino acid sequences of the variable regions were shown in Table 23, and the CDR regions of the antibodies were shown in Tables 1-20.

**Table 22 Mouse monoclonal antibody subtype**

| | | VH Sequence ID | VL Sequence ID |
|---|---|---|---|
| F1-41.16 | mIgG1 | SEQ ID NO:1 | SEQ ID NO:2 |
| F1-87.15 | mIgG2b | SEQ ID NO:3 | SEQ ID NO:4 |
| F1-111.5 | mIgG1 | SEQ ID NO:5 | SEQ ID NO:6 |
| F1-143.7 | mIgG2a | SEQ ID NO:7 | SEQ ID NO:8 |
| F2-55.10 | mIgG2b | SEQ ID NO:9 | SEQ ID NO:10 |
| F2-155.7 | mIgG2a | SEQ ID NO:11 | SEQ ID NO:12 |
| F2-186.1 | mIgG2a | SEQ ID NO:13 | SEQ ID NO:14 |
| F2-238.14 | mIgG2b | SEQ ID NO:15 | SEQ ID NO:16 |
| F2-324.7 | mIgG2a | SEQ ID NO:17 | SEQ ID NO:18 |
| F3-1.5 | mIgG2a | SEQ ID NO:19 | SEQ ID NO:20 |
| F1-76.12 | mIgG2b | SEQ ID NO:21 | SEQ ID NO:22 |
| F1-86.9 | mIgG2b | SEQ ID NO:23 | SEQ ID NO:24 |
| F1-99.29 | mIgG2b | SEQ ID NO:25 | SEQ ID NO:26 |
| F1-124.38 | mIgG2a | SEQ ID NO:27 | SEQ ID NO:28 |
| F1-127.8 | mIgG2a | SEQ ID NO:29 | SEQ ID NO:30 |
| F2-71.18 | mIgG2a | SEQ ID NO:31 | SEQ ID NO:32 |
| F2-89.7 | mIgG2a | SEQ ID NO:33 | SEQ ID NO:34 |
| F2-291.2 | mIgG2a | SEQ ID NO:35 | SEQ ID NO:36 |
| F3-6.18 | mIgG2a | SEQ ID NO:37 | SEQ ID NO:38 |
| F3-36.14 | mIgG1 | SEQ ID NO:39 | SEQ ID NO:40 |

The amino acid sequence of the variable region was shown in the table below.

**Table 23 Amino acid sequence of variable region**

| | |
|---|---|
| SEQ ID NO:1 | QVQLQQSGAELVKPGASVKLSCKASGYTFTSYHINWVRQRPE QGLEWIGWIFPGDGSTEYNEKFKDKATLTTDRSSSTAYMQLSR LTSDDSAVYFCTRAGGYNVFTYWGQGTLVAVSA |
| SEQ ID NO:2 | SIVMTQTPKFLLVSAGDRVTITCKASQTVSNDVAWYQQKPGQS PKLLIYYASNRYTGVPDRFTGSGYGTDFTFTISTVQAEDLAVYF CQQDYSSPYTFGGGTKLEIK |
| SEQ ID NO:3 | EVQLQQSGPELVKPGTSVKISCKTSGYTFTEYTMHWVKQSHGK SLDWIGGINPKNGDSTYNQKFKGKATLTVDKSSTTAYMELRSLT SEDSAVYYCARSHLSYGGKTWFAYWGQGTLVTVSA |
| SEQ ID NO:4 | DIVMSQSPSSLAVSVGEKVTMSCKSSQSLLYSSNQKNYLAWYQ QKPGQSPKLLIYWASTRESGVPDRFTGSGSGTDFTLTISSVKAE DLAVYYCHQYYSYPPWTFGGGTKLEIK |
| SEQ ID NO:5 | EVMLVESGGGLVKPGGSLKLSCAASGFTFSSYAMSWGRQTPE KRLEWVASISSGDGYTYYPDSVKGRFTISRDNAKNTLYLQMSSL RSEDTAMYFCARAGNFAYWGQGTLVTVSA |
| SEQ ID NO:6 | DIKMTQSPSTMHASLGERVTITCKASQDINNFLSWFQQRPGKS PKTLIYRTNRLVDGVPSRFSGSGSGQDYSLTISSLEYEDMGIYY CLKYDEFPLTFGAGTKLELK |
| SEQ ID NO:7 | QVQLQQSGAELVKPGASVKLSCKASGYSFTSYYMSWVKQRPG QGLEWIGEINPSHGGTIFNENFKNKATLAVDRSSSTAYMQLNSL TSEDSAVYYCIRTLRLRPPDYWGQGTTLTVSS |
| SEQ ID NO:8 | DIVLTQSPAIMSASPWEKVTMTCSASSSVSYMHWYQQKPGSS PKPLIYDTSNLASGFPARFSGSGSGTFSLKISSMEAEDAATYYC HQRSSYPWTFGGGTKLEIK |
| SEQ ID NO:9 | EVQLQQSGPELVKPGTSMKMSCKASGYSFTGYAMNWVKQSH GKNLEWIGLINPYNGATTYNQKFRGKATLTVDMSSTTAYMELLS LTSDDSAVYYCARWGNYPGYVMDYWGQGTSVTVSS |
| SEQ ID NO:10 | DIVLTQSPAIMSASLGERVTMTCTASSSVSSNYLHWFQQKPGS SPKLWIYSTSNLASGVPARFSGSGSGTSYSLTISSMEAEDAATY YCHQYHRSPYTFGGGTKLEIK |
| SEQ ID NO:11 | QVQLKQSGAELVRPGASVRLSCKTSGYTLTSHWIHWIKQRSGQ GLEWIARIYPGTGYTYYNENFRGKATLTADKSSNTAYMQLSSLK SEDSAVYFCARSYDAYFVGYFDYWGQGTTLTVSS |
| SEQ ID NO:12 | DIVLTQSPASLAVSLGQRATISCRASESVDSYGNSIMHWYQQKP GQPPKLLIYRASNLESGIPARFSGSGSRTDFTLIINPVEADDVAT YYCQQSNEDPYTFGGGTKLEIR |
| SEQ ID NO:13 | EVQLVESGGGLVKPGGSLKLSCVASGFTFSTYAMSWVRQTPE KRLEWVATINNGVSYTYYPDSVKGRFTISRDNAKNTLYLQMSSL RSEDTAMYYCARQGYDYAYFDHWGQGTTLTVSS |
| SEQ ID NO:14 | QVVLTQSPAIMSASLGERVTMTCTASSSVSSNYLHWFQQKPGS SPKLWIYSTSNLASGVPARFSGSGSGTSYSLTISSMEAEDAATY YCHQYHRSPYTFGGGTKLEIK |
| SEQ ID NO:15 | EVQLQQSGPELVKPGASMKISCKASGYSFTGYTVNWVKQSHG KNLEWIGLINPNNGGVNYNQKFKDKASLTVDKSSRTAYMELLSL TSEDSAIYFCARSLITTLVVHNYWYFDVWGAGTTVTVSS |
| SEQ ID NO:16 | DIVLTQSPASLPVSLGQRATVSCRASQSVSSSTYGYVHWYQQK PGQPPKLLIKYASTLESGVPARFSGSGSGTDFTLNIHPVEEEDIA TYYCQHSWEIPYTFGGGTKLEIK |
| SEQ ID NO:17 | QLQESGPSLVKPSQTLSLTCSVTGDAITSGYWNWIRQFPGNKL EYMGYITFSGDTYYSPSLKSRISITRDTSRNQFYLRLNSVTSEDT ATYYCARRDYGNYWFAYWGQGTLVTVSA |
| SEQ ID NO:18 | DIVMTQSHKFMSTSVGDRVNITCKASQDVSSAVAWYQQRPGQ SPKLLIYSASFRYTGVPDRFTGSGSGTDFTFTISSVQAADLAVYY CQQHYSGPFTFGSGTKLEIK |
| SEQ ID NO:19 | QVTLKESGPGILQPSQTLSLTCSFSGFSLSTSGMGVSWIRQPS GKGLEWLAHIFWDDDKRYNPSLKSRLTISKDTSRNQVFLKITSV DTADTATYYCARRQWAYQFDSWGQGTTLTVSS |
| SEQ ID NO:20 | DVVVTQTPLSLPVSLGDQASISCRSSQSILHSNGNTYLEWYLQK PGQSPNLLIYKVSNRFSGVPDRFSGSGSGTDFTLKISRVEAEDL GIYYCFQGSHVPPAFGGGTKLEIK |
| SEQ ID NO:21 | EVQLQQSGPELVKPGASVKISCKTSGYTFTEYTMHWVKQSHG KSLDWIGGINPNNGDTTYNQKFKGKATLTVDKSSTTVYMEFRSL TSEDSAVYYCARSHLSYGGKTWFAYWGQGTLVTVSA |
| SEQ ID NO:22 | DIVMSQSPSSLAVSVGEKVTMSCKSSQSLLYSSNQKNYLAWYQ QKPGQSPKLLIYWASTRESGVPDRFTGSGSGTDFTLTISSVKAE DLAVYYCHQYYSYPPWTFGGGTKLEIK |
| SEQ ID NO:23 | QVQLHQPGAELVRPGASVKLSCKASGYSFTSYWMNWVKQRP GQGLEWIGMIRPSDMETRLNQKFRDKATLTVDKSSSTAYMQLS SPTSEDSAVYYCAFGTGSWFAYWGQGTLVTVSA |
| SEQ ID NO:24 | ENVVTQSPAIMSASPGEKVTMTCSASSSVSYMHWYQQKSSTS PKLWIYDTSKLASGVPGRFSGSGSGNSYSLTISSMEAGDVATY YCFQGSGYPLTFGSGTKLEIK |
| SEQ ID NO:25 | EVQLQQSGPELVRPGASVKISCKTSGYTFTEYTMHWVRQSHG KGLDWIGGINPNNGDTTYNRKFKGKATLTVDKSSTTAYMELRSL TSEDSAVYSCARSHLSYGSKTWFAYWGQGTLVTVSA |
| SEQ ID NO:26 | DIVMSQSPSSLPVSVGEKVTMSCKSSQSLLYSSNQKNYLAWYQ QKPGQSPKLLIYWASTRESGVPDRFTGSGSGTDFTLTISSVKAE DLAVYYCHQYYSYPPWTFGGGTKLEIK |
| SEQ ID NO:27 | EVQLQQSGPDLVKPGASVKISCKASGYSFTDYYLHWVRQSHG KSLEWIGRINPKNGGTTYNQKFKGKAILTVGKSSSTAYMELRSL TSEDSAVYYCAKEYDNYDAMDYWGQGTSVTVSS |
| SEQ ID NO:28 | DIQMTQSSSYLSVSLGGRVTITCKASDHINNWLAWYQQKPGNA PRLLISGATSLETGVPSRFSGCGSGRDYTLSITSLQTEDVATYY CQWYWGNPPFRVGTKLELK |
| SEQ ID NO:29 | QVQLQQPGAELVRPGASVKLSCKASGYTFTTYWINWVKQRPG QGLEWIGNISPSGIFINYNQKFKDKATLTVDKSSSTAYMQLSSPT SEDSAVYYCTRSGGNYAMDYWGQGTSVTVSS |
| SEQ ID NO:30 | ENVLTQSPAIMSASLGEKVTMSCRASSSVNYMYWYQQKSDAS PKIWIYYTSNLAPGVPARFSGSGSGNSYSLTINSMEGEDAATYY CQQFTSSPFTFGSGTKLEIK |
| SEQ ID NO:31 | QIQLVQSGPELKKPGETVKISCKASGYTFTNYGMNWVKQAPGK GLKWLGWINTYTGAPTYADDFKGRFAFSLETSDSTAYLQISNLK NEDTATYFCARDHDGGFVYWGQGTLVTVSA |
| SEQ ID NO:32 | DIVMTQSPATLSVTPGDRVSLSCRASQSISDFLHWYHQKSHES PRLLIKYASQSISGIPSRFSGSGSGSDFTLSINSVEPEDVGVYYC QNGHSFPRTFGGGTKLEIK |
| SEQ ID NO:33 | EVKLVESGGGLLKPGGSLKLSCAASGFTFSTYAMSWVRQTPEK RLEWVASISSGGSTYYPDSVRGRFTISRDNARNILHLQMSSLRS DDTAMYYCTRGSFFTTVITDFDYWGQGATLTVSS |
| SEQ ID NO:34 | DIQMTQSPSSLSASLGERVSLTCRASQEISGYLSWLQQKPDGTI KRLIYAASTLDSGVPKRFSGSRSGSDYSLTISSLESEDFADYYCL QYAIYPWTFGGGTKLEIK |
| SEQ ID NO:35 | EVKLVESGGGLLKPGGSLKLSCAASGFTFSTYAMSWVRQTPEK RLDWVASISSGGSTYYPDSVRGRFTISRDNARDILYLQMSSLRS DDTAMYYCTRGSFFTTVVTDFDYWGQGTTLTVSS |
| SEQ ID NO:36 | DIQMTQSPSSLSASLGERVSLTCRASQEISGYLSWLQQKPDGTI KRLIYAASTLDSGVPKRFSGSRSGSDYSLTISSLESEDFADYYCL QYAIYPWTFGGGTKLEIK |
| SEQ ID NO:37 | QVQLQQPGAERVKPGASVKLSCKASGYTFSNYWMHWVKQRP GQGLEWIGEIDPSDSYTNYNQNFKGKATLTVDKSSSTAYMQLS SLTSEDSAVYYCARRGDDGVLDYWGQGTSVTVSS |
| SEQ ID NO:38 | DIVLTQSPASLAVSLGQRATISCRASQSVSTSGYSYMHWYQQK PGQPPKLLIKFASNLESGVPVRFSGSGSGTDFTLNIHPVEEEDT ATYYCQHSWEIPYTFGGGTKLEIK |
| SEQ ID NO:39 | EVQLQQSGPELVKPGASVKISCKASGYSFTDYFMNWMKQSHG KSLEWIGRIHPNSGETFYNRQFKGKATLTVDKSSSTAHMELLSL TSEDSAVYYCGRRRGYGNYFFDYWGQGTTLTVSS |
| SEQ ID NO:40 | DIVMTQSQKFMSTSVGDRVSITCKASQDVNIDVAWYQQKPGQS PKLLIYSASYRYTGVPDRFTGSGSGTDFTFTISSVQAEDLAVYC CQQHYGTPWTFGGGTKLEIK |
| SEQ ID NO:592 | QVQLVQSGAEVKKPGSSVKVSCKVSGYTFTDHTIHWMKQRPG QGLEWIGYIYPRDVITQYNEKFKGKVTLTADKSTSTAYMELSSL RSEDTAVYLCARWGYFYGSRSYYFDYWGQGTTVTVSSAKTTA PSVYPLAPVCGDTTGSSVTLGCLVKGYFPEPVTLTWNSGSLSS GVHTFPAVLQSDLYTLSSSVTVTSSTWPSQSITCNVAHPASSTK VDKKIEPRGPTIKPCPPCKCPAPNLLGGPSVFIFPPKIKDVLMISL SPIVTCVVVDVSEDDPDVQISWFVNNVEVHTAQTQTHREDYNS TLRVVSALPIQHQDWMSGKEFKCKVNNKDLPAPIERTISKPKGS VRAPQVYVLPPPEEEMTKKQVTLTCMVTDFMPEDIYVEWTNNG KTELNYKNTEPVLDSDGSYFMYSKLRVEKKNWVERNSYSCSVV HEGLHNHHTTKSFSRTPGK |
| SEQ ID NO:593 | DIQMTQSQSSLSASVGDRVGITCKASQNVGTAVAWYQQKPGK APKLLIYSASNRYTGVPSRFSGSGSGTDFTLTISSLQPKDFATYF CQQYSSYPWTFGQGTKLEIKRADAAPTVSIFPPSSEQLTSGGA SVVCFLNNFYPKDINVKWKIDGSERQNGVLNSWTDQDSKDSTY SMSSTLTLTKDEYERHNSYTCEATHKTSTSPIVKSFNRNEC |
| SEQ ID NO:598 | QVQLQESGGGLVQPGGSTRLSCVSSRTFSYYDMGWFRQAPG KEREFVALLSWNGENAEYSDSVMGRFTVSRGNTQNSVNLQMN NLKPEDTGIYYCAVTHGGARSVRSWGQGTQVTVSSEPRTGPTI KPCPPCKCPAPNLLGGPSVFIFPPKIKDVLMISLSPIVTCVVVDVS EDDPDVQISWFVNNVEVHTAQTQTHREDYNSTLRVVSALPIQH QDWMSGKEFKCKVNNKDLPAPIERTISKPKGSVRAPQVYVLPP PEEEMTKKQVTLTCMVTDFMPEDIYVEWTNNGKTELNYKNTEP VLDSDGSYFMYSKLRVEKKNWVERNSYSCSVVHEGLHNHHTT KSFSRTPGK |

### 5. Production of monoclonal antibody

Hybridoma cells were inoculated at 2×10⁶ cells/mL into a dialysis-based bioreactor, with antibody-containing supernatant harvested weekly. The mouse monoclonal antibodies were purified with Protein A (GE-MabSelectSuRe LX, Cat#17-5438-03) via FPLC. An antibody concentration was determined with a BCA kit or an A280 absorbance value. Antibody purity was determined via Size Exclusion Chromatography (SEC) and verified through Sodium Dodecyl Sulfate (SDS) gel electrophoresis and coomassie blue staining.

### Embodiment 2 Binding characteristic of monoclonal antibody for CDH17

CHO-CDH17 constructing and overexpressing the human CDH17 served as positive cells, while AsPC-1 (human metastatic pancreatic adenocarcinoma cells) served as positive cells endogenously expressing the CDH17. A flow cytometry analysis method was used to detect the binding activity of a monoclonal antibody to the CHO-CDH17 cells and AsPC-1 cells.

### 1. CHO-CDH17 cell binding

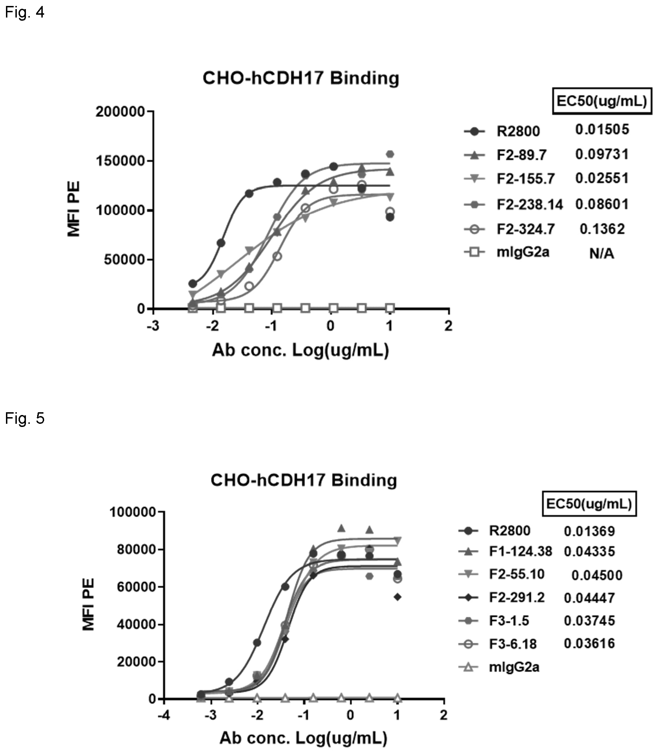

Sufficient CHO-CDH17 cells were cultured, centrifuged, and collected. The monoclonal antibody and corresponding positive control antibodies were diluted simultaneously with PBS+3%FBS, starting at a concentration of 10 µg/mL, with a 3-fold or 4-fold gradient dilution, so as to obtain 8 concentration gradients for later use. The collected cells were washed once with the PBS+3%FBS, then the PBS+3%FBS was added to resuspend the cells to 2×10⁶ cells/mL. The cells were spread on a 96-well plate with 100 µL per well (2×10⁵ cells), and 100 µL of the diluted antibodies was added and incubated at 4 °C for 30 minutes. Centrifugation was performed; the supernatant was discarded. The cells were washed twice with PBS, resuspended with a diluted PE-labeled anti-mouse IgG FC antibody (Biolegend, 405307), and incubated at 4 °C in the dark for 30 minutes. The cells were washed twice with the PBS and resuspended with 100 µL of the PBS. Loading was performed for detection. Then, a binding affinity EC₅₀ value of the antibodies and the CHO-CDH17 was analyzed and calculated with software GraphPad Prism 7.0 based on average fluorescence intensity. The results were shown in Figs. 3, 4, and 5, and indicated that most monoclonal antibodies had favorable binding to the CHO-hCDH17. The R2800 served as a positive control antibody, with a heavy chain amino acid sequence shown as SEQ ID NO: 592 and a light chain amino acid sequence shown as SEQ ID NO: 593 (as shown in Table 23). mIgG2a served as a negative control antibody.

### 2. AsPC-1 cell binding

The flow cytometry analysis method was used to detect the binding activity of the monoclonal antibodies to the AsPC-1 cells. The cells were digested with a Versene solution (Gibco, 15040066), centrifuged, and collected for later use. The antibodies were diluted in a 3-fold or 4-fold gradient starting from 10 µg/mL or 20 µg/mL, so as to obtain 8 concentration gradients. After the antibodies and the cells were co-incubated, subsequent experimental procedures were identical to the CHO-CDH17 cell binding experiment. The cells were resuspended with 100 µL of the PBS. Loading was performed for detection. Then, a binding affinity EC₅₀ value of the antibodies and the AsPC-1 cells was analyzed and calculated with software GraphPadPrism 7.0 based on average fluorescence intensity.

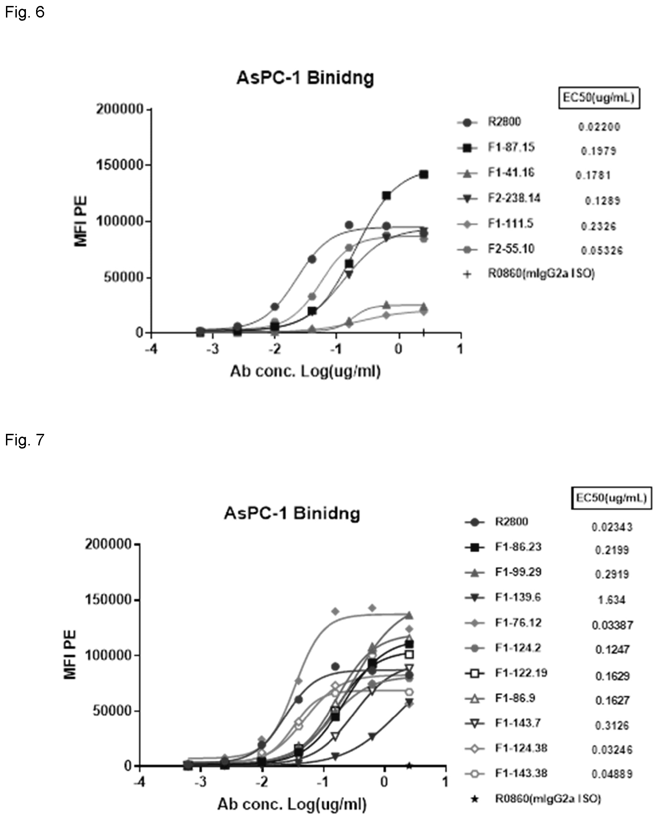

The results were shown in Figs. 6, 7, and 8, and indicated that all 20 mouse monoclonal antibodies had good binding to AsPC-1.

### Embodiment 3 Detection of binding activity of monoclonal antibody and monkey CDH17

In this embodiment, the binding activity of the monoclonal antibody to monkey CDH17 (Acrobiosystem) was detected. A Cyno.CDH17 recombinant protein (monkey CDH17) was diluted to 0.5 µg/mL in advance with a 50 mmol Coating Buffer, added 100 µL to each well, and placed overnight at 4 °C. The supernatant was discarded. The plate was washed for three times with 1×PBST buffer. Then, the PBS+1%FBS was added with 100 µL/well and placed and blocked at 37 °C for 1 hour. Meanwhile, the monoclonal antibody was diluted with the PBS+1%FBS to match the positive control R2800. Starting at a concentration of 10 µg/mL, a 4-fold gradient dilution was performed to obtain 8 concentration gradients for later use. After blocking, the supernatant was discarded. 100 µL of the diluted antibody was added and incubated at 37 °C for 1 hour. After incubation, the plate was washed for four times with the 1×PBST buffer. Then 100 µL of a diluted HRP-conjugated anti-mouse IgG C antibody (Sigma, A0168) was added and incubated at 37 °C for 30 minutes. After incubation, the plate was washed for five times with the 1×PBST buffer. An appropriate amount of a color development solution was added, and color development was terminated after color development. The absorbance was measured at 450 nm using a microplate reader, a binding affinity EC₅₀ value of the antibodies and the Cyno.CDH17 recombinant protein was analyzed and calculated with software GraphPadPrism 7.0.

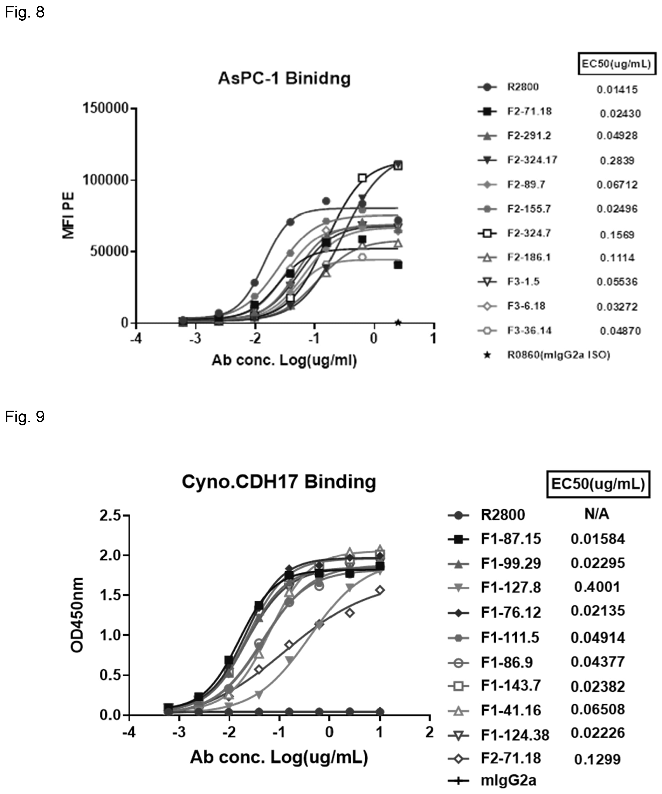

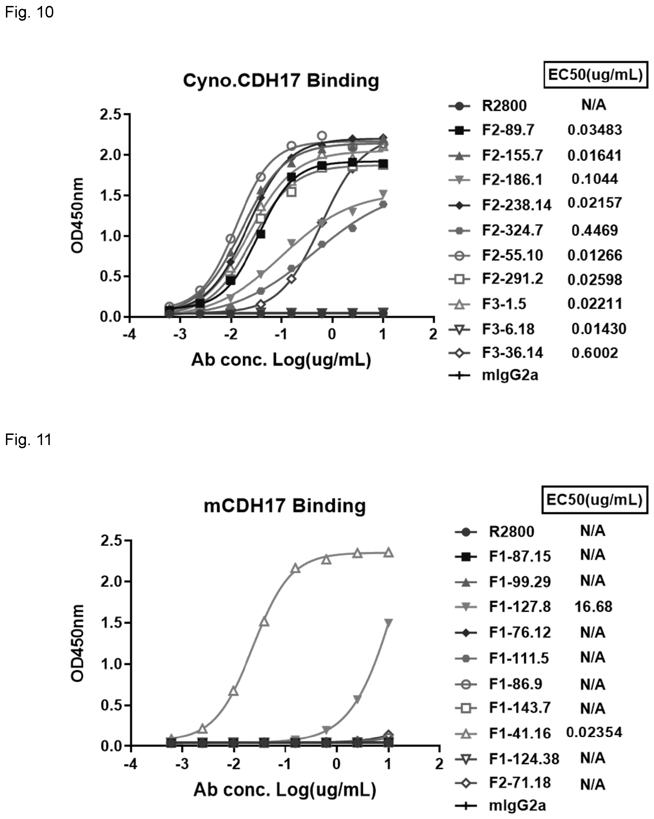

The results were shown in Figs. 9 and 10. The control antibody R2800 did not bind to the monkey CDH17. All the mouse monoclonal antibodies had good binding to the monkey CDH17 except for a few with slightly weaker binding activity.

### Embodiment 4 Detection of binding activity of monoclonal antibody and mouse CDH17

The binding activity of a monoclonal antibody and mouse CDH17 was detected. A mCDH17 recombinant protein (mouse CDH17) was diluted to 0.5 µg/mL in advance with a 50 mmol Coating Buffer, added 100 µL to each well, and placed overnight at 4 °C. The supernatant was discarded. The plate was washed for three times with 1×PBST buffer. Then, the PBS+1%FBS was added with 100 µL/well and placed and blocked at 37 °C for 1 hour. Meanwhile, the monoclonal antibody and the positive control R2800 were diluted with the PBS+1%FBS respectively. Starting at a concentration of 10 µg/mL, a 4-fold gradient dilution was performed to obtain 8 concentration gradients for later use. After blocking, the supernatant was discarded. 100 µL of the diluted antibody was added and incubated at 37 °C for 1 hour. After incubation, the plate was washed for four times with the 1×PBST buffer. Then a diluted HRP-conjugated anti-mouse IgG C antibody (Sigma, A0168) was added with 100 µL per well and incubated at 37 °C for 30 minutes. After incubation, the plate was washed for five times with the 1×PBST buffer. An appropriate amount of a color development solution was added, and color development was terminated after color development. The absorbance was measured at 450 nm using a microplate reader, a binding affinity EC₅₀ value of the antibodies and the mCDH17 recombinant protein was analyzed and calculated with software GraphPadPrism 7.0.

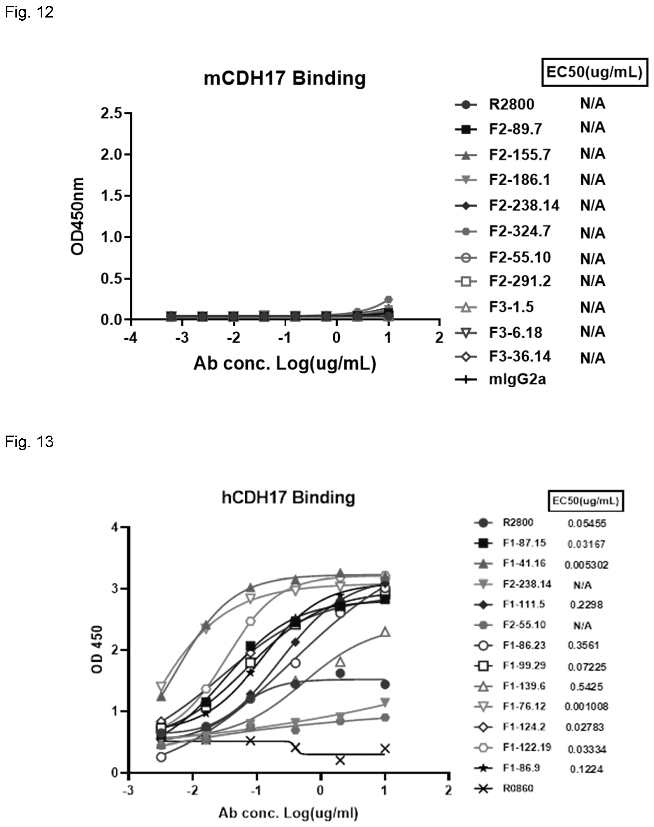

The results were shown in Figs. 11 and 12. The positive control R2800 did not bind to mCDH17. Among the remaining monoclonal antibodies, only F1-127.8 and F1-41.16 had good binding to the mCDH17, while the remaining monoclonal antibodies did not bind to the mCDH17.

### Embodiment 5 Detection of binding activity of monoclonal antibody and hCDH17

In this embodiment, the binding activity of the monoclonal antibody to hCDH17 (ACRO, CA7-H52H3) was detected. A hCDH17 recombinant protein (human CDH17) was diluted to 0.5 µg/mL in advance with a 50 mmol Coating Buffer, added 100 µL to each well, and placed overnight at 4 °C. The antibody was diluted in a 5-fold gradient at 10 µg/mL, so as to obtain 6 concentration gradients. All other procedures were identical to those in Embodiment 4. The absorbance was measured at 450 nm using a microplate reader, and a binding affinity EC₅₀ value of the antibodies and the hCDH17 recombinant protein was analyzed and calculated with software GraphPadPrism 7.0.

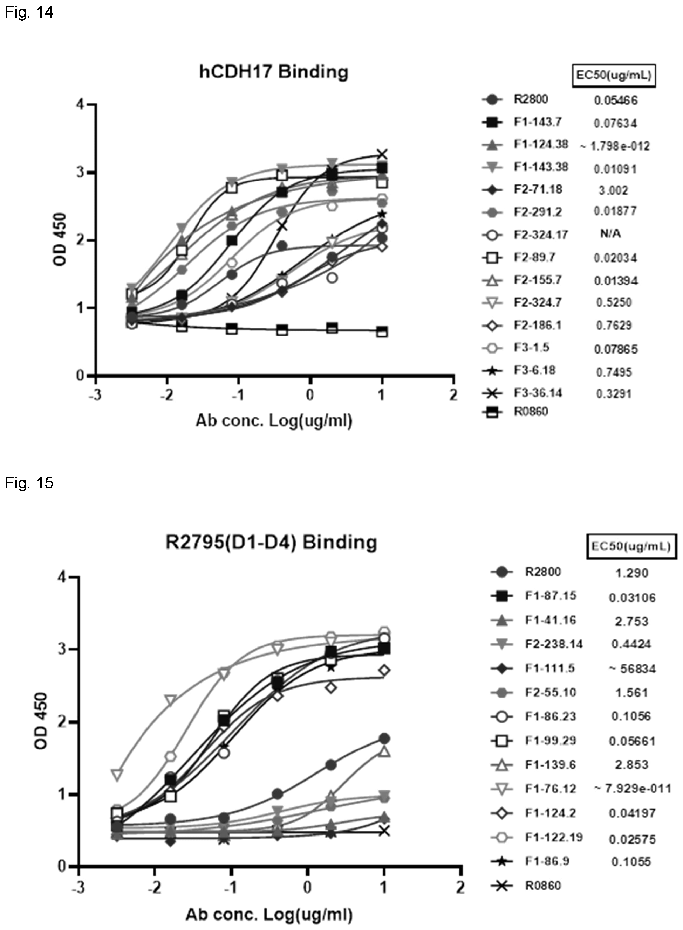

The results were shown in Figs. 13 and 14. Except for F2-238.14, F2-55.10, F1-139.6, F2-71.18, F2-324.17, F2-324.7, F2-186.1, and F3-6.18, all other antibodies had superior binding activity to full-leng**t**h CDH17 compared to the positive control antibody R2800 (either higher plateau or lower EC₅₀).

### Embodiment 6 Identification of binding epitope of monoclonal antibody

In this embodiment, a binding epitope of a monoclonal antibody was detected. R2795 (hCDH17 D1-D4.his) and R2796 (hCDH17 D5-D7.his) proteins were constructed respectively, an amino acid sequence of the R2795 was shown in SEQ ID NO: 615, and an amino acid sequence of the R2795 was shown in SEQ ID NO: 616.

Amino acid sequence of R2795 huCDH17(D1-D4)-his:

PLKPMTFSIYEGQEPSQIIFQFKANPPAVTFELTGETDNIFVIEREGLLYYNRALDRETRSTHNL
QVAALDANGIIVEGPVPITIKVKDINDNRPTFLQSKYEGSVRQNSRPGKPFLYVNATDLDDPATPNG
QLYYQIVIQLPMINNVMYFQINNKTGAISLTREGSQELNPAKNPSYNLVISVKDMGGQSENSFSDTT
SVDIIVTENIWKAPKPVEMVENSTDPHPIKITQVRWNDPGAQYSLVDKEKLPRFPFSIDQEGDIYVT
QPLDREEKDAYVFYAVAKDEYGKPLSYPLEIHVKVKDINDNPPTCPSPVTVFEVQENERLGNSIGTL
TAHDRDEENTANSFLNYRIVEQTPKLPMDGLFLIQTYAGMLQLAKQSLKKQDTPQYNLTIEVSDKDF
KTLCFVQINVIDINDQIPIFHHHHHH(SEQ ID NO:615).

Amino acid sequence of R2796 huCDH17(D5-D7)-his:

EKSDYGNLTLAEDTNIGSTILTIQATDADEPFTGSSKILYHIIKGDSEGRLGVDTDPHTNTGYVII
KKPLDFETAAVSNIVFKAENPEPLVFGVKYNASSFAKFTLIVTDVNEAPQFSQHVFQAKVSEDVAIG
TKVGNVTAKDPEGLDISYSLRGDTRGWLKIDHVTGEIFSVAPLDREAGSPYRVQVVATEVGGSSLS
SVSEFHLILMDVNDNPPRLAKDYTGLFFCHPLSAPGSLIFEATDDDQHLFRGPHFTFSLGSGSLQN
DWEVSKINGTHARLSTRHTEFEEREYVVLIRINDGGRPPLEGIVSLPVTFCSCVEGSCFRPAGHHH
HHH(SEQ ID NO:616).

The R2795 and the R2796 were diluted to 0.5 µg/mL in advance with a 50 mmol Coating Buffer, added 100 µL to each well of a plate, and placed overnight at 4 °C. The supernatant was discarded. The plate was washed for three times with 1×PBST buffer. Then, the PBS+1%FBS was added with 100 µL/well and placed and blocked at 37 °C for 1 hour. Meanwhile, the monoclonal antibody and the positive control R2800 were diluted with the PBS+1%FBS respectively. Starting at a concentration of 10 µg/mL, a 5-fold gradient dilution was performed to obtain 6 concentration gradients for later use. After blocking, the supernatant was discarded. 100 µL of the diluted antibody was added and incubated at 37 °C for 1 hour. After incubation, the plate was washed for four times with the 1×PBST buffer. Then a diluted HRP-conjugated anti-mouse IgG C antibody (Sigma, A0168) was added with 100 µL per well and incubated at 37 °C for 30 minutes. After incubation, the plate was washed for five times with the 1×PBST buffer. An appropriate amount of a color development solution was added, and color development was terminated after color development. The absorbance was measured at 450 nm using a microplate reader, a binding affinity EC₅₀ value of the antibodies and the binding epitope was analyzed and calculated with software GraphPadPrism 7.0.

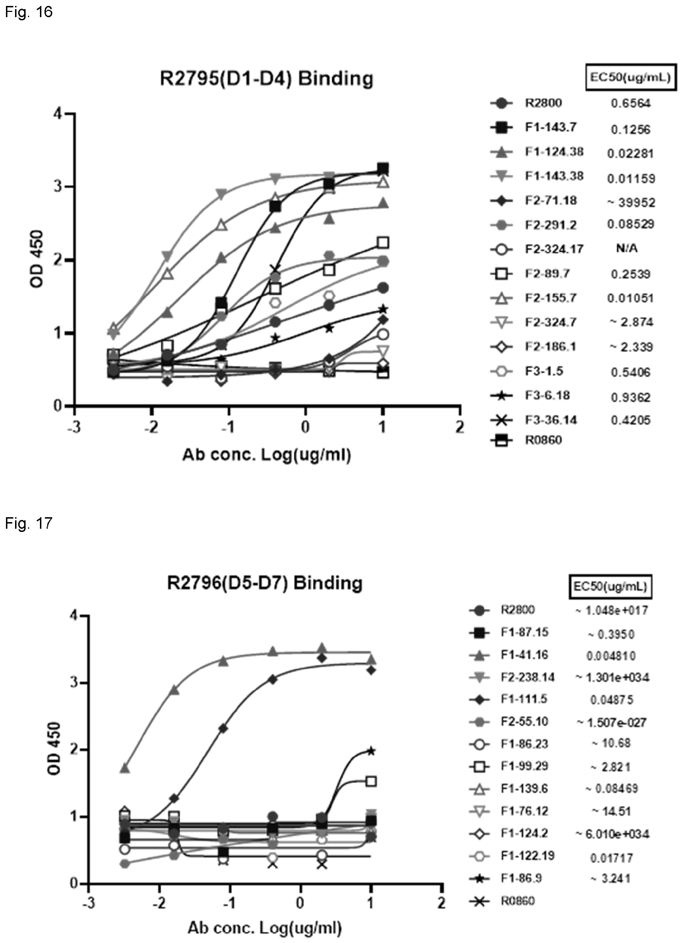

The results were shown in Figs. 15 -18. The positive control R2800 did not bind to mCDH17. Among the remaining monoclonal antibodies, only F1-127.8 and F1-41.16 had good binding to the mCDH17, while the remaining monoclonal antibodies did not bind to the mCDH17.

### Embodiment 7 Detection of killing activity of cells mediated by monoclonal antibody

A flow cytometry was used for detecting the killing activity of an overexpressed CHO-hCDH17 cell mediated by a monoclonal antibody.

Sufficient CHO-hCDH17 cells were cultured, centrifuged, collected, and resuspended after being labeled with a fluorescent dye CFSE (BD Bioscience, 565082), and cell density was adjusted to 4×10⁵ cells/mL for later use. The cultured NK92 cells were collected, centrifuged, and resuspended in a target cell (AsPC-1 cell) medium, and the cell density was adjusted to 1.2×10⁶ cells/mL. Simultaneously, the monoclonal antibody and corresponding positive control antibodies R2800 and R2803 (R2803 being a nanobody with an amino acid sequence shown in SEQ ID NO: 598) were diluted with a target cell culture medium. R0860 was a mIgG2a isotype control antibody (Isotype Control). Starting at a concentration of 20 µg/mL, a 40-fold gradient dilution was performed to obtain 3 concentration gradients for later use. The collected target cells were resuspended and plated with 50 µL (2×10⁴ cells) per well. Collected effector cells (NK92 cells) were added with 100 µL per well (1.2×10⁵ cells). Then, 50 µL of the diluted antibody was added and incubated at 37 °C for 6 hours. Centrifugation was performed, and the supernatant was discarded. The cells were resuspended with a diluted Propidium Iodide Solution (Biolegend, 421301) with 100 µL per well and incubated at room temperature in the dark for 20 minutes. After resuspension, loading was performed for detection. Then, the killing activity of the cells mediated by the antibody was analyzed and calculated by software GraphPadPrism 7.0 based on an FITC⁺PE⁺ percentage.

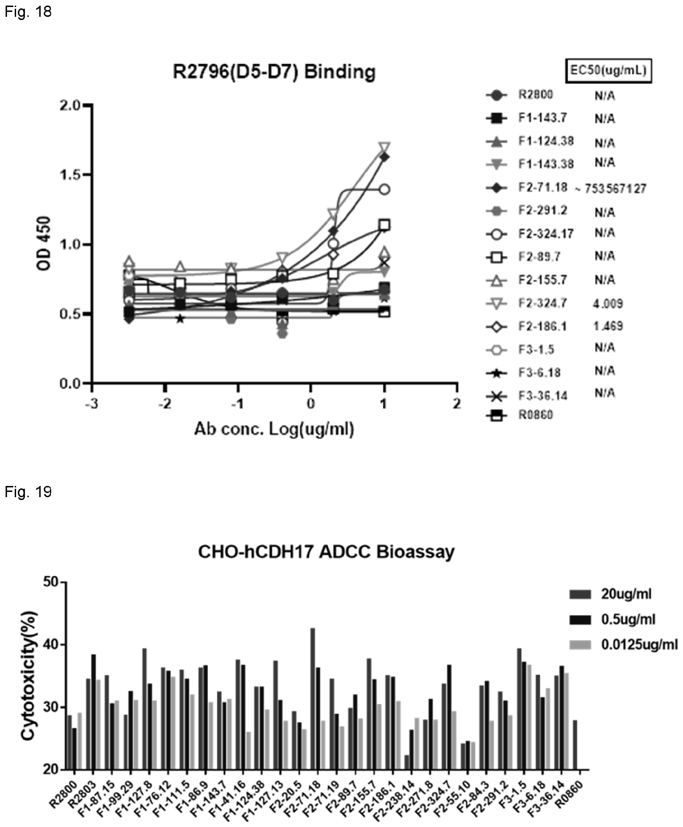

As shown in Fig. 19, no killing activity was detected in the positive antibody. Among the monoclonal antibodies, F1-87.15, F1-127.8, F1-86.9, F1-41.16, F1-127.13, F2-71.18, F2-71.19, F2-155.7, F2-186.1, F2-324.7, F2-84.3, and F2-291.2 had ADCC (antibody-dependent cell-mediated cytotoxicity) activity.

Moreover, an LDH (lactate dehydrogenase) cytotoxicity assay was used to detect the killing activity mediated by the monoclonal antibodies against AsPC-1 cells endogenously expressing human CDH17. Sufficient AsPC-1 cells were cultured, centrifuged, and collected, and the cell density was adjusted to 4×10⁵ cells/mL for later use. The cultured NK92 cells were collected, centrifuged, and resuspended in a target cell medium, and the cell density was adjusted to 1.2×10⁶ cells/mL. Simultaneously, the monoclonal antibody and corresponding positive control antibodies R2800 and R2803 were diluted with the target cell (AsPC-1 cell) culture medium. Starting at a concentration of 20 µg/mL, a 40-fold gradient dilution was performed to obtain 3 concentration gradients for later use. The collected target cells were resuspended and spread on the plate with 50 µL per well (2×10⁴ cells). The collected effector cells (NK92 cells) were added with 100 µL per well (1.2×10⁵ cells). Then, 50 µL of the diluted antibody was added and incubated at 37 °C for 6 hours. 20 µL of a lysis solution was added to a maximum release well of the target cell 45 minutes in advance. Centrifugation was performed, and 40 µL of the supernatant was transferred to a new 96-well transparent plate. A CytoTox 96^{®} (Promega, G1780) reagent was added with 40 µL per well, and well mixing for 30 minutes through shaking at room temperature. Then 40 µL/well of a stop solution was added and well mixing for 5-10 minutes through shaking at room temperature, and then the result at OD490 nm was read in a microplate reader. The killing activity of the cells mediated by the antibody was analyzed and calculated with software GraphPadPrism 7.0.

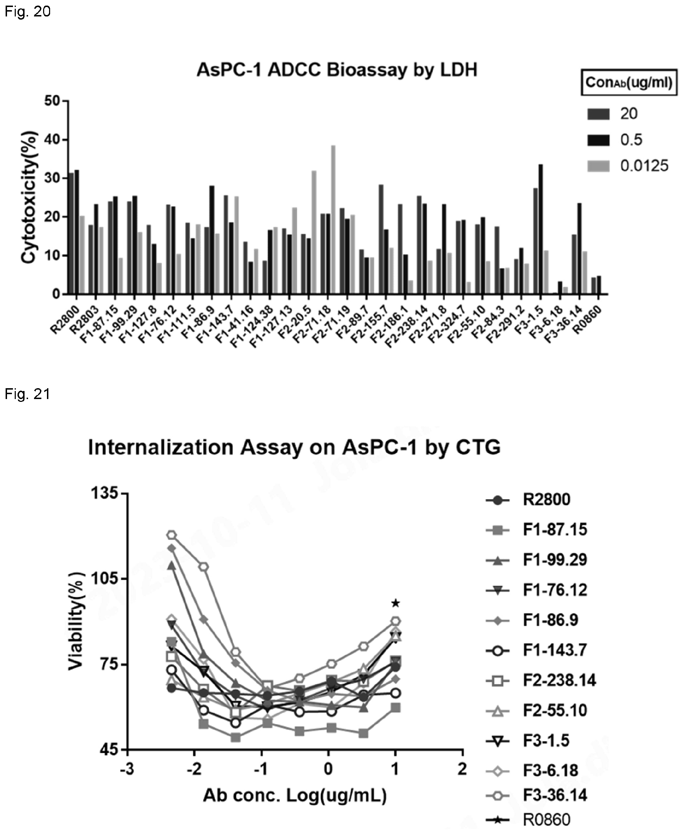

As shown in Fig. 20, most antibodies in the mouse monoclonal antibodies had detectable ADCC activity, with F1-87.15, F1-76.12, F2-155.7, F2-186.1, F2-238.14, F2-324.7, F2-55.10, F2-84.3, and F3-1.5 being superior.

### Embodiment 8 Detection of cell endocytosis activity mediated by monoclonal antibody

According to the above detection results, the endocytosis activity of AsPC-1 cells to F1-87.15, F1-99.29, F1-76.12, F1-86.9, F1-143.7, F2-238.14, F2-55.10, F3-1.5, F3-6.18, and F3-36.14, as well as a positive antibody, was detected.

Sufficient AsPC-1 cells were cultured, digested, centrifuged, and collected, the cell density was adjusted to 1×10⁵ cells/mL, and the cells were spread on the plate with 50 µL per well and placed overnight. Starting with a 10 µg/mL of the antibody diluted by the medium corresponding to the AsPC-1 cells, a 3-fold concentration dilution was performed to obtain 8 concentration points. A secondary antibody anti-mIgG FC-MMAF (MORADEC, AM-102-AF) was simultaneously diluted to 2 µg/mL. The antibody and the secondary antibody were added with 25 µL per well and well mixed, then placed in a 37 °C incubator, allowed to stand, and incubated for 3 days. CellTiter-Glo^{®} Luminescent Cell Viability Assay (Promega, G7571) was added with 100 µL per well and well mixed in a plate shaker. Luminescence was read in a microplate reader, and the endocytosis activity of the antibody was analyzed and calculated with software GraphPadPrism 7.0. The results were shown in Fig. 21.

### Embodiment 9 Preparation of conventional humanized CDH-17 mouse-derived antibody

Mouse-derived antibody sequences of F2-155.7 and F2-89.7 were respectively used to prepare hIgG1 subtype chimeric antibodies R4723 and R4850.

A humanized antibody was prepared with mouse-derived antibody sequences of R4723 and R4850. Three-dimensional structure homology modeling was performed on the selected sequences, and acceptable structures were evaluated and determined by methods such as a ramachandran plot, etc. By comparing the antibody sequences with an antibody GermLine database, a racial genealogy template with high homology was obtained. The CDR was transplanted into a corresponding human-source template. Three-dimensional structural simulation and analysis were performed again on the transplanted antibodies. Embedding residues, residues directly interacting with the CDR, and residues significantly affecting variable region conformation were subjected to restoration hybridization, and chemically-unstable amino acid residues in the CDR were optimized, so as to produce a series of humanized antibody molecules R4709-R4718 for R4723. Amino acid sequences of the heavy chain variable region of the antibodies were shown in Table 24, the amino acid sequences of the light chain variable region were shown in Table 25, and combination patterns of the heavy chain variable region and the light chain variable region were shown in Table 26. A series of humanized antibody molecules R4832-R4843 were produced for R4850. The amino acid sequences of the heavy chain variable region were shown in Table 27, the amino acid sequences of the light chain variable region were shown in Table 28, and the combination patterns of the heavy chain variable region and the light chain variable region were in Table 29.

**Table 24 Heavy chain variable region sequence of humanized antibody of R4723**

| | |
|---|---|
| SEQ ID NO:599 | QVQLVQSGAEVKKPGSSVKVSCKASGYTLTSHWIHWVRQAPGQGLEWIGR IYPGTGYTYYNENFRGRVTITADKSTNTAYMELSSLRSEDTAVYYCARSYDA YFVGYFDYWGQGTTVTVSS |
| SEQ ID NO:600 | QVQLVQSGAEVKKPGSSVKVSCKASGYTLTSHWIHWIRQAPGQGLEWIGRI YPGTGYTYYNENFRGRVTITADKSTNTAYMELSSLRSEDTAVYYCARSYDAY FVGYFDYWGQGTTVTVSS |
| SEQ ID NO:601 | QVQLVQSGAEVKKPGSSVKVSCKASGYTLTSHWIHWIRQAPGQGLEWIGRI YPGTGYTYYNENFRGRVTLTADKSTNTAYMELSSLRSEDTAVYYCARSYDAY FVGYFDYWGQGTTVTVSS |
| SEQ ID NO:602 | QVQLVQSGAEVKKPGSSVKVSCKTSGYTLTSHWIHWIKQAPGQGLEWIGRI YPGTGYTYYNENFRGRVTLTADKSTNTAYMELSSLRSEDTAVYYCARSYDAY FVGYFDYWGQGTTVTVSS |
| SEQ ID NO:603 | QVQLVQSGAEVKKPGSSVKVSCKTSGYTLTSHWIHWIKQAPGQGLEWIARI YPGTGYTYYNENFRGKATLTADKSTNTAYMELSSLRSEDTAVYYCARSYDAY FVGYFDYWGQGTTVTVSS |

**Table 25 Light chain variable region sequence of humanized antibody of R4723**

| | |
|---|---|
| SEQ ID NO:604 | DIVLTQSPASLAVSPGQRATITCRASESVDSYGNSIMHWYQQKPGQPPKLLI YRASNLESGVPARFSGSGSRTDFTLTINPVEANDTANYYCQQSNEDPYTFG QGTKLEIK |
| SEQ ID NO:605 | DIVLTQSPASLAVSPGQRATITCRASESVDSYGNSIMHWYQQKPGQPPKLLI YRASNLESGIPARFSGSGSRTDFTLTINPVEANDTANYYCQQSNEDPYTFGQ GTKLEIK |

**Table 26 Combination of heavy chain variable region and light chain variable region of humanized antibody of R4723**

| Number | Heavy chain | Light chain |
|---|---|---|
| R4709 | SEQ ID NO:599 | SEQ ID NO:604 |
| R4710 | SEQ ID NO:599 | SEQ ID NO:605 |
| R4711 | SEQ ID NO:600 | SEQ ID NO:604 |
| R4712 | SEQ ID NO:600 | SEQ ID NO:605 |
| R4713 | SEQ ID NO:601 | SEQ ID NO:604 |
| R4714 | SEQ ID NO:601 | SEQ ID NO:605 |
| R4715 | SEQ ID NO:602 | SEQ ID NO:604 |
| R4716 | SEQ ID NO:602 | SEQ ID NO:605 |
| R4717 | SEQ ID NO:603 | SEQ ID NO:604 |
| R4718 | SEQ ID NO:603 | SEQ ID NO:605 |

**Table 27 Heavy chain variable region sequence of humanized antibody of R4850**

| | |
|---|---|
| SEQ ID NO:606 | EVQLVESGGGLVQPGGSLRLSCAASGFTFSTYAMSWVRQAPGKGLEWVAS ISSGGSTYYVDSVKGRFTISRDNAKNSLYLQMNSLRAEDTAVYYCTRGSFFT TVITDFDYWGQGTTVTVSS |
| SEQ ID NO:607 | EVQLVESGGGLVQPGGSLRLSCAASGFTFSTYAMSWVRQAPGKGLEWVAS ISSGGSTYYPDSVRGRFTISRDNAKNSLYLQMNSLRAEDTAVYYCTRGSFFT TVITDFDYWGQGTTVTVSS |
| SEQ ID NO:608 | EVQLVESGGGLVQPGGSLRLSCAASGFTFSTYAMSWVRQAPGKGLEWVAS ISSGGSTYYPDSVRGRFTISRDNAKNILYLQMNSLRAEDTAVYYCTRGSFFTT VITDFDYWGQGTTVTVSS |
| SEQ ID NO:609 | EVKLVESGGGLVQPGGSLRLSCAASGFTFSTYAMSWVRQAPGKRLEWVASI SSGGSTYYPDSVRGRFTISRDNAKNILYLQMSSLRAEDTAVYYCTRGSFFTT VITDFDYWGQGTTVTVSS |

**Table 28 Light chain variable region sequence of humanized antibody of R4850**

| | |
|---|---|
| SEQ ID NO:610 | DIQMTQSPSSLSASVGDRVTITCRASQEISGYLSWLQQKPGKAPKRLIYAAS TLDSGVPSRFSGSRSGTEFTLTISSLQPEDFATYYCLQYAIYPWTFGQGTKL EIK |
| SEQ ID NO:611 | DIQMTQSPSSLSASVGDRVTITCRASQEISGYLSWLQQKPGKAPKRLIYAAS TLDSGVPKRFSGSRSGTEFTLTISSLQPEDFATYYCLQYAIYPWTFGQGTKL EIK |
| SEQ ID NO:612 | DIQMTQSPSSLSASVGDRVTITCRASQEISGYLSWLQQKPGKAPKRLIYAAS TLDSGVPKRFSGSRSGSDFTLTISSLQPEDFATYYCLQYAIYPWTFGQGTKL EIK |
| SEQ ID NO:613 | DIQMTQSPSSLSASVGDRVTITCRASQEISGYLSWLQQKPGKAPKRLIYAAS TLDSGVPKRFSGSRSGSDYTLTISSLQPEDFATYYCLQYAIYPWTFGQGTKL EIK |
| SEQ ID NO:614 | DIQMTQSPSSLSASVGDRVTITCRASQEISGYLSWLQQKPGGAIKRLIYAAST LDSGVPKRFSGSRSGSDYTLTISSLQPEDFATYYCLQYAIYPWTFGQGTKLEI K |

**Table 29 Combination of heavy chain variable region and light chain variable region of humanized antibody of R4850**

| Number | Heavy chain | Light chain |
|---|---|---|
| R4832 | SEQ ID NO:606 | SEQ ID NO:613 |
| R4833 | SEQ ID NO:607 | SEQ ID NO:610 |
| R4834 | SEQ ID NO:607 | SEQ ID NO:611 |
| R4835 | SEQ ID NO:607 | SEQ ID NO:612 |
| R4836 | SEQ ID NO:607 | SEQ ID NO:613 |
| R4837 | SEQ ID NO:608 | SEQ ID NO:611 |
| R4838 | SEQ ID NO:608 | SEQ ID NO:612 |
| R4839 | SEQ ID NO:608 | SEQ ID NO:613 |
| R4840 | SEQ ID NO:609 | SEQ ID NO:611 |
| R4841 | SEQ ID NO:609 | SEQ ID NO:612 |
| R4842 | SEQ ID NO:609 | SEQ ID NO:613 |
| R4843 | SEQ ID NO:609 | SEQ ID NO:614 |

### Embodiment 10 Detection of binding activity of humanized antibody

CHO-CDH17 constructing and overexpressing the human CDH17 served as positive cells, while AsPC-1 (human metastatic pancreatic adenocarcinoma cells) and OCUM-1 served as positive cells endogenously expressing the CDH17. A flow cytometry analysis method was used to detect the binding activity of a monoclonal antibody to the CHO-CDH17 cells, the AsPC-1 cells, and OCUM-1 cells.

### 1. CHO-CDH17 cell binding

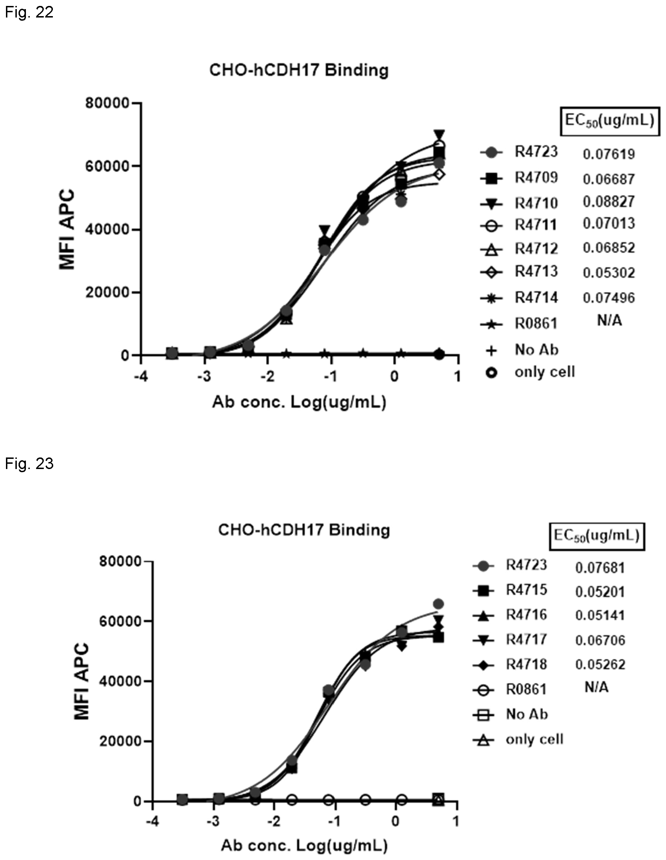

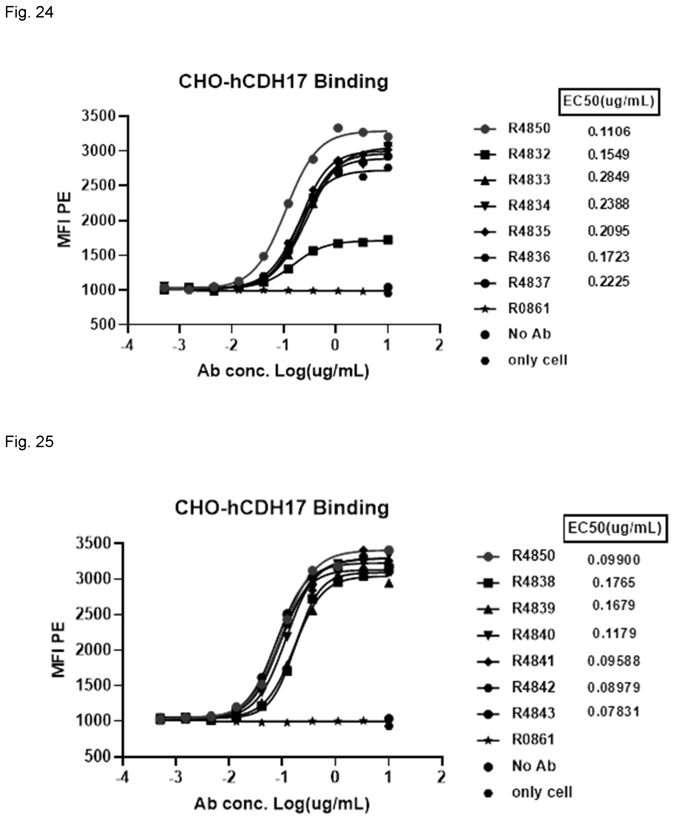

Sufficient CHO-CDH17 cells were cultured, centrifuged, and collected. The monoclonal antibody and corresponding positive control antibodies were diluted simultaneously with PBS+3%FBS, starting at a concentration of 10 µg/mL or 5 µg/mL, with a 3-fold or 4-fold gradient dilution, so as to obtain 10 or 8 concentration gradients for later use. The collected cells were washed once with the PBS+3%FBS, then the PBS+3%FBS was added to resuspend the cells to 2×10⁶ cells/mL. The cells were spread on a 96-well plate with 100 µL per well (2×10⁵ cells), and 100 µL of the diluted antibodies was added and incubated at 4 °C for 30 minutes. Centrifugation was performed; the supernatant was discarded. The cells were washed twice with PBS, resuspended with a diluted APC-labeled anti-human IgG FC antibody (Biolegend, 409306) or a PE-labeled anti-human IgG FC antibody (Biolegend, 409304), and incubated at 4 °C in the dark for 30 minutes. The cells were washed twice with the PBS and resuspended with 100 µL of the PBS. Loading was performed for detection. Then, a binding affinity EC₅₀ value of the antibodies and the CHO-CDH17 was analyzed and calculated with software GraphPad Prism 7.0 based on average fluorescence intensity. The results were shown in Figs. 22-Fig. 25. Compared with a parent antibody R4723, the binding activity of the humanized antibody was basically consistent with that of the parent antibody; and compared with a parent antibody R4850, the binding activity of partial antibody in the humanized antibody was basically consistent with that of the parent antibody. R0861 served as a hIgG1 negative control antibody.

### 2. AsPC-1 cell binding

The flow cytometry analysis method was used to detect the binding activity of the monoclonal antibodies to the AsPC-1 cells. The cells were digested with a Versene solution, centrifuged, and collected for later use. The antibodies were diluted in a 3-fold or 4-fold gradient starting at a concentration of 10 µg/mL or 5 µg/mL, so as to obtain 10 or 8 concentration gradients. After the antibodies and the cells were co-incubated, subsequent experimental procedures were identical to the CHO-CDH17 cell binding experiment. The cells were resuspended with 100 µL of the PBS. Loading was performed for detection. Then, a binding affinity EC₅₀ value of the antibodies and the AsPC-1 cells was analyzed and calculated with software GraphPadPrism 7.0 based on average fluorescence intensity.

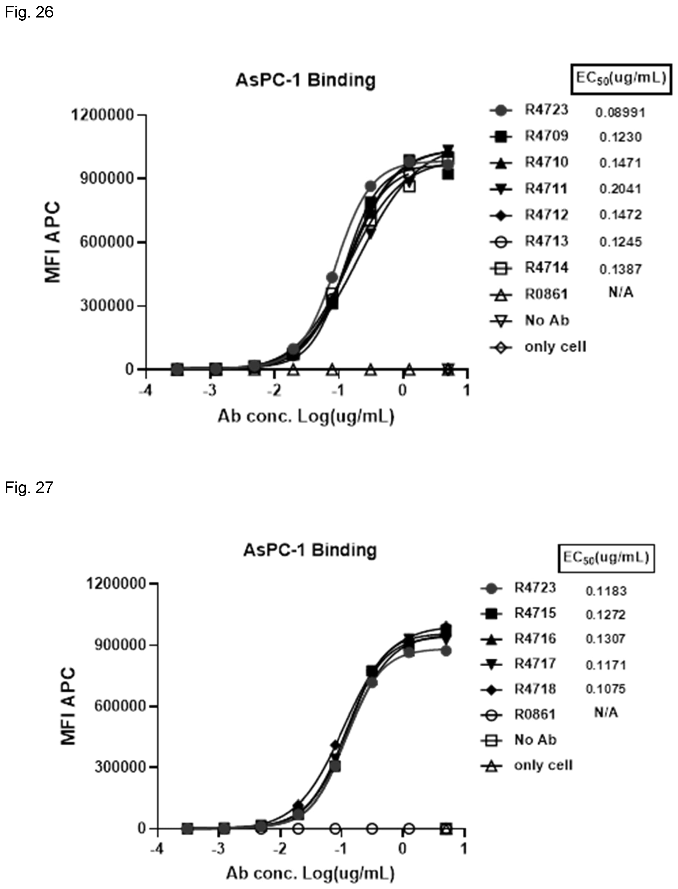

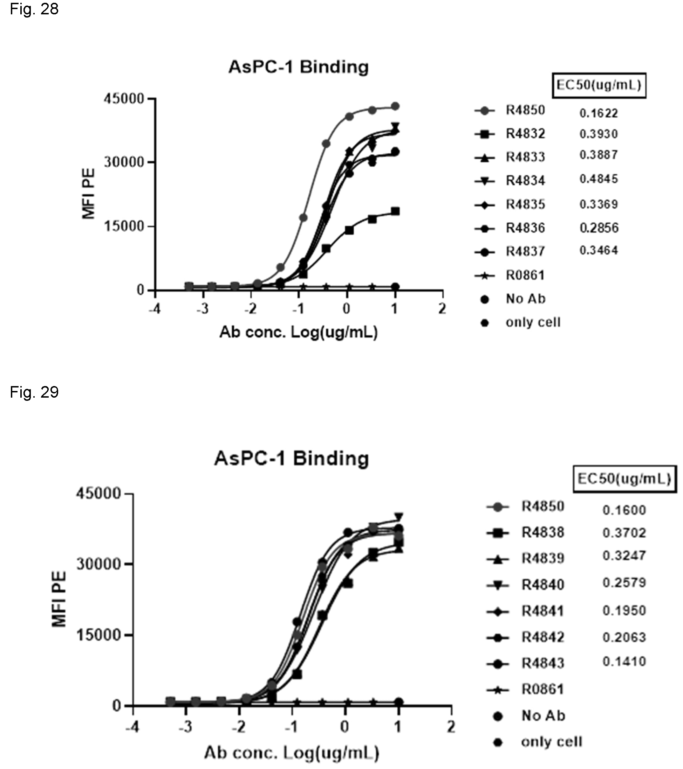

The results were shown in Figs. 26-Fig. 29. Compared with a parent antibody R4723, the binding activity of the humanized antibody was basically consistent with that of the parent antibody; and compared with a parent antibody R4850, the binding activity of partial antibody in the humanized antibody was basically consistent with that of the parent antibody. R0861 served as a hIgG1 negative control antibody.

### 3. OCUM-1 cell binding

The flow cytometry analysis method was used to detect the binding activity of the monoclonal antibodies to the OCUM-1 cells. The cells were collected, centrifuged, digested with a Versene buffer, and then centrifuged again for later use. The antibodies were diluted in a 3-fold or 4-fold gradient starting at a concentration of 10 µg/mL or 5 µg/mL, so as to obtain 10 or 8 concentration gradients. After the antibodies and the cells were co-incubated, subsequent experimental procedures were identical to the CHO-CDH17 cell binding experiment. The cells were resuspended with 100 µL of the PBS. Loading was performed for detection. Then, a binding affinity EC₅₀ value of the antibodies and the AsPC-1 cells was analyzed and calculated with software GraphPadPrism 7.0 based on average fluorescence intensity.

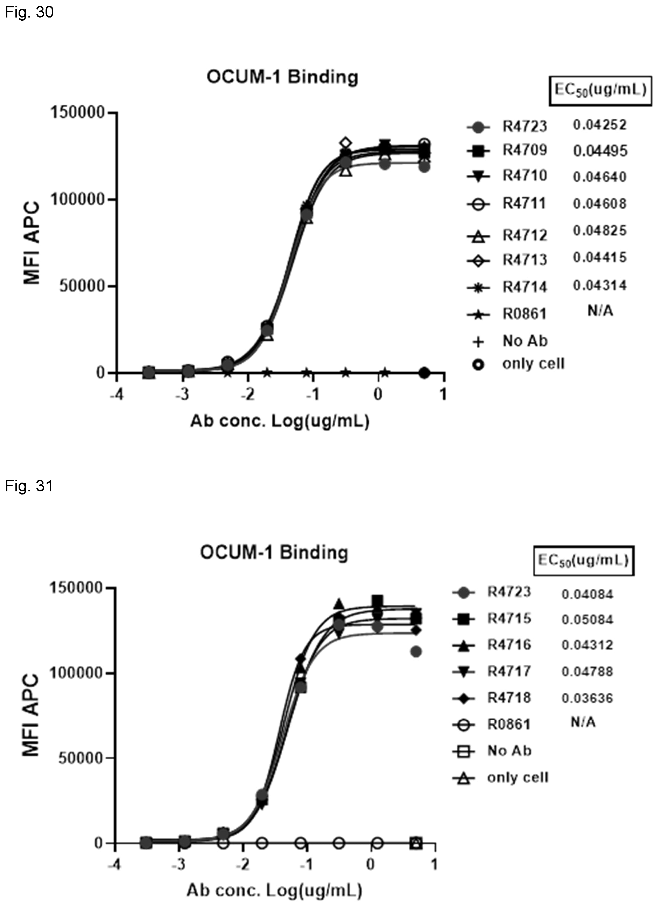

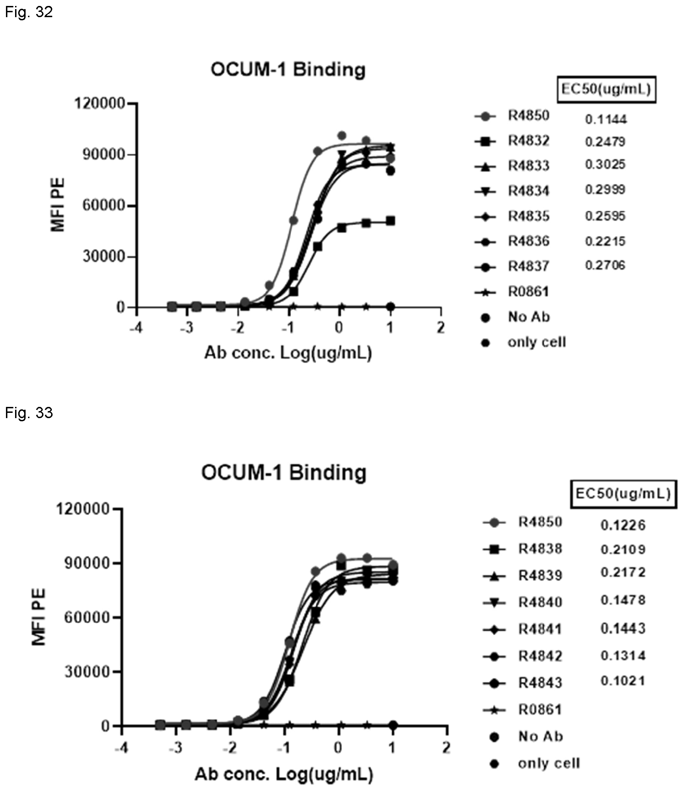

The results were shown in Figs. 30-Fig. 33. Compared with a parent antibody R4723, the binding activity of the humanized antibody was basically consistent with that of the parent antibody; and compared with a parent antibody R4850, the binding activity of partial antibody in the humanized antibody was basically consistent with that of the parent antibody. R0861 served as a hIgG1 negative control antibody.

### Embodiment 11 Detection of binding activity of humanized antibody to monkey CDH17

In this embodiment, the binding activity of the monoclonal antibody to monkey CDH17 (Acrobiosystem) was detected. A Cyno.CDH17 recombinant protein (monkey CDH17) was diluted to 0.5 µg/mL in advance with a 50 mmol Coating Buffer, added 100 µL to each well, and placed overnight at 4 °C. The supernatant was discarded. The plate was washed for three times with 1×PBST buffer. Then, the PBS+1%FBS was added with 100 µL/well and placed and blocked at 37 °C for 1 hour. Meanwhile, the antibody was diluted with the PBS+1%FBS. Starting at a concentration of 10 µg/mL, a 5-fold or 3-fold gradient dilution was performed to obtain 8 or 10 concentration gradients for later use. After blocking, the supernatant was discarded. 100 µL of the diluted antibody was added and incubated at 37 °C for 1 hour. After incubation, the plate was washed for four times with the 1×PBST buffer. Then 100 µL of a diluted HRP-conjugated anti-human IgG antibody (Sigma, A0170) was added and incubated at 37 °C for 30 minutes. After incubation, the plate was washed for five times with the 1×PBST buffer. An appropriate amount of a color development solution was added, and color development was terminated after color development. The absorbance was measured at 450 nm using a microplate reader, a binding affinity EC₅₀ value of the antibodies and the Cyno.CDH17 recombinant protein was analyzed and calculated with software GraphPadPrism 7.0.

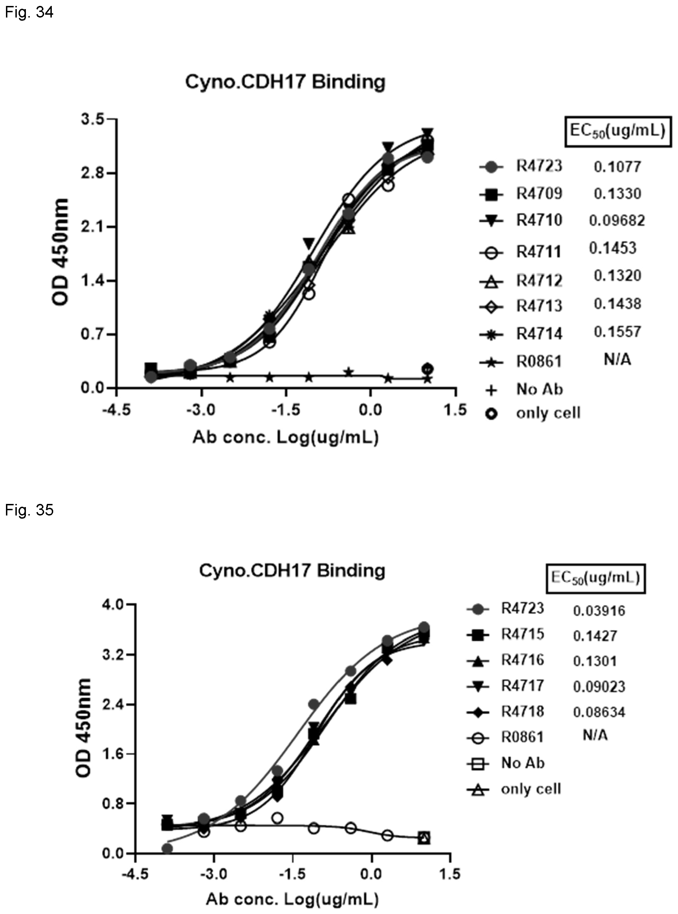

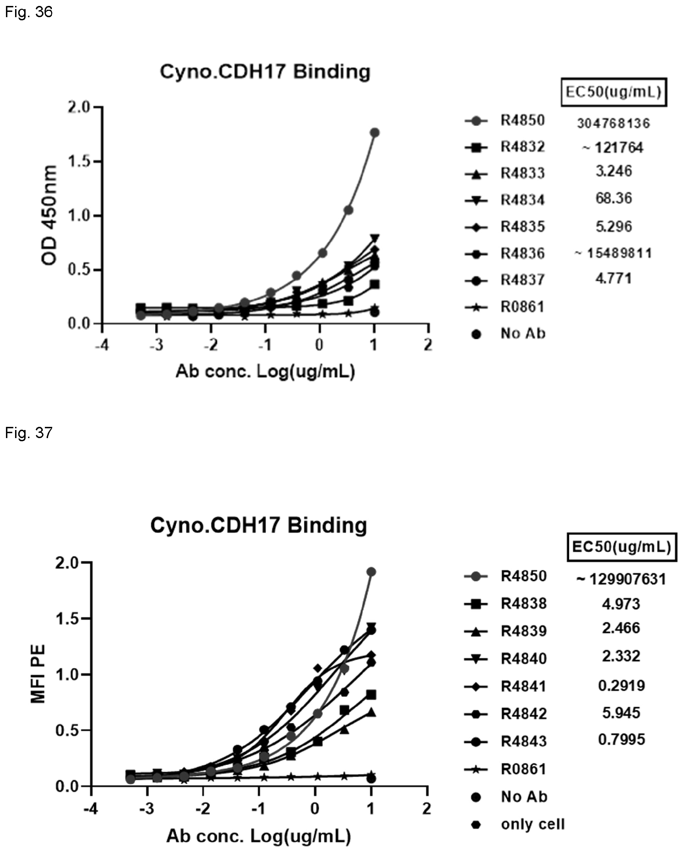

The results were shown in Fig. 34-Fig. 37. Compared with a parent antibody R4723, the binding activity of the humanized antibody was basically consistent with that of the parent antibody; and compared with a parent antibody R4850, the binding activity of partial antibody in the humanized antibody was relatively close to that of the parent antibody. R0861 served as a hIgG1 negative control antibody.

It is to be noted at last: the above various embodiments are only used to illustrate the technical solutions of the present disclosure and not used to limit the same. Although the present disclosure has been described in detail with reference to the foregoing embodiments, for those of ordinary skill in the art, they can still modify the technical solutions described in the foregoing embodiments, or equivalently replace part or all of the technical features; all these modifications and replacements shall not cause the essence of the corresponding technical solutions to depart from the scope of the technical solutions of the embodiments of the present disclosure.

## Claims

1. An anti-CDH17 antibody or an antigen-binding fragment, wherein the anti-CDH17 antibody or the antigen-binding fragment binds to one or more of a CDH17 Extra Cellular Domain (ECD) 1, a CDH17 ECD 2, a CDH17 ECD 3, a CDH17 ECD 4, a CDH17 ECD 5, a CDH17 ECD 6, and a CDH17 ECD 7.

2. The anti-CDH17 antibody or the antigen-binding fragment thereof according to claim 1, comprising Complementary Determinant Regions (CDRs) HCDR1, HCDR2, and HCDR3 of a heavy chain variable region, and/or, CDRs LCDR1, LCDR2, and LCDR3 of a light chain variable region, wherein the HCDR1 comprises an amino acid sequence of DY, an amino acid sequence of EY, an amino acid sequence of GY, an amino acid sequence of NY, an amino acid sequence of SG, an amino acid sequence of SH, an amino acid sequence of SY, an amino acid sequence of TS, or an amino acid sequence of TY;
the HCDR2 comprises an amino acid sequence shown in any one of SEQ ID NOs: 184-186, 188, 191-199, 202-205, or 208;
the HCDR3 comprises an amino acid sequence shown in any one of SEQ ID NOs: 209, 212, 215, 218, 221, 224, 227, 230, 233, 236, 239, 242, 245, 248, 251, 254, 257, 260, or 278;
the LCDR1 comprises an amino acid sequence of NID, an amino acid sequence of NNF, an amino acid sequence of NNW, an amino acid sequence of NY, an amino acid sequence of SDF, an amino acid sequence of SGY, an amino acid sequence of SND, an amino acid sequence of SSA, or an amino acid sequence of SY, or an amino acid sequence shown in any one of SEQ ID NOs: 290, 294, 299, 308, 319, or 332;
the LCDR2 comprises an amino acid sequence of AA, an amino acid sequence of DT, an amino acid sequence of FA, an amino acid sequence of GA, an amino acid sequence of KV, an amino acid sequence of RA, an amino acid sequence of RT, an amino acid sequence of SA, an amino acid sequence of ST, an amino acid sequence of WA, an amino acid sequence of YA, or an amino acid sequence of YT;
the LCDR3 comprises an amino acid sequence shown in any one of SEQ ID NOs: 368, 370, 372, 374, 376, 378, 380, 382, 384, 386, 388, 390, 392, 394, or 396;
optionally, the HCDR1, HCDR2, and HCDR3 comprise amino acid sequences consistent with those of HCDR1, HCDR2, and HCDR3 of a heavy chain variable region shown in any one of SEQ ID NOs: 1, 3, 5, 7, 9, 11, 13, 15, 17, 19, 21, 23, 25, 27, 29, 31, 33, 35, 37, or 39, and the LCDR1, LCDR2, and LCDR3 comprise amino acid sequences consistent with those of LCDR1, LCDR2, and LCDR3 of a light chain variable region shown in any one of SEQ ID NO: 2, 4, 6, 8, 10, 12, 14, 16, 18, 20, 22, 24, 26, 28, 30, 32, 34, 36, 38, or 40;
optionally, the HCDR1, HCDR2, HCDR3, LCDR1, LCDR2, or LCDR3 of the variable region is defined by any one system of Kabat, Chothia, IMGT, ABM, and Contact or a combination of a plurality of definition systems;
optionally, according to an IMGT definition manner:
the HCDR1 comprises an amino acid sequence shown in any one of SEQ ID NOs: 45, 50, 54, 58, 63, 67, 72, 77, 81, 85, 90, 95, 100, 104, 107, or 113;
the HCDR2 comprises an amino acid sequence shown in any one of SEQ ID NOs: 121, 125, 129, 133, 137, 141, 145, 149, 153, 157, 162, 167, 171, 175, 179, 183, 189, or 190;
the HCDR3 comprises an amino acid sequence shown in any one of SEQ ID NOs: 211, 214, 217, 220, 223, 226, 229, 232, 235, 238, 241, 244, 247, 250, 253, 256, 259, 262, or 266;
the LCDR1 comprises an amino acid sequence shown in any one of SEQ ID NOs: 286, 288, 292, 293, 296, 298, 301, 303, 305, 307, 310, 312, 321, 322, or 333;
the LCDR2 comprises an amino acid sequence of AA, an amino acid sequence of DT, an amino acid sequence of FA, an amino acid sequence of GA, an amino acid sequence of KV, an amino acid sequence of RA, an amino acid sequence of RT, an amino acid sequence of SA, an amino acid sequence of ST, an amino acid sequence of WA, an amino acid sequence of YA, or an amino acid sequence of YT; and
the LCDR3 comprises an amino acid sequence shown in any one of SEQ ID NOs: 369, 371, 373, 375, 377, 379, 381, 383, 385, 387, 389, 391, 393, 395, or 397.

3. The anti-CDH17 antibody or the antigen-binding fragment thereof according to claim 1 or 2, wherein the CDR of the anti-CDH17 antibody or the antigen-binding fragment comprises any one of the following (a)-(t):
(a) the HCDR1 comprises the amino acid sequence of SY, the HCDR2 comprises an amino acid sequence shown in SEQ ID NO: 185, the HCDR3 comprises an amino acid sequence shown in SEQ ID NO: 209, the LCDR1 comprises the amino acid sequence of SND, the LCDR2 comprises the amino acid sequence of YA, and the LCDR3 comprises an amino acid sequence shown in SEQ ID NO: 386; or
(b) the HCDR1 comprises the amino acid sequence of EY, the HCDR2 comprises an amino acid sequence shown in SEQ ID NO: 192, the HCDR3 comprises an amino acid sequence shown in SEQ ID NO: 278, the LCDR1 comprises an amino acid sequence shown in SEQ ID NO: 319, the LCDR2 comprises the amino acid sequence of WA, and the LCDR3 comprises an amino acid sequence shown in SEQ ID NO: 376; or
(c) the HCDR1 comprises the amino acid sequence of SY, the HCDR2 comprises an amino acid sequence shown in SEQ ID NO: 203, the HCDR3 comprises an amino acid sequence shown in SEQ ID NO: 212, the LCDR1 comprises the amino acid sequence of NNF, the LCDR2 comprises the amino acid sequence of RT, and the LCDR3 comprises an amino acid sequence shown in SEQ ID NO: 378; or
(d) the HCDR1 comprises the amino acid sequence of SY, the HCDR2 comprises an amino acid sequence shown in SEQ ID NO: 196, the HCDR3 comprises an amino acid sequence shown in SEQ ID NO: 215, the LCDR1 comprises the amino acid sequence of SY, the LCDR2 comprises the amino acid sequence of DT, and the LCDR3 comprises an amino acid sequence shown in SEQ ID NO: 372; or
(e) the HCDR1 comprises the amino acid sequence of GY, the HCDR2 comprises an amino acid sequence shown in SEQ ID NO: 197, the HCDR3 comprises an amino acid sequence shown in SEQ ID NO: 218, the LCDR1 comprises an amino acid sequence shown in SEQ ID NO: 332, the LCDR2 comprises the amino acid sequence of ST, and the LCDR3 comprises an amino acid sequence shown in SEQ ID NO: 374; or
(f) the HCDR1 comprises the amino acid sequence of SH, the HCDR2 comprises an amino acid sequence shown in SEQ ID NO: 208, the HCDR3 comprises an amino acid sequence shown in SEQ ID NO: 221, the LCDR1 comprises an amino acid sequence shown in SEQ ID NO: 290, the LCDR2 comprises the amino acid sequence of RA, and the LCDR3 comprises an amino acid sequence shown in SEQ ID NO: 394; or
(g) the HCDR1 comprises the amino acid sequence of TY, the HCDR2 comprises an amino acid sequence shown in SEQ ID NO: 191, the HCDR3 comprises an amino acid sequence shown in SEQ ID NO: 224, the LCDR1 comprises an amino acid sequence shown in SEQ ID NO: 332, the LCDR2 comprises the amino acid sequence of ST, and the LCDR3 comprises an amino acid sequence shown in SEQ ID NO: 374; or
(h) the HCDR1 comprises the amino acid sequence of GY, the HCDR2 comprises an amino acid sequence shown in SEQ ID NO: 195, the HCDR3 comprises an amino acid sequence shown in SEQ ID NO: 227, the LCDR1 comprises an amino acid sequence shown in SEQ ID NO: 294, the LCDR2 comprises the amino acid sequence of YA, and the LCDR3 comprises an amino acid sequence shown in SEQ ID NO: 382; or
(i) the HCDR1 comprises the amino acid sequence of SG, the HCDR2 comprises an amino acid sequence shown in SEQ ID NO: 205, the HCDR3 comprises an amino acid sequence shown in SEQ ID NO: 230, the LCDR1 comprises the amino acid sequence of SSA, the LCDR2 comprises the amino acid sequence of SA, and the LCDR3 comprises an amino acid sequence shown in SEQ ID NO: 392; or
(j) the HCDR1 comprises the amino acid sequence of TS, the HCDR2 comprises an amino acid sequence shown in SEQ ID NO: 186, the HCDR3 comprises an amino acid sequence shown in SEQ ID NO: 233, the LCDR1 comprises an amino acid sequence shown in SEQ ID NO: 299, the LCDR2 comprises the amino acid sequence of KV, and the LCDR3 comprises an amino acid sequence shown in SEQ ID NO: 370; or
(k) the HCDR1 comprises the amino acid sequence of EY, the HCDR2 comprises an amino acid sequence shown in SEQ ID NO: 194, the HCDR3 comprises an amino acid sequence shown in SEQ ID NO: 278, the LCDR1 comprises an amino acid sequence shown in SEQ ID NO: 319, the LCDR2 comprises the amino acid sequence of WA, and the LCDR3 comprises an amino acid sequence shown in SEQ ID NO: 376; or
(l) the HCDR1 comprises the amino acid sequence of SY, the HCDR2 comprises an amino acid sequence shown in SEQ ID NO: 199, the HCDR3 comprises an amino acid sequence shown in SEQ ID NO: 236, the LCDR1 comprises the amino acid sequence of SY, the LCDR2 comprises the amino acid sequence of DT, and the LCDR3 comprises an amino acid sequence shown in SEQ ID NO: 368; or
(m) the HCDR1 comprises the amino acid sequence of EY, the HCDR2 comprises an amino acid sequence shown in SEQ ID NO: 194, the HCDR3 comprises an amino acid sequence shown in SEQ ID NO: 239, the LCDR1 comprises an amino acid sequence shown in SEQ ID NO: 319, the LCDR2 comprises the amino acid sequence of WA, and the LCDR3 comprises an amino acid sequence shown in SEQ ID NO: 376; or
(n) the HCDR1 comprises the amino acid sequence of DY, the HCDR2 comprises an amino acid sequence shown in SEQ ID NO: 193, the HCDR3 comprises an amino acid sequence shown in SEQ ID NO: 242, the LCDR1 comprises the amino acid sequence of NNW, the LCDR2 comprises the amino acid sequence of GA, and the LCDR3 comprises an amino acid sequence shown in SEQ ID NO: 396; or
(o) the HCDR1 comprises the amino acid sequence of TY, the HCDR2 comprises an amino acid sequence shown in SEQ ID NO: 202, the HCDR3 comprises an amino acid sequence shown in SEQ ID NO: 245, the LCDR1 comprises the amino acid sequence of NY, the LCDR2 comprises the amino acid sequence of YT, and the LCDR3 comprises an amino acid sequence shown in SEQ ID NO: 388; or
(p) the HCDR1 comprises the amino acid sequence of NY, the HCDR2 comprises an amino acid sequence shown in SEQ ID NO: 198, the HCDR3 comprises an amino acid sequence shown in SEQ ID NO: 248, the LCDR1 comprises the amino acid sequence of SDF, the LCDR2 comprises the amino acid sequence of YA, and the LCDR3 comprises an amino acid sequence shown in SEQ ID NO: 384; or
(q) the HCDR1 comprises the amino acid sequence of TY, the HCDR2 comprises an amino acid sequence shown in SEQ ID NO: 204, the HCDR3 comprises an amino acid sequence shown in SEQ ID NO: 251, the LCDR1 comprises the amino acid sequence of SGY, the LCDR2 comprises the amino acid sequence of AA, and the LCDR3 comprises an amino acid sequence shown in SEQ ID NO: 380; or
(r) the HCDR1 comprises the amino acid sequence of TY, the HCDR2 comprises an amino acid sequence shown in SEQ ID NO: 204, the HCDR3 comprises an amino acid sequence shown in SEQ ID NO: 254, the LCDR1 comprises the amino acid sequence of SGY, the LCDR2 comprises the amino acid sequence of AA, and the LCDR3 comprises an amino acid sequence shown in SEQ ID NO: 380; or
(s) the HCDR1 comprises the amino acid sequence of NY, the HCDR2 comprises an amino acid sequence shown in SEQ ID NO: 184, the HCDR3 comprises an amino acid sequence shown in SEQ ID NO: 257, the LCDR1 comprises an amino acid sequence shown in SEQ ID NO: 308, the LCDR2 comprises the amino acid sequence of FA, and the LCDR3 comprises an amino acid sequence shown in SEQ ID NO: 382; or
(t) the HCDR1 comprises the amino acid sequence of DY, the HCDR2 comprises an amino acid sequence shown in SEQ ID NO: 188, the HCDR3 comprises an amino acid sequence shown in SEQ ID NO: 260, the LCDR1 comprises the amino acid sequence of NID, the LCDR2 comprises the amino acid sequence of SA, and the LCDR3 comprises an amino acid sequence shown in SEQ ID NO: 390; and
optionally, according to the IMGT definition manner, the CDR of the anti-CDH17 antibody or the antigen-binding fragment comprises any one of the following (a')-(t'):
(a') the HCDR1 comprises an amino acid sequence shown in SEQ ID NO: 63, the HCDR2 comprises an amino acid sequence shown in SEQ ID NO: 141, the HCDR3 comprises an amino acid sequence shown in SEQ ID NO: 223, the LCDR1 comprises an amino acid sequence shown in SEQ ID NO: 292, the LCDR2 comprises the amino acid sequence of RA, and the LCDR3 comprises an amino acid sequence shown in SEQ ID NO: 395; or
(b') the HCDR1 comprises an amino acid sequence shown in SEQ ID NO: 113, the HCDR2 comprises an amino acid sequence shown in SEQ ID NO: 125, the HCDR3 comprises an amino acid sequence shown in SEQ ID NO: 266, the LCDR1 comprises an amino acid sequence shown in SEQ ID NO: 322, the LCDR2 comprises the amino acid sequence of WA, and the LCDR3 comprises an amino acid sequence shown in SEQ ID NO: 377; or
(c') the HCDR1 comprises an amino acid sequence shown in SEQ ID NO: 50, the HCDR2 comprises an amino acid sequence shown in SEQ ID NO: 129, the HCDR3 comprises an amino acid sequence shown in SEQ ID NO: 214, the LCDR1 comprises an amino acid sequence shown in SEQ ID NO: 288, the LCDR2 comprises the amino acid sequence of RT, and the LCDR3 comprises an amino acid sequence shown in SEQ ID NO: 379; or
(d') the HCDR1 comprises an amino acid sequence shown in SEQ ID NO: 54, the HCDR2 comprises an amino acid sequence shown in SEQ ID NO: 133, the HCDR3 comprises an amino acid sequence shown in SEQ ID NO: 217, the LCDR1 comprises an amino acid sequence shown in SEQ ID NO: 333, the LCDR2 comprises the amino acid sequence of DT, and the LCDR3 comprises an amino acid sequence shown in SEQ ID NO: 373; or
(e') the HCDR1 comprises an amino acid sequence shown in SEQ ID NO: 58, the HCDR2 comprises an amino acid sequence shown in SEQ ID NO: 137, the HCDR3 comprises an amino acid sequence shown in SEQ ID NO: 220, the LCDR1 comprises an amino acid sequence shown in SEQ ID NO: 293, the LCDR2 comprises the amino acid sequence of ST, and the LCDR3 comprises an amino acid sequence shown in SEQ ID NO: 375; or
(f') the HCDR1 comprises an amino acid sequence shown in SEQ ID NO: 45, the HCDR2 comprises an amino acid sequence shown in SEQ ID NO: 121, the HCDR3 comprises an amino acid sequence shown in SEQ ID NO: 211, the LCDR1 comprises an amino acid sequence shown in SEQ ID NO: 286, the LCDR2 comprises the amino acid sequence of YA, and the LCDR3 comprises an amino acid sequence shown in SEQ ID NO: 387; or
(g') the HCDR1 comprises an amino acid sequence shown in SEQ ID NO: 107, the HCDR2 comprises an amino acid sequence shown in SEQ ID NO: 145, the HCDR3 comprises an amino acid sequence shown in SEQ ID NO: 226, the LCDR1 comprises an amino acid sequence shown in SEQ ID NO: 293, the LCDR2 comprises the amino acid sequence of ST, and the LCDR3 comprises an amino acid sequence shown in SEQ ID NO: 375; or
(h') the HCDR1 comprises an amino acid sequence shown in SEQ ID NO: 67, the HCDR2 comprises an amino acid sequence shown in SEQ ID NO: 149, the HCDR3 comprises an amino acid sequence shown in SEQ ID NO: 229, the LCDR1 comprises an amino acid sequence shown in SEQ ID NO: 296, the LCDR2 comprises the amino acid sequence of YA, and the LCDR3 comprises an amino acid sequence shown in SEQ ID NO: 383; or
(i') the HCDR1 comprises an amino acid sequence shown in SEQ ID NO: 72, the HCDR2 comprises an amino acid sequence shown in SEQ ID NO: 153, the HCDR3 comprises an amino acid sequence shown in SEQ ID NO: 232, the LCDR1 comprises an amino acid sequence shown in SEQ ID NO: 298, the LCDR2 comprises the amino acid sequence of SA, and the LCDR3 comprises an amino acid sequence shown in SEQ ID NO: 393; or
(j') the HCDR1 comprises an amino acid sequence shown in SEQ ID NO: 77, the HCDR2 comprises an amino acid sequence shown in SEQ ID NO: 157, the HCDR3 comprises an amino acid sequence shown in SEQ ID NO: 235, the LCDR1 comprises an amino acid sequence shown in SEQ ID NO: 301, the LCDR2 comprises the amino acid sequence of KV, and the LCDR3 comprises an amino acid sequence shown in SEQ ID NO: 371; or
(k') the HCDR1 comprises an amino acid sequence shown in SEQ ID NO: 113, the HCDR2 comprises an amino acid sequence shown in SEQ ID NO: 189, the HCDR3 comprises an amino acid sequence shown in SEQ ID NO: 266, the LCDR1 comprises an amino acid sequence shown in SEQ ID NO: 322, the LCDR2 comprises the amino acid sequence of WA, and the LCDR3 comprises an amino acid sequence shown in SEQ ID NO: 377; or
(l') the HCDR1 comprises an amino acid sequence shown in SEQ ID NO: 81, the HCDR2 comprises an amino acid sequence shown in SEQ ID NO: 162, the HCDR3 comprises an amino acid sequence shown in SEQ ID NO: 238, the LCDR1 comprises an amino acid sequence shown in SEQ ID NO: 333, the LCDR2 comprises the amino acid sequence of DT, and the LCDR3 comprises an amino acid sequence shown in SEQ ID NO: 369; or
(m') the HCDR1 comprises an amino acid sequence shown in SEQ ID NO: 113, the HCDR2 comprises an amino acid sequence shown in SEQ ID NO: 189, the HCDR3 comprises an amino acid sequence shown in SEQ ID NO: 241, the LCDR1 comprises an amino acid sequence shown in SEQ ID NO: 322, the LCDR2 comprises the amino acid sequence of WA, and the LCDR3 comprises an amino acid sequence shown in SEQ ID NO: 377; or
(n') the HCDR1 comprises an amino acid sequence shown in SEQ ID NO: 85, the HCDR2 comprises an amino acid sequence shown in SEQ ID NO: 167, the HCDR3 comprises an amino acid sequence shown in SEQ ID NO: 244, the LCDR1 comprises an amino acid sequence shown in SEQ ID NO: 303, the LCDR2 comprises the amino acid sequence of GA, and the LCDR3 comprises an amino acid sequence shown in SEQ ID NO: 397; or
(o') the HCDR1 comprises an amino acid sequence shown in SEQ ID NO: 90, the HCDR2 comprises an amino acid sequence shown in SEQ ID NO: 171, the HCDR3 comprises an amino acid sequence shown in SEQ ID NO: 247, the LCDR1 comprises an amino acid sequence shown in SEQ ID NO: 305, the LCDR2 comprises the amino acid sequence of YT, and the LCDR3 comprises an amino acid sequence shown in SEQ ID NO: 389; or
(p') the HCDR1 comprises an amino acid sequence shown in SEQ ID NO: 95, the HCDR2 comprises an amino acid sequence shown in SEQ ID NO: 175, the HCDR3 comprises an amino acid sequence shown in SEQ ID NO: 250, the LCDR1 comprises an amino acid sequence shown in SEQ ID NO: 307, the LCDR2 comprises the amino acid sequence of YA, and the LCDR3 comprises an amino acid sequence shown in SEQ ID NO: 385; or
(q') the HCDR1 comprises an amino acid sequence shown in SEQ ID NO: 107, the HCDR2 comprises an amino acid sequence shown in SEQ ID NO: 190, the HCDR3 comprises an amino acid sequence shown in SEQ ID NO: 253, the LCDR1 comprises an amino acid sequence shown in SEQ ID NO: 321, the LCDR2 comprises the amino acid sequence of AA, and the LCDR3 comprises an amino acid sequence shown in SEQ ID NO: 381; or
(r') the HCDR1 comprises an amino acid sequence shown in SEQ ID NO: 107, the HCDR2 comprises an amino acid sequence shown in SEQ ID NO: 190, the HCDR3 comprises an amino acid sequence shown in SEQ ID NO: 256, the LCDR1 comprises an amino acid sequence shown in SEQ ID NO: 321, the LCDR2 comprises the amino acid sequence of AA, and the LCDR3 comprises an amino acid sequence shown in SEQ ID NO: 381; or
(s') the HCDR1 comprises an amino acid sequence shown in SEQ ID NO: 100, the HCDR2 comprises an amino acid sequence shown in SEQ ID NO: 179, the HCDR3 comprises an amino acid sequence shown in SEQ ID NO: 259, the LCDR1 comprises an amino acid sequence shown in SEQ ID NO: 310, the LCDR2 comprises the amino acid sequence of FA, and the LCDR3 comprises an amino acid sequence shown in SEQ ID NO: 383; or
(t') the HCDR1 comprises an amino acid sequence shown in SEQ ID NO: 104, the HCDR2 comprises an amino acid sequence shown in SEQ ID NO: 183, the HCDR3 comprises an amino acid sequence shown in SEQ ID NO: 262, the LCDR1 comprises an amino acid sequence shown in SEQ ID NO: 312, the LCDR2 comprises the amino acid sequence of SA, and the LCDR3 comprises an amino acid sequence shown in SEQ ID NO: 391.

4. The anti-CDH17 antibody or the antigen-binding fragment thereof according to any one of claims 1 to 3, wherein the antibody comprises a heavy chain Frame Region (FR) and/or a light chain FR, and the heavy chain FR and/or the light chain FR is derived from at least one of a mouse-derived antibody, a human antibody, a primate-derived antibody, or mutants thereof.

5. The anti-CDH17 antibody or the antigen-binding fragment thereof according to any one of claims 1 to 4, wherein the FR of the heavy chain variable region comprises HFR1, HFR2, HFR3, and HFR4, and the FR of the light chain variable region comprises LFR1, LFR2, LFR3, and LFR4, wherein
the HFR1 comprises an amino acid sequence shown in any one of SEQ ID NOs: 398-415, or comprises a sequence having at least 78% sequence identity to the amino acid sequence shown in any one of SEQ ID NOs: 398-415; and/or
the HFR2 comprises an amino acid sequence shown in any one of SEQ ID NO: 416, 418, 422, 426, 428, 431, 433, 436, 439, 442, or 445-448, or comprises a sequence having at least 70% sequence identity to the amino acid sequence shown in any one of SEQ ID NO: 416, 418, 422, 426, 428, 431, 433, 436, 439, 442, or 445-448; and/or
the HFR3 comprises an amino acid sequence shown in any one of SEQ ID NOs: 449, 451, 453, 455, 457, 459, 461, 463, 465, 467, 469, 472, 474, 477, 479, 481, 483, 485, or 487, or comprises a sequence having at least 72% sequence identity to the amino acid sequence shown in any one of SEQ ID NOs: 449, 451, 453, 455, 457, 459, 461, 463, 465, 467, 469, 472, 474, 477, 479, 481, 483, 485, or 487; and/or
the HFR4 comprises an amino acid sequence shown in any one of SEQ ID NOs: 488-493, or comprises a sequence having at least 86% sequence identity to the amino acid sequence shown in any one of SEQ ID NOs: 488-493; and/or
the LFR1 comprises an amino acid sequence shown in any one of SEQ ID NOs: 494, 496, 498, 500, 502, 504, 506, 508, 510, 512, 514, 516, 518, 520, 522, 524, or 526, or comprises a sequence having at least 70% sequence identity to the amino acid sequence shown in any one of SEQ ID NOs: 494, 496, 498, 500, 502, 504, 506, 508, 510, 512, 514, 516, 518, 520, 522, 524, or 526; and/or
the LFR2 comprises an amino acid sequence shown in any one of SEQ ID NOs: 528, 533, 535, 537, 539, 541, 543, or 549-552, or comprises a sequence having at least 80% sequence identity to the amino acid sequence shown in any one of SEQ ID NOs: 528, 533, 535, 537, 539, 541, 543, or 549-552; and/or
the LFR3 comprises an amino acid sequence shown in any one of SEQ ID NOs: 554, 556, 558, 560, 562, 564, 566, 568, 570, 572, 574, 576, 578, 580, 581, or 582, or comprises a sequence having at least 79% sequence identity to the amino acid sequence shown in any one of SEQ ID NOs: 554, 556, 558, 560, 562, 564, 566, 568, 570, 572, 574, 576, 578, 580, 581, or 582; and/or the LFR4 comprises an amino acid sequence shown in any one of SEQ ID NOs: 586-590, or comprises a sequence having at least 95% sequence identity to the amino acid sequence shown in any one of SEQ ID NOs: 586-590;
optionally, according to the IMGT definition manner, the heavy chain FR of the anti-CDH17 antibody or the antigen-binding fragment comprises any one of the following (A)-(T):
(A) the HFR1 comprises an amino acid sequence shown in SEQ ID NO: 414; the HFR2 comprises an amino acid sequence shown in SEQ ID NO: 417; the HFR3 comprises an amino acid sequence shown in SEQ ID NO: 450, and the HFR4 comprises an amino acid sequence shown in SEQ ID NO: 490;
(B) the HFR1 comprises an amino acid sequence shown in SEQ ID NO: 405; the HFR2 comprises an amino acid sequence shown in SEQ ID NO: 444; the HFR3 comprises an amino acid sequence shown in SEQ ID NO: 452, and the HFR4 comprises an amino acid sequence shown in SEQ ID NO: 491;
(C) the HFR1 comprises an amino acid sequence shown in SEQ ID NO: 399; the HFR2 comprises an amino acid sequence shown in SEQ ID NO: 419; the HFR3 comprises an amino acid sequence shown in SEQ ID NO: 454, and the HFR4 comprises an amino acid sequence shown in SEQ ID NO: 491;
(D) the HFR1 comprises an amino acid sequence shown in SEQ ID NO: 414; the HFR2 comprises an amino acid sequence shown in SEQ ID NO: 420; the HFR3 comprises an amino acid sequence shown in SEQ ID NO: 456, and the HFR4 comprises an amino acid sequence shown in SEQ ID NO: 493;
(E) the HFR1 comprises an amino acid sequence shown in SEQ ID NO: 404; the HFR2 comprises an amino acid sequence shown in SEQ ID NO: 421; the HFR3 comprises an amino acid sequence shown in SEQ ID NO: 458, and the HFR4 comprises an amino acid sequence shown in SEQ ID NO: 492;
(F) the HFR1 comprises an amino acid sequence shown in SEQ ID NO: 411; the HFR2 comprises an amino acid sequence shown in SEQ ID NO: 423; the HFR3 comprises an amino acid sequence shown in SEQ ID NO: 460, and the HFR4 comprises an amino acid sequence shown in SEQ ID NO: 493;
(G) the HFR1 comprises an amino acid sequence shown in SEQ ID NO: 407; the HFR2 comprises an amino acid sequence shown in SEQ ID NO: 424; the HFR3 comprises an amino acid sequence shown in SEQ ID NO: 462, and the HFR4 comprises an amino acid sequence shown in SEQ ID NO: 493;
(H) the HFR1 comprises an amino acid sequence shown in SEQ ID NO: 401; the HFR2 comprises an amino acid sequence shown in SEQ ID NO: 425; the HFR3 comprises an amino acid sequence shown in SEQ ID NO: 464, and the HFR4 comprises an amino acid sequence shown in SEQ ID NO: 488;
(I) the HFR1 comprises an amino acid sequence shown in SEQ ID NO: 409; the HFR2 comprises an amino acid sequence shown in SEQ ID NO: 427; the HFR3 comprises an amino acid sequence shown in SEQ ID NO: 466, and the HFR4 comprises an amino acid sequence shown in SEQ ID NO: 491;
(J) the HFR1 comprises an amino acid sequence shown in SEQ ID NO: 415; the HFR2 comprises an amino acid sequence shown in SEQ ID NO: 429; the HFR3 comprises an amino acid sequence shown in SEQ ID NO: 468, and the HFR4 comprises an amino acid sequence shown in SEQ ID NO: 493;
(K) the HFR1 comprises an amino acid sequence shown in SEQ ID NO: 403; the HFR2 comprises an amino acid sequence shown in SEQ ID NO: 444; the HFR3 comprises an amino acid sequence shown in SEQ ID NO: 470, and the HFR4 comprises an amino acid sequence shown in SEQ ID NO: 491;
(L) the HFR1 comprises an amino acid sequence shown in SEQ ID NO: 410; the HFR2 comprises an amino acid sequence shown in SEQ ID NO: 430; the HFR3 comprises an amino acid sequence shown in SEQ ID NO: 471, and the HFR4 comprises an amino acid sequence shown in SEQ ID NO: 491;
(M) the HFR1 comprises an amino acid sequence shown in SEQ ID NO: 406; the HFR2 comprises an amino acid sequence shown in SEQ ID NO: 432; the HFR3 comprises an amino acid sequence shown in SEQ ID NO: 473, and the HFR4 comprises an amino acid sequence shown in SEQ ID NO: 491;
(N) the HFR1 comprises an amino acid sequence shown in SEQ ID NO: 400; the HFR2 comprises an amino acid sequence shown in SEQ ID NO: 434; the HFR3 comprises an amino acid sequence shown in SEQ ID NO: 475, and the HFR4 comprises an amino acid sequence shown in SEQ ID NO: 492;
(O) the HFR1 comprises an amino acid sequence shown in SEQ ID NO: 412; the HFR2 comprises an amino acid sequence shown in SEQ ID NO: 435; the HFR3 comprises an amino acid sequence shown in SEQ ID NO: 476, and the HFR4 comprises an amino acid sequence shown in SEQ ID NO: 492;
(P) the HFR1 comprises an amino acid sequence shown in SEQ ID NO: 408; the HFR2 comprises an amino acid sequence shown in SEQ ID NO: 437; the HFR3 comprises an amino acid sequence shown in SEQ ID NO: 478, and the HFR4 comprises an amino acid sequence shown in SEQ ID NO: 491;
(Q) the HFR1 comprises an amino acid sequence shown in SEQ ID NO: 398; the HFR2 comprises an amino acid sequence shown in SEQ ID NO: 438; the HFR3 comprises an amino acid sequence shown in SEQ ID NO: 480, and the HFR4 comprises an amino acid sequence shown in SEQ ID NO: 489;
(R) the HFR1 comprises an amino acid sequence shown in SEQ ID NO: 398; the HFR2 comprises an amino acid sequence shown in SEQ ID NO: 440; the HFR3 comprises an amino acid sequence shown in SEQ ID NO: 482, and the HFR4 comprises an amino acid sequence shown in SEQ ID NO: 493;
(S) the HFR1 comprises an amino acid sequence shown in SEQ ID NO: 413; the HFR2 comprises an amino acid sequence shown in SEQ ID NO: 441; the HFR3 comprises an amino acid sequence shown in SEQ ID NO: 484, and the HFR4 comprises an amino acid sequence shown in SEQ ID NO: 492;
(T) the HFR1 comprises an amino acid sequence shown in SEQ ID NO: 402; the HFR2 comprises an amino acid sequence shown in SEQ ID NO: 443; the HFR3 comprises an amino acid sequence shown in SEQ ID NO: 486, and the HFR4 comprises an amino acid sequence shown in SEQ ID NO: 493; and
optionally, according to the IMGT definition manner, the light chain FR of the anti-CDH17 antibody or an antigen-binding fragment comprises any one of the following (A')-(T'):
(A') the LFR1 comprises an amino acid sequence shown in SEQ ID NO: 495; the LFR2 comprises an amino acid sequence shown in SEQ ID NO: 553; the LFR3 comprises an amino acid sequence shown in SEQ ID NO: 555, and the LFR4 comprises an amino acid sequence shown in SEQ ID NO: 587;
(B') the LFR1 comprises an amino acid sequence shown in SEQ ID NO: 527; the LFR2 comprises an amino acid sequence shown in SEQ ID NO: 546; the LFR3 comprises an amino acid sequence shown in SEQ ID NO: 585, and the LFR4 comprises an amino acid sequence shown in SEQ ID NO: 587;
(C') the LFR1 comprises an amino acid sequence shown in SEQ ID NO: 497; the LFR2 comprises an amino acid sequence shown in SEQ ID NO: 529; the LFR3 comprises an amino acid sequence shown in SEQ ID NO: 557, and the LFR4 comprises an amino acid sequence shown in SEQ ID NO: 586;
(D') the LFR1 comprises an amino acid sequence shown in SEQ ID NO: 499; the LFR2 comprises an amino acid sequence shown in SEQ ID NO: 530; the LFR3 comprises an amino acid sequence shown in SEQ ID NO: 559, and the LFR4 comprises an amino acid sequence shown in SEQ ID NO: 587;
(E') the LFR1 comprises an amino acid sequence shown in SEQ ID NO: 501; the LFR2 comprises an amino acid sequence shown in SEQ ID NO: 547; the LFR3 comprises an amino acid sequence shown in SEQ ID NO: 583, and the LFR4 comprises an amino acid sequence shown in SEQ ID NO: 587;
(F') the LFR1 comprises an amino acid sequence shown in SEQ ID NO: 525; the LFR2 comprises an amino acid sequence shown in SEQ ID NO: 531; the LFR3 comprises an amino acid sequence shown in SEQ ID NO: 561, and the LFR4 comprises an amino acid sequence shown in SEQ ID NO: 588;
(G') the LFR1 comprises an amino acid sequence shown in SEQ ID NO: 503; the LFR2 comprises an amino acid sequence shown in SEQ ID NO: 547; the LFR3 comprises an amino acid sequence shown in SEQ ID NO: 583, and the LFR4 comprises an amino acid sequence shown in SEQ ID NO: 587;
(H') the LFR1 comprises an amino acid sequence shown in SEQ ID NO: 505; the LFR2 comprises an amino acid sequence shown in SEQ ID NO: 532; the LFR3 comprises an amino acid sequence shown in SEQ ID NO: 563, and the LFR4 comprises an amino acid sequence shown in SEQ ID NO: 587;
(I') the LFR1 comprises an amino acid sequence shown in SEQ ID NO: 507; the LFR2 comprises an amino acid sequence shown in SEQ ID NO: 534; the LFR3 comprises an amino acid sequence shown in SEQ ID NO: 565, and the LFR4 comprises an amino acid sequence shown in SEQ ID NO: 589;
(J') the LFR1 comprises an amino acid sequence shown in SEQ ID NO: 509; the LFR2 comprises an amino acid sequence shown in SEQ ID NO: 536; the LFR3 comprises an amino acid sequence shown in SEQ ID NO: 567, and the LFR4 comprises an amino acid sequence shown in SEQ ID NO: 587;
(K') the LFR1 comprises an amino acid sequence shown in SEQ ID NO: 527; the LFR2 comprises an amino acid sequence shown in SEQ ID NO: 546; the LFR3 comprises an amino acid sequence shown in SEQ ID NO: 585, and the LFR4 comprises an amino acid sequence shown in SEQ ID NO: 587;
(L') the LFR1 comprises an amino acid sequence shown in SEQ ID NO: 511; the LFR2 comprises an amino acid sequence shown in SEQ ID NO: 538; the LFR3 comprises an amino acid sequence shown in SEQ ID NO: 569, and the LFR4 comprises an amino acid sequence shown in SEQ ID NO: 589;
(M') the LFR1 comprises an amino acid sequence shown in SEQ ID NO: 513; the LFR2 comprises an amino acid sequence shown in SEQ ID NO: 546; the LFR3 comprises an amino acid sequence shown in SEQ ID NO: 585, and the LFR4 comprises an amino acid sequence shown in SEQ ID NO: 587;
(N') the LFR1 comprises an amino acid sequence shown in SEQ ID NO: 515; the LFR2 comprises an amino acid sequence shown in SEQ ID NO: 540; the LFR3 comprises an amino acid sequence shown in SEQ ID NO: 571, and the LFR4 comprises an amino acid sequence shown in SEQ ID NO: 590;
(O') the LFR1 comprises an amino acid sequence shown in SEQ ID NO: 517; the LFR2 comprises an amino acid sequence shown in SEQ ID NO: 542; the LFR3 comprises an amino acid sequence shown in SEQ ID NO: 573, and the LFR4 comprises an amino acid sequence shown in SEQ ID NO: 589;
(P') the LFR1 comprises an amino acid sequence shown in SEQ ID NO: 519; the LFR2 comprises an amino acid sequence shown in SEQ ID NO: 544; the LFR3 comprises an amino acid sequence shown in SEQ ID NO: 575, and the LFR4 comprises an amino acid sequence shown in SEQ ID NO: 587;
(Q') the LFR1 comprises an amino acid sequence shown in SEQ ID NO: 523; the LFR2 comprises an amino acid sequence shown in SEQ ID NO: 548; the LFR3 comprises an amino acid sequence shown in SEQ ID NO: 584, and the LFR4 comprises an amino acid sequence shown in SEQ ID NO: 587;
(R') the LFR1 comprises an amino acid sequence shown in SEQ ID NO: 523; the LFR2 comprises an amino acid sequence shown in SEQ ID NO: 548; the LFR3 comprises an amino acid sequence shown in SEQ ID NO: 584, and the LFR4 comprises an amino acid sequence shown in SEQ ID NO: 587;
(S') the LFR1 comprises an amino acid sequence shown in SEQ ID NO: 525; the LFR2 comprises an amino acid sequence shown in SEQ ID NO: 545; the LFR3 comprises an amino acid sequence shown in SEQ ID NO: 577, and the LFR4 comprises an amino acid sequence shown in SEQ ID NO: 587; and
(T') the LFR1 comprises an amino acid sequence shown in SEQ ID NO: 521; the LFR2 comprises an amino acid sequence shown in SEQ ID NO: 553; the LFR3 comprises an amino acid sequence shown in SEQ ID NO: 579, and the LFR4 comprises an amino acid sequence shown in SEQ ID NO: 587.

6. The anti-CDH17 antibody or the antigen-binding fragment thereof according to any one of claims 1 to 5, comprising a heavy chain variable region having an amino acid sequence shown in any one of SEQ ID NOs: 1, 3, 5, 7, 9, 11, 13, 15, 17, 19, 21, 23, 25, 27, 29, 31, 33, 35, 37, 39, 599-603, or 606-609, or a sequence having at least 64% sequence identity to the amino acid sequence shown in any one of SEQ ID NOs: 1, 3, 5, 7, 9, 11, 13, 15, 17, 19, 21, 23, 25, 27, 29, 31, 33, 35, 37, 39, 599-603, or 606-609; and/or
a light chain variable region having an amino acid sequence shown in any one of SEQ ID NOs: 2, 4, 6, 8, 10, 12, 14, 16, 18, 20, 22, 24, 26, 28, 30, 32, 34, 36, 38, 40, 604, 605, or 610-614, or a sequence having at least 70% sequence identity to the amino acid sequence shown in any one of SEQ ID NOs: 2, 4, 6, 8, 10, 12, 14, 16, 18, 20, 22, 24, 26, 28, 30, 32, 34, 36, 38, 40, 604, 605, or 610-614.

7. The anti-CDH17 antibody or the antigen-binding fragment thereof according to any one of claims 1 to 6, comprising any one of the following heavy chain variable regions and light chain variable regions:
(a") the heavy chain variable region having the amino acid sequence shown in SEQ ID NO: 11; and the light chain variable region having the amino acid sequence shown in SEQ ID NO: 12;
(b") the heavy chain variable region having the amino acid sequence shown in SEQ ID NO: 599; and the light chain variable region having the amino acid sequence shown in SEQ ID NO: 604;
(c") the heavy chain variable region having the amino acid sequence shown in SEQ ID NO: 5; and the light chain variable region having the amino acid sequence shown in SEQ ID NO: 6;
(d") the heavy chain variable region having the amino acid sequence shown in SEQ ID NO: 7; and the light chain variable region having the amino acid sequence shown in SEQ ID NO: 8;
(e") the heavy chain variable region having the amino acid sequence shown in SEQ ID NO: 9; and the light chain variable region having the amino acid sequence shown in SEQ ID NO: 10;
(f") the heavy chain variable region having the amino acid sequence shown in SEQ ID NO: 1; and the light chain variable region having the amino acid sequence shown in SEQ ID NO: 2;
(g") the heavy chain variable region having the amino acid sequence shown in SEQ ID NO: 13; and the light chain variable region having the amino acid sequence shown in SEQ ID NO: 14;
(h") the heavy chain variable region having the amino acid sequence shown in SEQ ID NO: 15; and the light chain variable region having the amino acid sequence shown in SEQ ID NO: 16;
(i") the heavy chain variable region having the amino acid sequence shown in SEQ ID NO: 17; and the light chain variable region having the amino acid sequence shown in SEQ ID NO: 18;
(j") the heavy chain variable region having the amino acid sequence shown in SEQ ID NO: 19; and the light chain variable region having the amino acid sequence shown in SEQ ID NO: 20;
(k") the heavy chain variable region having the amino acid sequence shown in SEQ ID NO: 21; and the light chain variable region having the amino acid sequence shown in SEQ ID NO: 22;
(l") the heavy chain variable region having the amino acid sequence shown in SEQ ID NO: 23; and the light chain variable region having the amino acid sequence shown in SEQ ID NO: 24;
(m") the heavy chain variable region having the amino acid sequence shown in SEQ ID NO: 25; and the light chain variable region having the amino acid sequence shown in SEQ ID NO: 26;
(n") the heavy chain variable region having the amino acid sequence shown in SEQ ID NO: 27; and the light chain variable region having the amino acid sequence shown in SEQ ID NO: 28;
(o") the heavy chain variable region having the amino acid sequence shown in SEQ ID NO: 29; and the light chain variable region having the amino acid sequence shown in SEQ ID NO: 30;
(p") the heavy chain variable region having the amino acid sequence shown in SEQ ID NO: 31; and the light chain variable region having the amino acid sequence shown in SEQ ID NO: 32;
(q") the heavy chain variable region having the amino acid sequence shown in SEQ ID NO: 33; and the light chain variable region having the amino acid sequence shown in SEQ ID NO: 34;
(r") the heavy chain variable region having the amino acid sequence shown in SEQ ID NO: 35; and the light chain variable region having the amino acid sequence shown in SEQ ID NO: 36;
(s") the heavy chain variable region having the amino acid sequence shown in SEQ ID NO: 37; and the light chain variable region having the amino acid sequence shown in SEQ ID NO: 38;
(t") the heavy chain variable region having the amino acid sequence shown in SEQ ID NO: 39; and the light chain variable region having the amino acid sequence shown in SEQ ID NO: 40;
(u") the heavy chain variable region having the amino acid sequence shown in SEQ ID NO: 3; and the light chain variable region having the amino acid sequence shown in SEQ ID NO: 4;
(v") the heavy chain variable region having the amino acid sequence shown in SEQ ID NO: 599; and the light chain variable region having the amino acid sequence shown in SEQ ID NO: 605;
(w") the heavy chain variable region having the amino acid sequence shown in SEQ ID NO: 600; and the light chain variable region having the amino acid sequence shown in SEQ ID NO: 604;
(x") the heavy chain variable region having the amino acid sequence shown in SEQ ID NO: 600; and the light chain variable region having the amino acid sequence shown in SEQ ID NO: 605;
(y") the heavy chain variable region having the amino acid sequence shown in SEQ ID NO: 601; and the light chain variable region having the amino acid sequence shown in SEQ ID NO: 604;
(z") the heavy chain variable region having the amino acid sequence shown in SEQ ID NO: 601; and the light chain variable region having the amino acid sequence shown in SEQ ID NO: 605;
(a"') the heavy chain variable region having the amino acid sequence shown in SEQ ID NO: 602; and the light chain variable region having the amino acid sequence shown in SEQ ID NO: 604;
(b"') the heavy chain variable region having the amino acid sequence shown in SEQ ID NO: 602; and the light chain variable region having the amino acid sequence shown in SEQ ID NO: 605;
(c"') the heavy chain variable region having the amino acid sequence shown in SEQ ID NO: 603; and the light chain variable region having the amino acid sequence shown in SEQ ID NO: 604;
(d"') the heavy chain variable region having the amino acid sequence shown in SEQ ID NO: 603; and the light chain variable region having the amino acid sequence shown in SEQ ID NO: 605;
(e"') the heavy chain variable region having the amino acid sequence shown in SEQ ID NO: 606; and the light chain variable region having the amino acid sequence shown in SEQ ID NO: 613;
(f"') the heavy chain variable region having the amino acid sequence shown in SEQ ID NO: 607; and the light chain variable region having the amino acid sequence shown in SEQ ID NO: 610;
(g"') the heavy chain variable region having the amino acid sequence shown in SEQ ID NO: 607; and the light chain variable region having the amino acid sequence shown in SEQ ID NO: 611;
(h"') the heavy chain variable region having the amino acid sequence shown in SEQ ID NO: 607; and the light chain variable region having the amino acid sequence shown in SEQ ID NO: 612;
(i"') the heavy chain variable region having the amino acid sequence shown in SEQ ID NO: 607; and the light chain variable region having the amino acid sequence shown in SEQ ID NO: 613;
(j"') the heavy chain variable region having the amino acid sequence shown in SEQ ID NO: 608; and the light chain variable region having the amino acid sequence shown in SEQ ID NO: 611;
(k"') the heavy chain variable region having the amino acid sequence shown in SEQ ID NO: 608; and the light chain variable region having the amino acid sequence shown in SEQ ID NO: 612;
(l"') the heavy chain variable region having the amino acid sequence shown in SEQ ID NO: 608; and the light chain variable region having the amino acid sequence shown in SEQ ID NO: 613;
(m"') the heavy chain variable region having the amino acid sequence shown in SEQ ID NO: 609; and the light chain variable region having the amino acid sequence shown in SEQ ID NO: 611;
(n"') the heavy chain variable region having the amino acid sequence shown in SEQ ID NO: 609; and the light chain variable region having the amino acid sequence shown in SEQ ID NO: 612;
(o"') the heavy chain variable region having the amino acid sequence shown in SEQ ID NO: 609; and the light chain variable region having the amino acid sequence shown in SEQ ID NO: 613; and
(p"') the heavy chain variable region having the amino acid sequence shown in SEQ ID NO: 610; and the light chain variable region having the amino acid sequence shown in SEQ ID NO: 614;

8. The anti-CDH17 antibody or the antigen-binding fragment thereof according to any one of claims 1 to 7, comprising part or all sequences of a constant region, wherein the constant region is derived from at least one of a mouse-derived antibody, a human antibody, a primate-derived antibody, or mutants thereof;
optionally, a heavy chain constant region comprises sequences of part or all constant regions of any one of IgG1, IgG2a, IgG2b, IgG3, IgG4, IgA, IgM, IgE, or IgD; and a light chain constant region of the antibody is a κ or λ chain;
optionally, the heavy chain constant region comprises a constant region sequence of the IgG1, IgG2a, or IgG2b;
optionally, the heavy chain constant region comprises sequences of part or all constant regions of the IgG1, and the anti-CDH17 antibody or the antigen-binding fragment thereof comprises any one of the following heavy chain variable regions and light chain variable regions:
(a") the heavy chain variable region having the amino acid sequence shown in SEQ ID NO: 599; and the light chain variable region having the amino acid sequence shown in SEQ ID NO: 604;
(c") the heavy chain variable region having the amino acid sequence shown in SEQ ID NO: 5;
and the light chain variable region having the amino acid sequence shown in SEQ ID NO: 6;
(t") the heavy chain variable region having the amino acid sequence shown in SEQ ID NO: 39; and the light chain variable region having the amino acid sequence shown in SEQ ID NO: 40;
(u") the heavy chain variable region having the amino acid sequence shown in SEQ ID NO: 1; and the light chain variable region having the amino acid sequence shown in SEQ ID NO: 2;
(v") the heavy chain variable region having the amino acid sequence shown in SEQ ID NO: 599; and the light chain variable region having the amino acid sequence shown in SEQ ID NO: 605;
(w") the heavy chain variable region having the amino acid sequence shown in SEQ ID NO: 600; and the light chain variable region having the amino acid sequence shown in SEQ ID NO: 604;
(x") the heavy chain variable region having the amino acid sequence shown in SEQ ID NO: 600; and the light chain variable region having the amino acid sequence shown in SEQ ID NO: 605;
(y") the heavy chain variable region having the amino acid sequence shown in SEQ ID NO: 601; and the light chain variable region having the amino acid sequence shown in SEQ ID NO: 604;
(z") the heavy chain variable region having the amino acid sequence shown in SEQ ID NO: 601; and the light chain variable region having the amino acid sequence shown in SEQ ID NO: 605;
(a"') the heavy chain variable region having the amino acid sequence shown in SEQ ID NO: 602; and the light chain variable region having the amino acid sequence shown in SEQ ID NO: 604;
(b"') the heavy chain variable region having the amino acid sequence shown in SEQ ID NO: 602; and the light chain variable region having the amino acid sequence shown in SEQ ID NO: 605;
(c"') the heavy chain variable region having the amino acid sequence shown in SEQ ID NO: 603; and the light chain variable region having the amino acid sequence shown in SEQ ID NO: 604;
(d"') the heavy chain variable region having the amino acid sequence shown in SEQ ID NO: 603; and the light chain variable region having the amino acid sequence shown in SEQ ID NO: 605;
(e"') the heavy chain variable region having the amino acid sequence shown in SEQ ID NO: 606; and the light chain variable region having the amino acid sequence shown in SEQ ID NO: 613;
(f"') the heavy chain variable region having the amino acid sequence shown in SEQ ID NO: 607; and the light chain variable region having the amino acid sequence shown in SEQ ID NO: 610;
(g"') the heavy chain variable region having the amino acid sequence shown in SEQ ID NO: 607; and the light chain variable region having the amino acid sequence shown in SEQ ID NO: 611;
(h"') the heavy chain variable region having the amino acid sequence shown in SEQ ID NO: 607; and the light chain variable region having the amino acid sequence shown in SEQ ID NO: 612;
(i"') the heavy chain variable region having the amino acid sequence shown in SEQ ID NO: 607; and the light chain variable region having the amino acid sequence shown in SEQ ID NO: 613;
(j"') the heavy chain variable region having the amino acid sequence shown in SEQ ID NO: 608; and the light chain variable region having the amino acid sequence shown in SEQ ID NO: 611;
(k"') the heavy chain variable region having the amino acid sequence shown in SEQ ID NO: 608; and the light chain variable region having the amino acid sequence shown in SEQ ID NO: 612;
(l"') the heavy chain variable region having the amino acid sequence shown in SEQ ID NO: 608; and the light chain variable region having the amino acid sequence shown in SEQ ID NO: 613;
(m"') the heavy chain variable region having the amino acid sequence shown in SEQ ID NO: 609; and the light chain variable region having the amino acid sequence shown in SEQ ID NO: 611;
(n"') the heavy chain variable region having the amino acid sequence shown in SEQ ID NO: 609; and the light chain variable region having the amino acid sequence shown in SEQ ID NO: 612;
(o"') the heavy chain variable region having the amino acid sequence shown in SEQ ID NO: 609; and the light chain variable region having the amino acid sequence shown in SEQ ID NO: 613; and
(p"') the heavy chain variable region having the amino acid sequence shown in SEQ ID NO: 610; and the light chain variable region having the amino acid sequence shown in SEQ ID NO: 614;
optionally, the heavy chain constant region comprises sequences of part or all constant regions of the IgG2a, and the anti-CDH17 antibody or the antigen-binding fragment thereof comprises any one of the following heavy chain variable regions and light chain variable regions:
(d") the heavy chain variable region having the amino acid sequence shown in SEQ ID NO: 7; and the light chain variable region having the amino acid sequence shown in SEQ ID NO: 8;
(f") the heavy chain variable region having the amino acid sequence shown in SEQ ID NO: 11; and the light chain variable region having the amino acid sequence shown in SEQ ID NO: 12;
(g") the heavy chain variable region having the amino acid sequence shown in SEQ ID NO: 13; and the light chain variable region having the amino acid sequence shown in SEQ ID NO: 14;
(i") the heavy chain variable region having the amino acid sequence shown in SEQ ID NO: 17; and the light chain variable region having the amino acid sequence shown in SEQ ID NO: 18;
(j") the heavy chain variable region having the amino acid sequence shown in SEQ ID NO: 19; and the light chain variable region having the amino acid sequence shown in SEQ ID NO: 20;
(n") the heavy chain variable region having the amino acid sequence shown in SEQ ID NO: 27; and the light chain variable region having the amino acid sequence shown in SEQ ID NO: 28;
(o") the heavy chain variable region having the amino acid sequence shown in SEQ ID NO: 29; and the light chain variable region having the amino acid sequence shown in SEQ ID NO: 30;
(p") the heavy chain variable region having the amino acid sequence shown in SEQ ID NO: 31; and the light chain variable region having the amino acid sequence shown in SEQ ID NO: 32;
(q") the heavy chain variable region having the amino acid sequence shown in SEQ ID NO: 33; and the light chain variable region having the amino acid sequence shown in SEQ ID NO: 34;
(r") the heavy chain variable region having the amino acid sequence shown in SEQ ID NO: 35; and the light chain variable region having the amino acid sequence shown in SEQ ID NO: 36;
(s") the heavy chain variable region having the amino acid sequence shown in SEQ ID NO: 37; and the light chain variable region having the amino acid sequence shown in SEQ ID NO: 38; and
optionally, the heavy chain constant region comprises sequences of part or all constant regions of the IgG2b, and the anti-CDH17 antibody or the antigen-binding fragment thereof comprises any one of the following heavy chain variable regions and light chain variable regions:
(b") the heavy chain variable region having the amino acid sequence shown in SEQ ID NO: 3; and the light chain variable region having the amino acid sequence shown in SEQ ID NO: 4;
(e") the heavy chain variable region having the amino acid sequence shown in SEQ ID NO: 9; and the light chain variable region having the amino acid sequence shown in SEQ ID NO: 10;
(h") the heavy chain variable region having the amino acid sequence shown in SEQ ID NO: 15; and the light chain variable region having the amino acid sequence shown in SEQ ID NO: 16;
(k") the heavy chain variable region having the amino acid sequence shown in SEQ ID NO: 21; and the light chain variable region having the amino acid sequence shown in SEQ ID NO: 22;
(l") the heavy chain variable region having the amino acid sequence shown in SEQ ID NO: 23; and the light chain variable region having the amino acid sequence shown in SEQ ID NO: 24; and
(m") the heavy chain variable region having the amino acid sequence shown in SEQ ID NO: 25; and the light chain variable region having the amino acid sequence shown in SEQ ID NO: 26.

9. The anti-CDH17 antibody or the antigen-binding fragment thereof according to any one of claims 1 to 8, wherein the antigen-binding fragment comprises one or more of F(ab')₂, Fab', Fab, Fv, scFv, dsFv, or a minimum recognition unit of the antibody.

10. The anti-CDH17 antibody or the antigen-binding fragment thereof according to any one of claims 1 to 9, having one or more properties in the following (i) to (iv):
(i) binding to human CDH17 with EC50 not exceeding 1 µg/mL, as determined by a flow cytometry fluorescence sorting technology;
(ii) binding to monkey CDH17 with EC50 not exceeding 1 µg/mL, as determined by an ELISA technology;
(iii) having ADCC activity; and
(iv) having mediated cell endocytosis activity.

11. An anti-CDH17 antibody or an antigen-binding fragment thereof, wherein the anti-CDH17 antibody or the antigen-binding fragment either:
competes with the anti-CDH17 antibody according to any one of claims 1 to 10 for binding to CDH17, or
binds to an epitope on CDH17 that is identical to an epitope bound by the anti-CDH17 antibody according to any one of claims 1 to 10.

12. A biomaterial, comprising any one of the following (i) to (vi):
(i) a polyspecific antibody, comprising the anti-CDH17 antibody according to any one of claims 1 to 11;
(ii) a Chimeric Antigen Receptor (CAR), comprising an Extra Cellular Domain (ECD), wherein the ECD comprises an antigen-binding domain, and the antigen-binding domain comprises the anti-CDH17 antibody or the antigen-binding fragment thereof according to any one of claims 1 to 11;
(iii) a nucleic acid molecule, encoding the anti-CDH17 antibody or the antigen-binding fragment thereof according to any one of claims 1 to 11, or encoding the polyspecific antibody in (i), or encoding the CAR in (ii);
(iv) a vector, having the nucleic acid molecule in (iii);
(v) a recombinant cell, expressing the anti-CDH17 antibody or the antigen-binding fragment thereof according to any one of claims 1 to 11, or expressing the polyspecific antibody in (i), or expressing the CAR in (ii), or comprising the nucleic acid molecule in (iii), or comprising the vector in (iv); and
(vi) an engineered immune cell, expressing the CAR in (ii) or comprising the nucleic acid molecule encoding the CAR in (ii).

13. A pharmaceutical composition, comprising the anti-CDH17 antibody or the antigen-binding fragment thereof according to any of claims 1 to 11, or the biomaterial according to claim 12, wherein
optionally, the pharmaceutical composition further comprises a pharmaceutically acceptable carriers and/or excipient.

14. An application of the anti-CDH17 antibody or the antigen-binding fragment thereof according to any of claims 1 to 11, or the biomaterial according to claim 12, or the pharmaceutical composition according to claim 13 is as follows:
(I) detecting CDH17 or cells expressing the CDH17;
(I) preparing a product for detecting the CDH17 or cells expressing the CDH17;
(III) using for treating, preventing, or alleviating diseases, symptoms, or conditions associated with the CDH17; or
(IV) preparing a pharmaceutical composition for treating, preventing, or alleviating diseases, symptoms, or conditions associated with the CDH17.

15. A method for preparing the anti-CDH17 antibody or the antigen-binding fragment thereof according to any of claims 1 to 11, wherein the method comprises the following steps:
(a) culturing the recombinant cell according to claim 12 under a condition expressing the anti-CDH17 antibody or the antigen-binding fragment thereof; and
(b) separating and purifying the anti-CDH17 antibody or the antigen-binding fragment thereof obtained in step (a).

16. A method for treating a disease, wherein the method comprises applying an effective dosage of the anti-CDH17 antibody or the antigen-binding fragment thereof according to any of claims 1 to 11, the biomaterial according to claim 12, or the pharmaceutical composition according to claim 13 to a subject in need; optionally, the disease is a disease associated with high expression of human CDH17;
optionally, the disease is a tumor; and
optionally, the tumor is selected from lung cancer, prostate cancer, breast cancer, head and neck cancer, esophageal cancer, gastric cancer, colon cancer, rectal cancer, bladder cancer, cervical cancer, uterine cancer, ovarian cancer, liver cancer, melanoma, renal cancer, squamous cell carcinoma, or hematological system cancer.

17. A kit for detecting CDH17, comprising the anti-CDH17 antibody or the antigen-binding fragment thereof according to any of claims 1 to 11, or the polyspecific antibody according to claim 12.
